# EUROPÄISCHE PATENTSCHRIFT

(11) **EP 0 296 120 B1**
(45) Veröffentlichungstag und Bekanntmachung des Hinweises auf die Patenterteilung: **15.04.1992**
(21) Anmeldenummer: 88810397.5
(22) Anmeldetag: 13.06.1988
(51) Int. Cl.: C07C 335/18, C07C 335/32, C07C 267/00, C07C 331/28, C07C 217/90, A01N 47/30, A01N 47/40, A01N 47/42

(54) **Aryloxyphenyl-thioharnstoffe, -isothioharnstoffe und -carbodiimide, ihre Herstellung und Verwendung bei der Kontrolle von Schädlingen**
Aryloxyphenyl ureas, isothio ureas and carbodiimides, their preparation and their use for the control of pests
Thio-urée, isothio-urée et carbodiimides aryloxyphényliques, leur préparation et leur utilisation pour la lutte contre la vermine

(30) Priorität: 18.06.1987 CH 2302/87
(43) Veröffentlichungstag der Anmeldung: 21.12.1988
(73) Patentinhaber: CIBA-GEIGY AG, 4002 Basel (CH)
(72) Erfinder: Böger, Manfred, D-7858 Weil am Rhein 5 (DE); Drabek, Jozef, Dr., CH-4104 Oberwil (CH); Ehrenfreund, Josef, Dr., CH-4123 Allschwil (CH)

(56) Entgegenhaltungen:
- EP-A- 0 025 010
- EP-A- 0 304 025
- EP-A- 0 304 402
- WO-A-82/03390

## Beschreibung

Die vorliegende Erfindung betrifft neue substituierte Aryloxyphenyl-thioharnstoffe, -isothioharnstoffe und -carbodiimide, ihre Salze mit organischen und anorganischen Säuren, Verfahren und Zwischenprodukte zu ihrer Herstellung, Schädlingsbekämpfungsmittel, die diese Verbindungen enthalten, sowie ihre Verwendung bei Kontrolle von Schädlingen.

Die erfindungsgemässen Verbindungen entsprechen der Formel 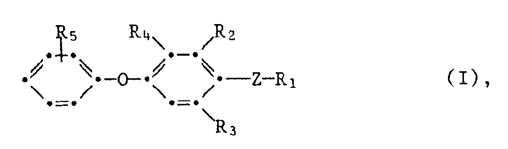 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein-oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine (CH=CH)₂, (CH₂)₃, (CH₂)₄-Brücke in 2,3-oder 3,4-Stellung;
Z für -NH-CS-NH-, -N=C(SR₆)-NH- oder -N=C=N- und
R₆ für C₁-C₁₀-Alkyl oder Allyl
stehen.

Aus der Literatur sind verschiedene Phenoxyphenyl-thioharnstoffe und -isothioharnstoffe mit pestizider Wirkung beispielsweise aus EP-A-0025010 und WO 82/03390 bekannt. Da diese Wirkstoffe nicht in jeder Hinsicht befriedigende Resultate bei der Schädlingsbekämpfung bewirken, besteht auch weiterhin ein Bedürfnis nach verbesserten Wirkstoffen dieses Strukturtyps. Ueberraschenderweise wird dieses Bedürfnis von den neuen Verbindungen der Formel weitgehend befriedigt.

Zu den Definitionen der in der Formel charakterisierten erfindungsgemässen Wirkstoffen werden die folgenden Erläuterungen abgegeben:
Bei den als Substituenten in Betracht kommenden Halogenen handelt es sich sowohl um Fluor und Chlor als auch um Brom und Jod, wobei Fluor und Chlor bevorzugt sind.

Die als Substituenten in Betracht kommenden Alkyle können geradkettig oder verzweigt sein. Als Beispiele solcher Alkyle seien Methyl, Aethyl, Propyl, Isopropyl, Butyl, i-Butyl, sek.Butyl, tert.Butyl oder Pentyl, Hexyl, Octyl usw. und ihre Isomeren genannt.

Die als Substituenten in Betracht kommenden ein- oder mehrfach durch Halogen und/oder C₁-C₆-Alkoxy substituierten C₁-C₁₂-Alkyle können geradkettig oder verzweigt und nur teilweise oder auch perhalogeniert und/ oder ein-bis fünffach durch C₁-C₆-Alkoxy substituiert sein, wobei fürdie Halogene und die Alkyle die oben gegebenen Definitionen gelten. Geeignete Beispiele solcher Substituenten sind u.a. das ein- bis dreifach durch Fluor, Chlor und/oder Brom substituierte Methyl wie z.B. CHF₂ oder CF₃; das ein- bis fünffach durch Fluor, Chlor und/oder Brom substituierte Aethyl wie z.B. CH₂CF₃, CF₂CF₃, CF₂CCI₃, CF₂CHCl₂, CF₂CHF₂, CF₂CFCI₂, CF₂ CHBr₂, CF₂CHCIF, CF₂CHBrF oder CCIFCHCIF; das ein- bis siebenfach durch Fluor, Chlor und/oder Brom substituierte Propyl oder Isopropyl wie z.B. CH₂CHBrCH₂Br, CF₂CHFCF₃, CH₂CF₂CF₃ oder CH(CF₃)₂; das ein- bis neunfach durch Fluor, Chlor und/oder Brom substituierte Butyl oder eines seiner Isomeren wie z.B. CF(CF₃) CHFCF₃ oder CH₂(CF₂)₂CF₃; Methoxymethyl, Methoxyäthyl, Aethoxyäthyl, Methoxypropyl, Aethoxypropyl, Propoxypropyl, Methyoxybutyl, Aethoxybutyl, Propoxybutyl oder Butoxybutyl, 1,2-Dimethoxyäthyl, 1,3-Dimethoxypropyl oder 2,4-Dimethoxybutyl.

Bei den als Substituenten in Betracht kommenden Cycloalkylen handelt es sich beispielsweise um Cyclopropyl, Cyclobutyl, Cyclopentyl oder Cyclohexyl. Diese können ein- oder mehrfach durch einen C₁-C₃-Alkylrest substituiert und/oder über eine C₁-C₄-Alkylenbrücke mit dem Rest des Moleküls verbunden sein.

Die Verbindungen der Formel in denen Z für -N=C(SR₆)-NH- steht, können auch in Form von Säureadditionssalzen vorliegen. Zur Bildung solcher Salze eignen sich sowohl organische als auch anorganische Säuren. Beispiele solcher Säuren sind u.a Chlorwasserstoffsäure, Bromwasser stoffsäure, Jodwasserstoffsäure, Salpetersäure, verschiedene Phosphorsäuren, Schwefelsäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäuren oder Salicylsäure.

Verbindungen der Formel I, in denen Z für -N=C(SR₆)-NH- steht, können in den tautomeren Formen 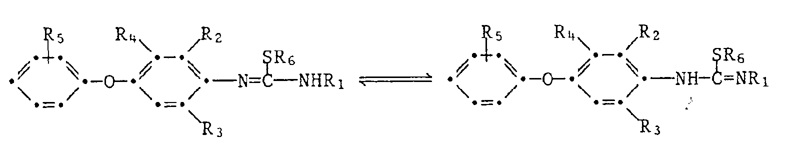 vorliegen. Die Erfindung umfasst sowohl die einzelnen Tautomeren, als auch Tautomerengemische.

Unter den Verbindungen der Formel stehen diejenigen im Vordergrund, in denen R₁ für C₁-C₈-Alkly, ein-oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder Cyclopentyl; R₄ für Wasserstoff; R₅ für eine (CH=CH)₂, -(CH₂)₃ oder 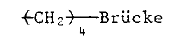 in 2,3- oder 3,4-Stellung; Z für -NH-CS-NH-, N=C(SR₆)-NH- oder -N=C=N-; und R₆ für C₁-C₅-Alkyl oder Allyl stehen.

Dabei sind diejenigen Verbindungen der Formel I bevorzugt, in denen
a) R₁ für C₁-C₄-Alkyl; R₂ und R₃ je für C₁-C₃-Alkyl; R₄ für Wasserstoff; R₅ für eine (CH=CH)₂ oder 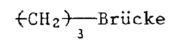 in 3,4-Stellung; und Z für -NH-CS-NH- stehen oder
b) R₁ für C₁-C₄-Alkyl; R₂ und R₃ je für C₁-C₃-Alkyl; R₄ für Wasserstoff; R₅ für eine (CH=CH)₂ oder 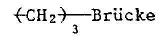 in 3,4-Stellung; Z für -N=C(SR₆)-NH-; und R₆ für C₁-C₃-Alkyl stehen oder
c) R₁ für C₁-C₄-Alkyl; R₂ und R₃ je für C₁-C₃-Alkyl; R₄ für Wasserstoff; R₅ für eine (CH=CH)₂ oder 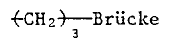 in 3,4-Stellung; und Z für -N=C=N- stehen.

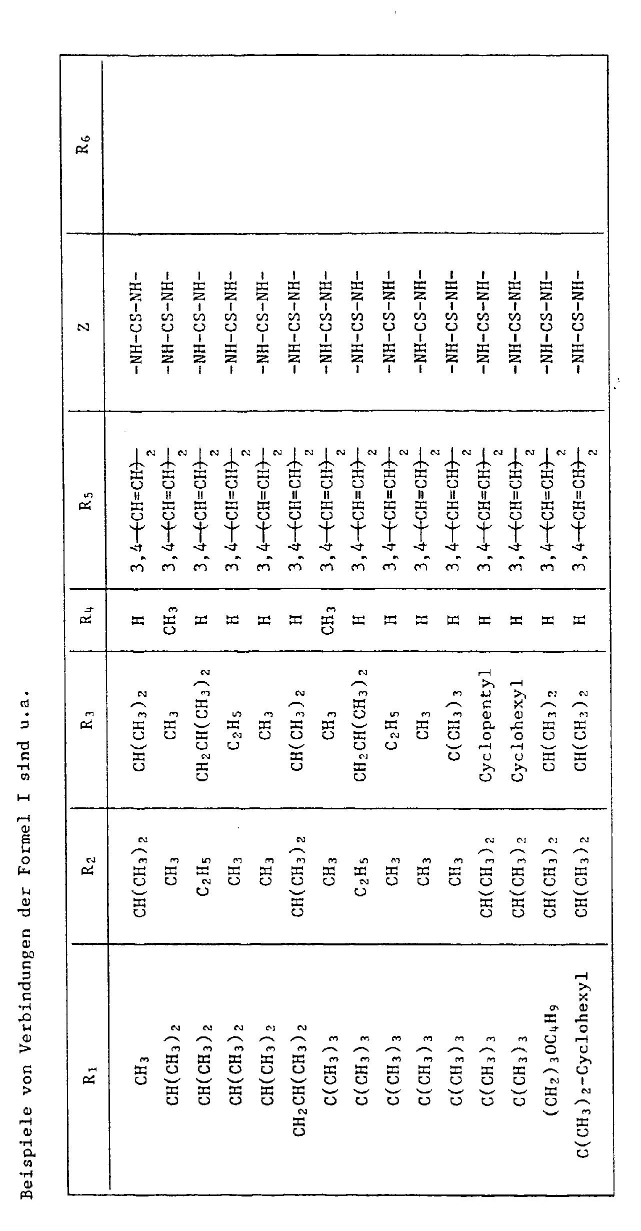

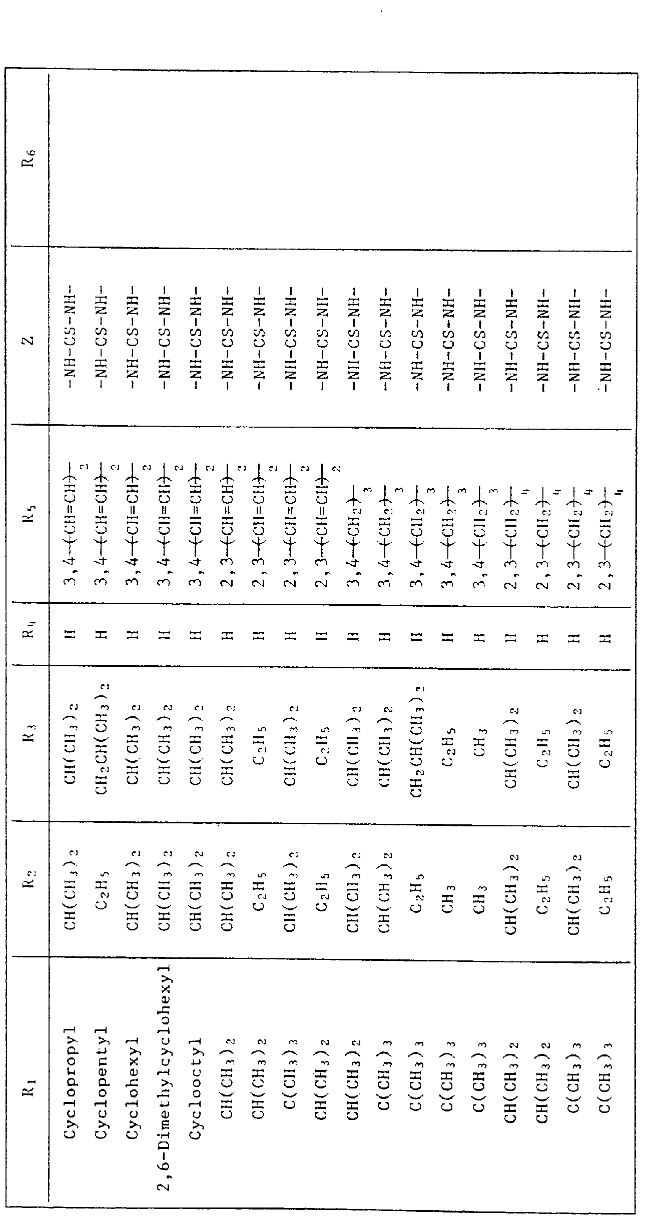

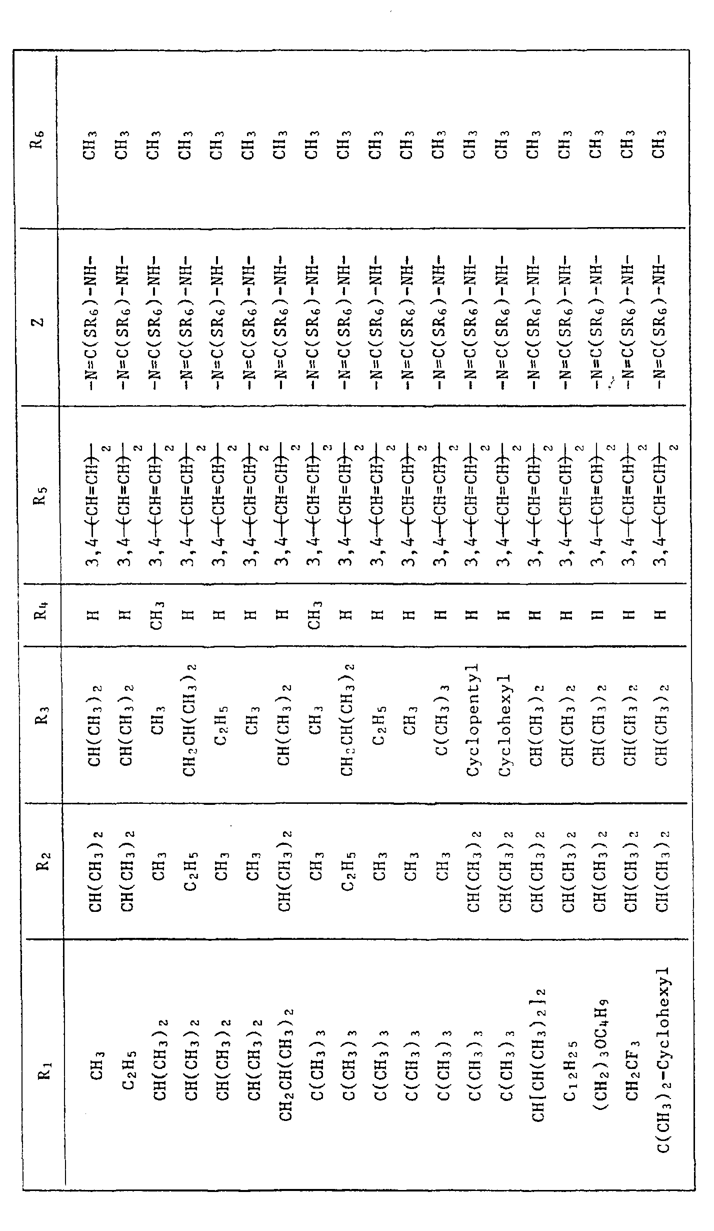

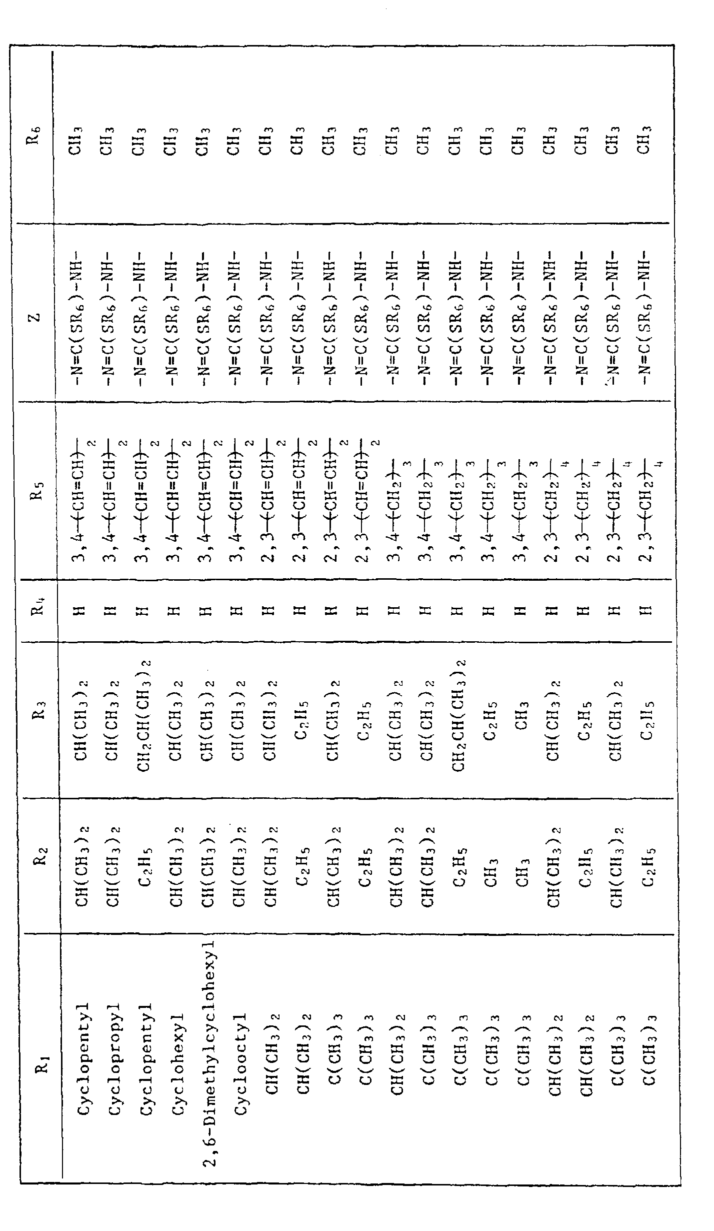

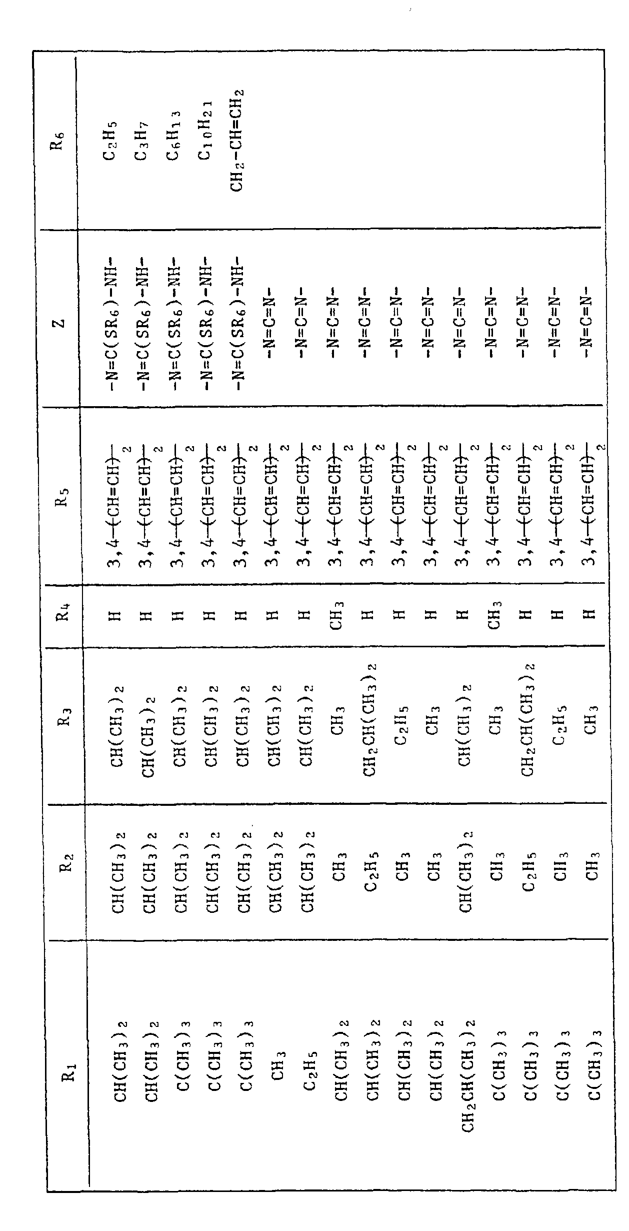

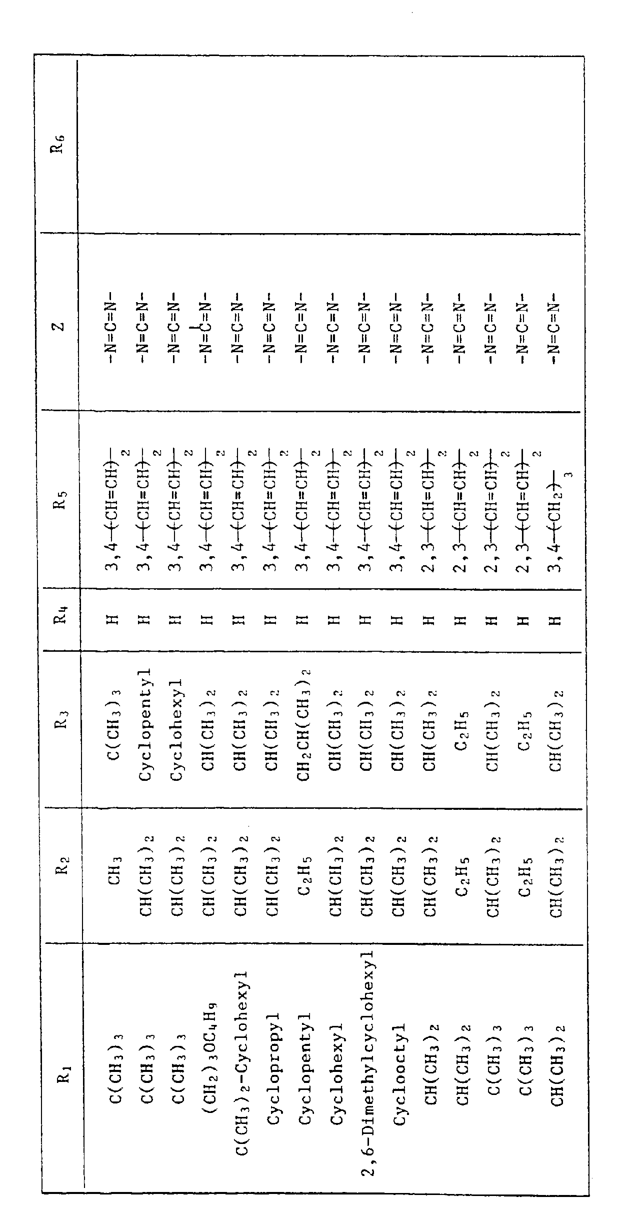

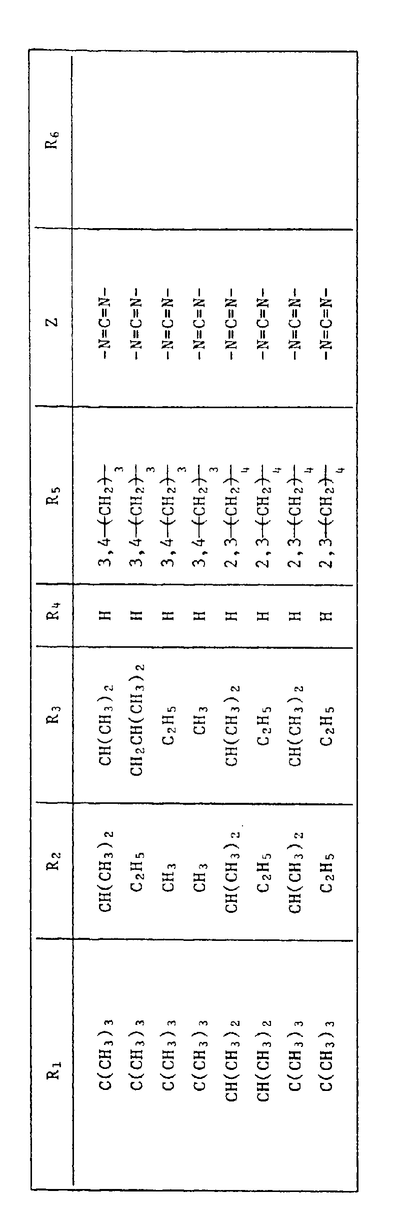

Die erfindungsgemässen Verbindungen der Formel können nach im Prinzip bekannten Verfahren hergestellt werden, indem man z.B.
A) ein Isothiocyanat der Formel II 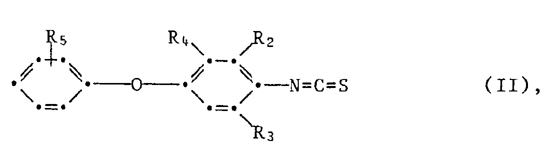 mit einem Amin der Formel III 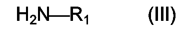 zum Thioharnstoff umsetzt und gegebenenfalls
B) den erhaltenen Thioharnstoff mit einer Verbindung der Formel IV 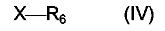 zum Isothioharnstoff umsetzt oder
C) den erhaltenen Thioharnstoff durch Abspalten von Schwefelwasserstoff in das Carbodiimid überführt.
Rᵢ, R₂, R₃, R₄, R₅ und R₆ haben dabei die angegebene Bedeutung und X steht für eine geeignete Abgangsgruppe wie z.B. ein Halogenatom, insbesondere Chlor, Brom oder Jod, oder Alkylsulfat.

Das Verfahren Awird üblicherweise unter normalem Druck, und in Gegenwart eines organischen Lösungs-oder Verdünnungsmittels durchgeführt. Die Temperatur beträgt dabei zwischen 0 und +150_{°}C, vorzugsweise +10 bis 70°C. Als Lösungs- oder Verdünnungsmittel eignen sich z.B. Aether und ätherartige Verbindungen, wie Diäthyläther, Dipropyläther, Dibutyläther, Dioxan, Dimethoxyäthan oder Te trahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetontril oder Propionitril; Ketone, wie Aceton, Methyläthylketon, Methylisopropylketon, Methylisobutylketon oder Cyclohexanon.

Das Verfahren B wird zweckmässigerweise in einem inerten organischen Lösungsmittel und unter leicht erhöhtem oder normalem Druck durchgeführt. Die Temperatur beträgt dabei zwischen +10 und 250°C, vorzugsweise Siedetemperatur des verwendeten Lösungsmittels oder +50 bis 150°C. Geeignete Lösungs- oder Verdünnungsmittel sind z.B. Aether oder ätherartige Verbindungen, wie Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon, Alkohole oder Dimethylformamid.

Das Verfahren C wird zweckmässigerweise in einem aprotischen organischen Lösungs- oder Verdünnungsmittel unter normalem Druck durchgeführt. Die Temperatur beträgt dabei zwischen 0 und +150_{°}C, vorzugsweise +10 bis 50°C. Geeignete Lösungs- oder Verdünnungsmittel sind z.B. Aether oder ätherartige Verbindungen, wie Diäthyläther, Dipropyläther, Dibutyläther, Dioxan, Dimethoxyäthan oder Tetrahydrofuran; N,N-dialkylierte Carbonsäureamide; aliphatische, aromatische sowie halogenierte Kohlenwasserstoffe, wie Benzol, Toluol, Xylole, Chloroform, Methylenchlorid, Tetrachlorkohlenstoff oder Chlorbenzol; Nitrile, wie Acetonitril oder Propionitril; Ketone, wie Aceton, Methyläthylketon, Methylisopropylketon oder Cyclohexanon. Die Abspaltung von Schwefelwasserstoff erfolgt nach in der Literatur beschriebenen Arbeitsweisen (T. Shibanuma, Chemistry Letters 1977, p. 575-76; S. Kim, Tetrahedron Letters 1985, p. 1661-64; W. Weith, B. 6,1873, p. 1398; G. Amiard, Bull. Soc. chim. 1956, p. 1360). Als Abspaltungs-Reagenzien werden dabei u.a. HgO, bestimmte Pyridinium Salze, Chloressigsäureester, Cyanursäurechlorid, p-Toluolsulfochlorid oder bestimmte Phosphorsäureester-Derivate verwendet.

Die Isothiocyanate der Formel 11 können nach im Prinzip bekannten Methoden hergestellt werden, z.B. indem man ein Phenoxyanilin der Formel V 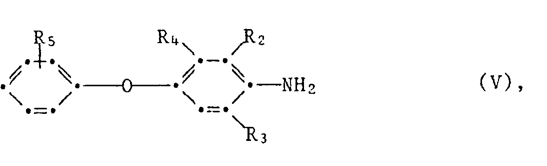 mit Thiophosgen umsetzt, wobei R₂, R₃, R₄ und R₅ die für Formel 1 angegebene Bedeutung haben.

Das Verfahren zur Herstellung der Verbindungen der Formel II wird zweckmässigerweise in Gegenwart einer organischen oder anorganischen Base, wie z.B. Triäthylamin oder Calciumcarbonat, und einem gegenüber den Reaktionsteilnehmern inerten Lösungs- oder Verdünnungsmittels unter normalem Druck durchgeführt. Die Temperatur beträgt dabei zwischen 0 und +100_{°}C, vorzugsweise Siedetemperatur des verwendeten Lösungs- oder Verdünnungsmittels oder +20 bis 80°C. Geeignete Lösungs- und Verdünnungsmittel sind u.a. Aether oder ätherartige Verbindungen, wie z.B. Diäthyläther, Diisopropyläther, Dioxan oder Tetrahydrofuran; aromatische Kohlenwasserstoffe, wie Benzol, Toluol oder Xylole; Ketone, wie Aceton, Methyläthylketon oder Cyclohexanon; oder chlorierte Kohlenwasserstoffe wie z.B. Dichlormethan. Die Herstellung kann auch in Anwesenheit von Wasser im 2-Phasensystem erfolgen.

Die Phenoxyaniline der Formel V können nach im Prinzip bekannten Methoden hergestellt werden, z. B. indem man ein Anilin der Formel VI 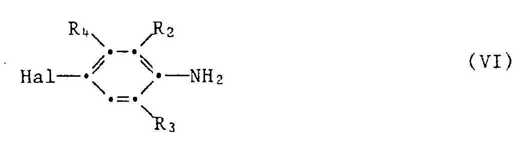 mit einem Phenol der Formel VII 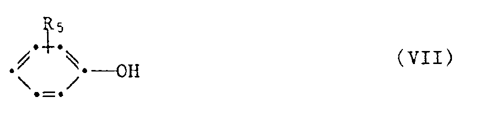 umsetzt, wobei R₂, R₃, R₄ und R₅ die für Formel I angegebene Bedeutung haben und Hal für Halogen, insbesondere für Chlor oder Brom steht.

Das Verfahren zur Herstellung der Verbindungen der Formel V wird zweckmässigerweise in Gegenwart einer organischen oder insbesondere anorganischen Base, wie z.B einem Aklalihydroxid oder -carbonat, und einem gegenüber den Reaktionsteilnehmern inerten, vorzugsweise polaren Lösungs- oder Verdünnungsmittels unter normalem Druck durchgeführt. Die Temperatur beträgt dabei 0 bis +200_{°}C, vorzugsweise Siedetemperaturdes verwendeten Lösungs- oder verdünnungsmittels oder +50 bis 170°C. Als vorteilhaft kann sich auch der Zusatz eines Schwermetall-Katalysators wie z.B. Kupferpulver oder basisches Kupfer-II-carbonat erweisen. Geeignete Lösungs- und Verdünnungsmittel sind u.a. Amide wie Dimethylformamid, Dimethylsulfoxid, N-Methylpyrrolidon und andere polare aprotische Lösungsmittel.

Die Verbindungen der Formeln II und V sind neu und bilden ebenfalls einen Gegenstand der vorliegenden Erfindung. Die Verbindungen der Formeln 111, IV, VI und VII sind dagegen bekannt oder können nach im Prinzip bekannten Methoden hergestellt werden.

Ueberraschenderweise wurde gefunden, dass die erfindungsgemässen Verbindungen der Formel 1 bei günstiger Warmblüter- und Pflanzenverträglichkeit wertvolle Wirkstoffe in der Schädlingsbekämpfung sind. So eignen sich die Verbindungen der Formel z.B. zur Bekämpfung von Schädlingen an Tieren und Pflanzen. Solche Schädlinge gehören hauptsächlich dem Stamm der Arthropoden an, wie insbesondere Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera oder Hymenoptera und Arachniden der Ordnung Acarina, wie z.B. Milben und Zecken. Dabei kann jedes Entwicklungsstadium der Schädlinge bekämpft werden, d.h. sowohl die Adulten, Puppen und Nymphen, als auch insbesondere die Larven und Eier. So können vor allem Larven und Eier von phytopathogenen Schadinsekten und -milben in Zier- und Nutzpflanzungen, wie z.B. in Obst- und Gemüsepflanzungen, und insbesondere in Baumwollpflanzungen, wirkungsvoll bekämpft werden. Werden Verbindungen der Formel von Imagines aufgenommen, so kann sich ihre Wirkung in unmittelbarer Abtötung der Schädlinge zeigen oder aber in verminderter Eiablage und/oder Schlupfrate. Die letztere Erscheinung kann insbesondere bei Coleopteren beobachtet werden. Bei der Bekämpfung von tierparasitären Schädlingen, insbesondere an Haus-und Nutztieren, kommen vor allem Ektroparasiten, wie z.B. Milben und Zecken und Dipteren, wie z.B. Lucilia sericata in Betracht.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel werden in unveränderter Form oder vorzugsweise zusammen mit in der Formulierungstechnik üblichen, inerten und pflanzenverträglichen Hilfsmitteln eingesetzt und können daher z.B. zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen C₈ bis C₁₂, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder-äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxiderte Pflanzenöle, wie epoxidertes Kokosnussöl oder Sojaöl, oder Wasser.

Als feste Trägerstoffe z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsären (C_{1O}-C₂₂), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Aklylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonyphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesätigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthlenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykol-Einheiten.

Als Beispiele nichtionischer Tenside seien Nonylphenolpolyäthoxyäthanole, Ricinusölpolyglykoläther, Ricinusölthioxilat, Polypropylen-Polyäthlenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979;

Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, Wirkstoff der Formel oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbracher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

### Beispiel 1: Herstellung

### 1.1. Zwischenprodukte

### 1.1.1. 4-Aryloxyaniline

### 1.1.1.1. 2,6-Diisopropyl-4-(naphthyl-2-oxy)-anilin

120 g 2-Naphthol werden in 800 ml Xylol gelöst und unter Stickstoff mit 55 g pulverisisertem Kaliumhydroxid versetzt. Das Reaktionsgemisch wird unter Rühren bis zum Siedepunkt erhitzt und das Wasser ausgekreist. Nach Zugabe von 5 g Kupferchlorid und 160 g 2,6-Diisopropyl-4-bromanilin wird das Gemisch 12 Stunden lang bei 150-155°C gerührt, anschliessend abgekühlt und der entstandene Niederschlag abgenutscht. Das Filtrat wird mit einer 15%igen wässrigen Lösung von Natriumhydroxid und zweimal mit je 200 ml Wasser gewaschen. Die organische Phase wird abgetrennt, über Natriumsulfat getrocknet, vom Lösungsmittel befreit und der Rückstand destilliert. Die Titelverbindung der Formel 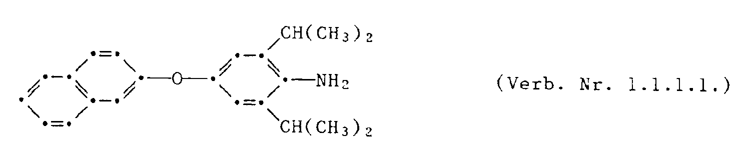 liegt als hellgelbe Flüssigkeit vor; Sdp. 173°C/0,06 Torr.

Auf analoge Weise werden die folgenden Verbindungen hergestellt:

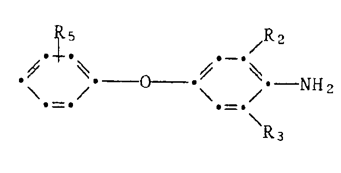

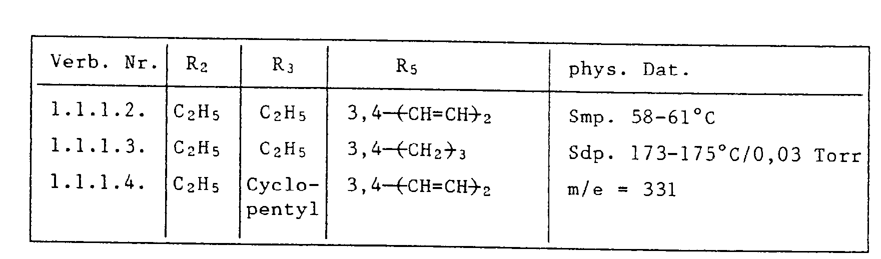

### 1.1.2. Aryloxyphenylisothiocyanate

### 1.1.2.1. 2,6-Diisopropyl-4-(naphthyl-2-oxy)-phenylisothiocyanat

50,0 g 2,6-Diisopropyl-4-(naphthyl-2-oxy)-anilin gelöst in 100 ml Dichlormethan werden unter starkem Rühren tropfenweise bei 0 bis +10_{°}C zu 21,6 g Thiophosgen, 300 ml Dichlormethan, 200 ml Wasser und 31,3 g gemahlenem Calciumcarbonat gegeben. Das Reaktionsgemisch wird 2 Stunden lang bei Raumtemperatur gerührt und dann filtriert. Die organische Phase wird abgetrennt, mit 200 ml Wasser gewaschen, über Natriumsulfat getrocknet und anschliessend eingedampft. Die Titelverbindung der Formel 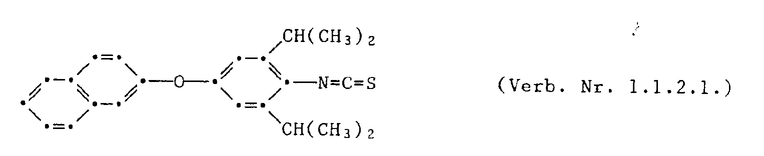 liegt als Rohprodukt in Form eines gelben Oels vor und kann in diesem Zustand weiterverarbeitet werden. Beim Stehenlassen bilden sich Kristalle; Smp. 76-78°C.

Auf analoge Weise werden die folgenden Verbindungen hergestellt:

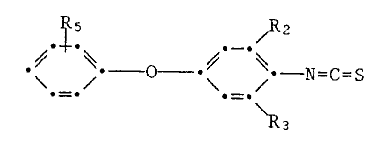

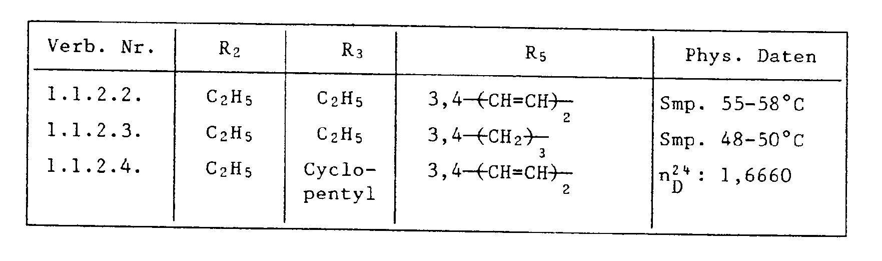

### 1.2. Endprodukte

### 1.2.1. Aryloxyphenylthioharnstoffe

### 1.2.1.1. N-[2,6-Diisopropyl-4-(naphthyl-2-oxy)-phenyl]-N'-tert.butyl-thioharnstoff

25,0 g 2,6-Diisopropyl-4-(naphthyl-2-oxy)-phenylisothiocyanat werden in 100 ml Hexan gelöst und unter Rühren mit 7,7 g tert.Butylamin versetzt. Das Reaktionsgemisch wird 12 Stunden lang bei 20 bis 25°C gerührt. Alsdann wird der entstandene Niederschlag abgenutscht, mit Hexan gewaschen und getrocknet. Die Titelverbindung der Formel 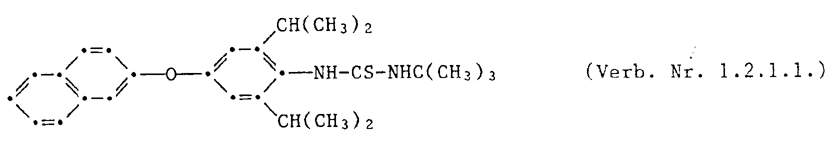 liegt als hellbeiges Pulver vor; Smp. 155°C (Zers.).

Auf analoge Weise werden die folgenden Verbindungen hergestellt:

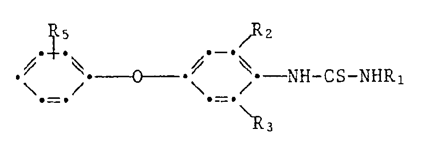

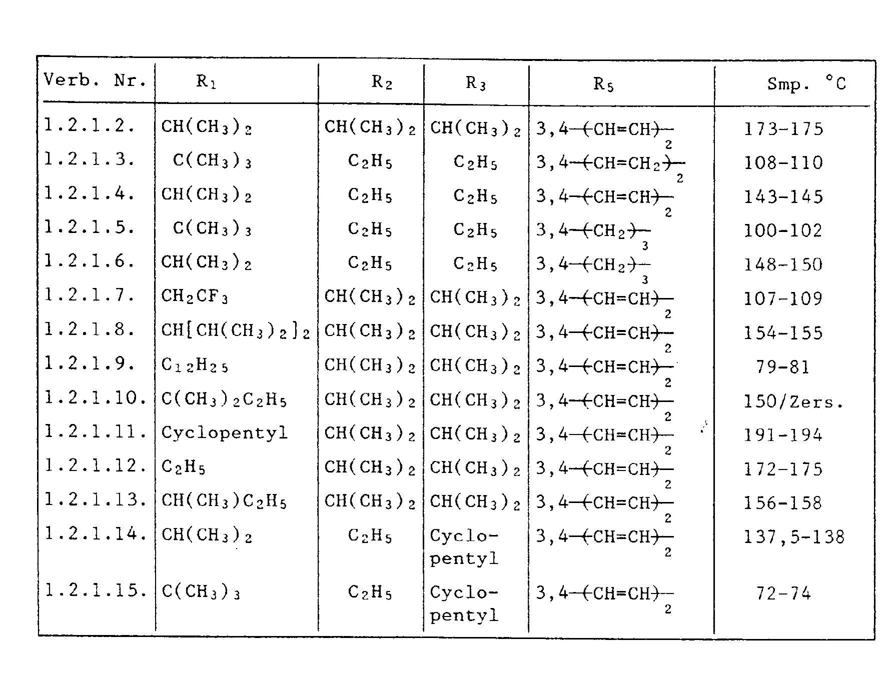

### 1.2.2. Aryloxyphenylisothioharnstoffe

### 1.2.2.1. N-[2,6-Diisopropyl-4-(naphthyl-2-oxy)-phenyl]-N'-tert.butyl-S-methyl-isothioharnstoff

5,0 g N-[2,6-Diisopropyl-4-(naphthyl-2-oxy)-phenyl]-N'-tert.butyl-thioharnstoff werden in 40 ml Aethanol vorgelegt, bei Raumtemperatur mit 2,7 g Methyljodid versetzt und während 6 Stunden unter leichtem Rückfluss gerührt. Anschliessend wird das Lösungsmittel abgedampft und der Rückstand in 80 ml Dichlormethan und 40 ml 10%iger wässriger Sodalösung aufgenommen. Die organische Phase wird abgetrennt, über Natriumsulfat getrocknet und vom Lösungsmittel befreit. Die Rückstand wird in Hexan verrührt, abfiltriert und getrocknet. Die Titelverbindung der Formel 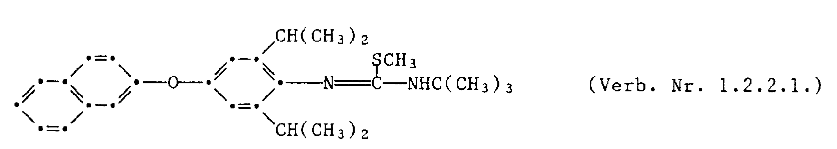 liegt als hellbeiges Pulver vor; Smp. 102-103°C.

Auf analoge Weise werden die folgenden Verbindungen hergestellt:

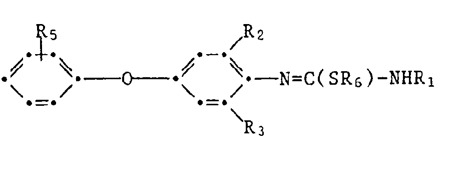

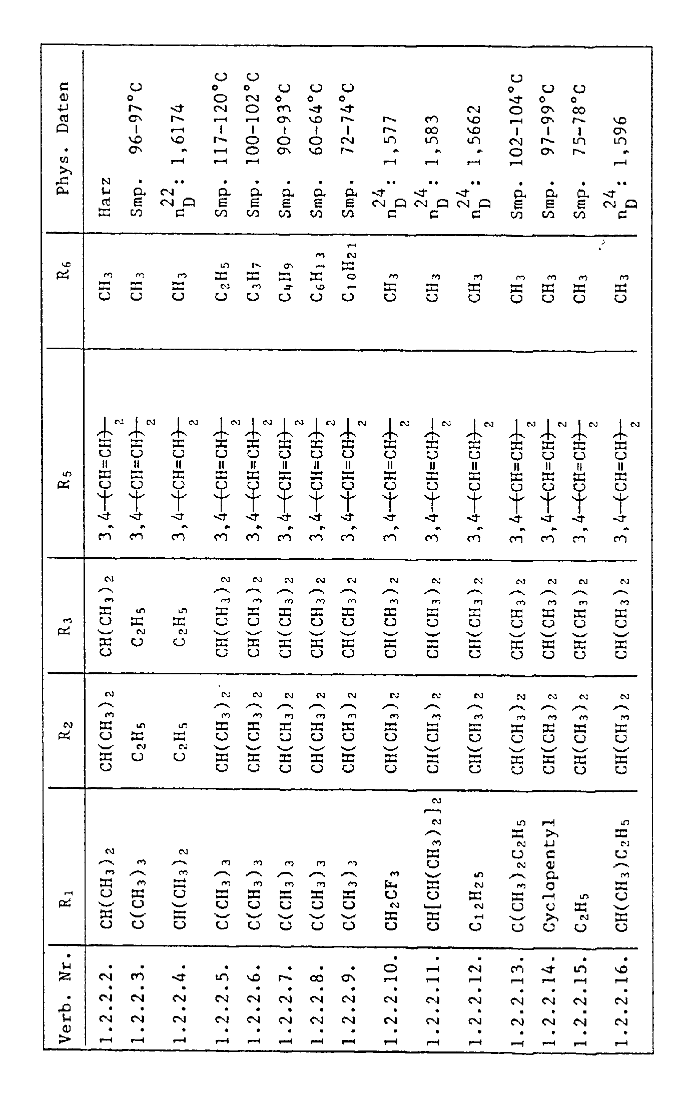

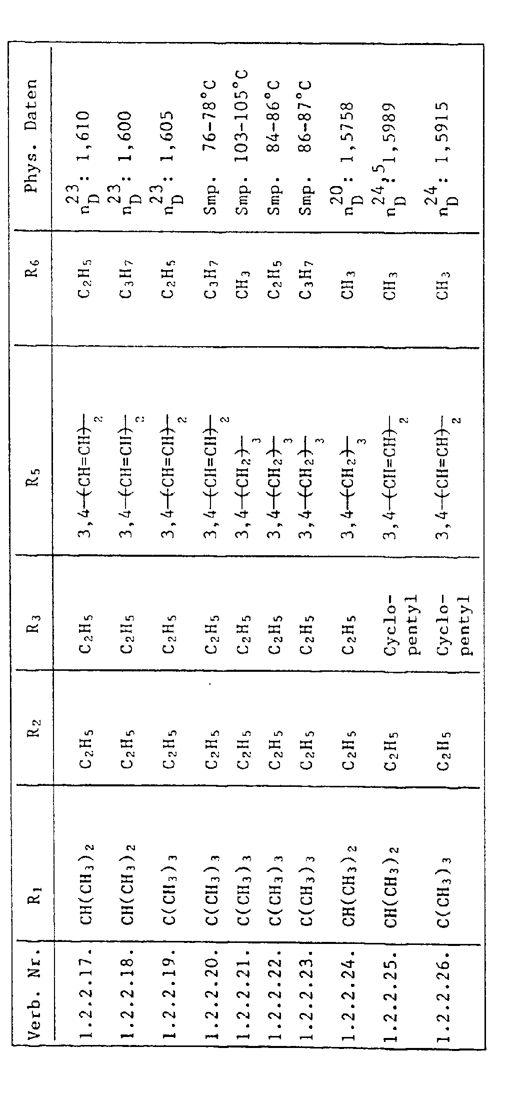

### 1.2.3.1. N-[2,6-Diisopropyl-4-(naphthyl-2-oxy)-phenyl]-N'-tert.butyl-S-methyl-isothioharnstoff 4-Toluolsulfonsäuresalz

5,0 g N-[2,6-Diisopropyl-4-(naphthyl-2-oxy)-phenyl]-N'-tert.butyl-S-methyl-isothioharnstoffwerden in 20 ml absolutem Diäthyläther gelöst und tropfenweise mit 2,2 g 4-Toluolsulfonsäure, gelöst in 10 ml Diäthyläther versetzt. Die Reaktionslösung wird 3 Stunden lang bei Raumtemperatur nachgerührt. Der entstandene Niederschlag wird abgenutscht und getrocknet. Die Titelverbindung der Formel 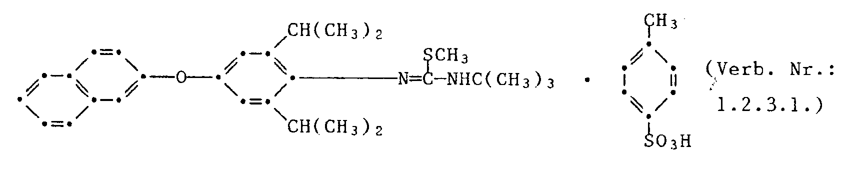 liegt als weisses kristallines Pulver vor; Smp. 180°C/Zers.

Auf analoge Weise werden die folgenden Verbindungen hergestellt:

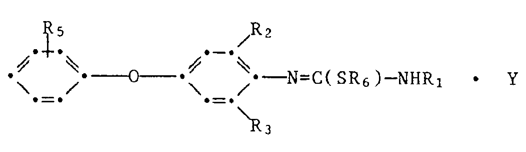

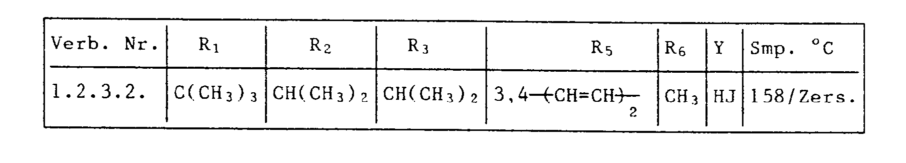

### 1.2.4. Aryloxyphenylcarbodiimide

### 1.2.4.1. N-[2,6-Diisopropyl-4-(naphthyl-2-oxy)-phenyl]-N'-tert.butyl-carbodiimid

5,0 g N-[2,6-Diisopropyl-4-(naphthyl-2-oxy)-phenyl]-N'-tert.butyl-thioharnstoff und 2,9 g 2-Chlor-1-methyl- pyridiniumjodid werden in 40 ml Acetonitril vorgelegt, bei Raumtemperatur tropfenweise unter Rühren mit 2,5 g Triäthylamin in 10 ml Acetonitril versetzt und 2 Stunden lang bei 70°C gerührt. Anschliessend wird das Reaktionsgemisch am Rotationsverdampfer bei 50°C eingedampft und der Rückstand in 50 ml Hexan und 30 ml kaltem Wasser aufgenommen. Die Hexanphase wird abgetrennt, mit 20 ml kaltem Wasser gewaschen, über Natriumsulfat getrocknet und schliesslich eingedampft. Die Titelverbindung der Formel 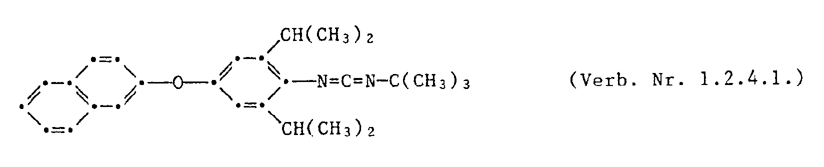 liegt als hellbraunes Oel vor, aus dem sich beim Stehenlassen Kristalle bilden; Smp. 48-51°C.

Auf analoge Weise werden die folgenden Verbindungen hergestellt:

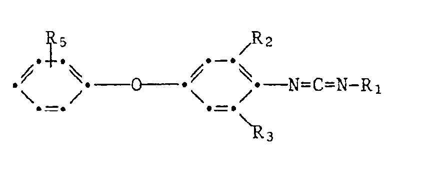

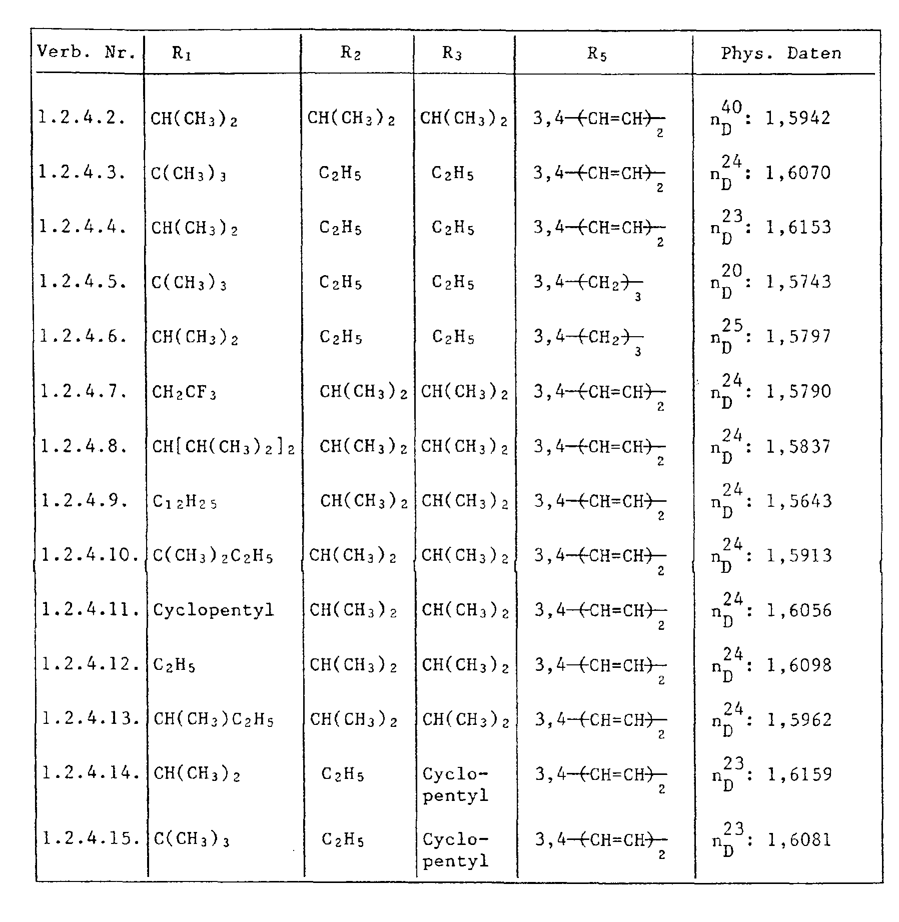

### Beispiel 2: Formulierungen von Wirkstoffen der Formel I gemäss den Herstellungsbeispielen 1.2.

(% = Gewichtsprozent)

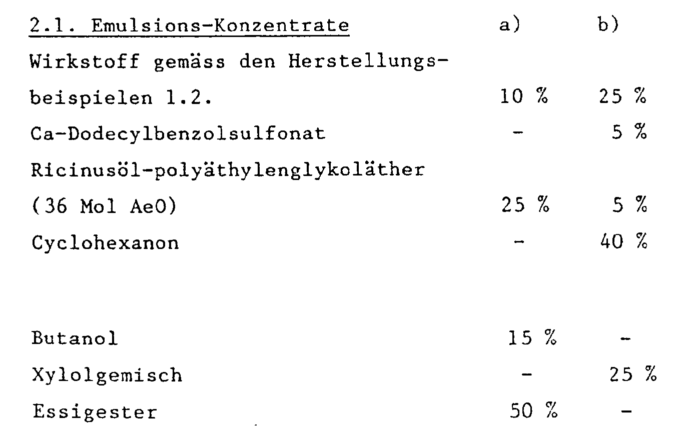

Aus solchen Konzentraten können durch Verdünnen mit Wasser Emulsionen jeder gewünschten Konzentration hergestellt werden.

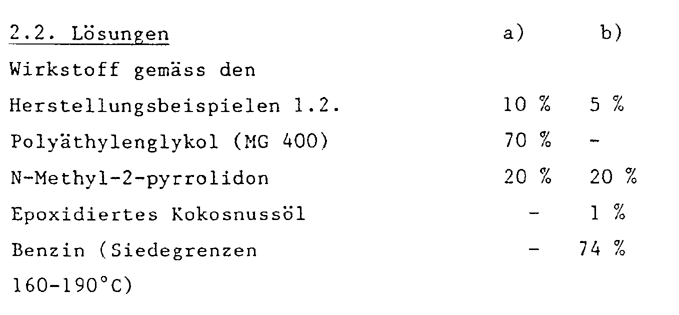

Die Lösungen sind zur Anwendung in Form kleinster Tropfen geeignet.

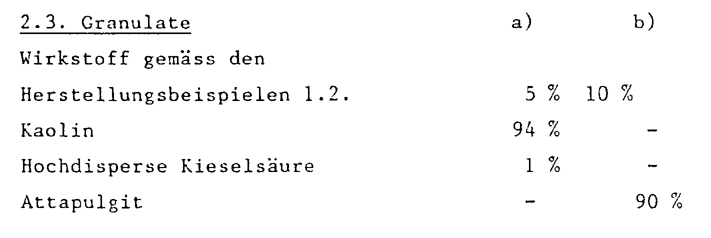

Der Wirkstoff wird in Mehylenchlorid gelöst, auf den Träger aufgesprüht und das Lösungsmittel anschliessend im Vakuum abgedampft.

### 2.4. Extruder Granulat

Wirkstoff gemäss den

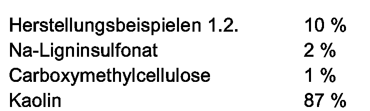

Der Wirkstoff wird mit den Zusatzstoffen vermischt, vermahlen und mit Wasser angefeuchtet. Dieses Gemisch wird extrudiert und anschliessend im Luftstrom getrocknet.

### 2.5. Umhüllungs-Granulat

### Wirkstoff gemäss den

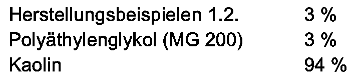

Der feingemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

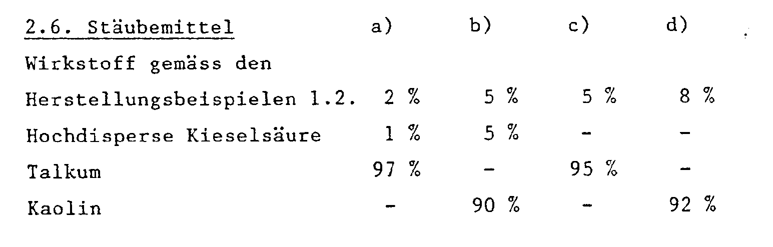

Durch inniges Vermischen der Trägerstoffe mit dem Wirkstoff und gegebenenfalls Vermahlen auf einer geeigneten Mühle erhält man gebrauchsfertige Stäubemittel.

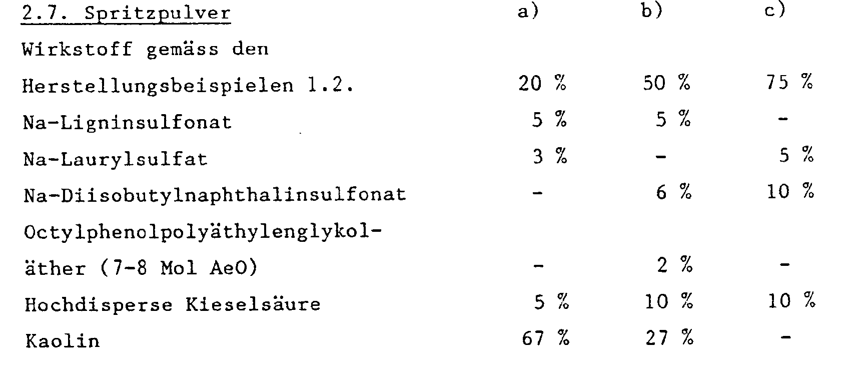

Der Wirkstoff wird mit den Zusaztstoffen gut vermischt und in einer geeigneten Mühle vermahlen. Man erhält Spritzpulver, die sich mit Wasser zu Suspensionen jeder gewünschten Konzentration verdünnen lassen.

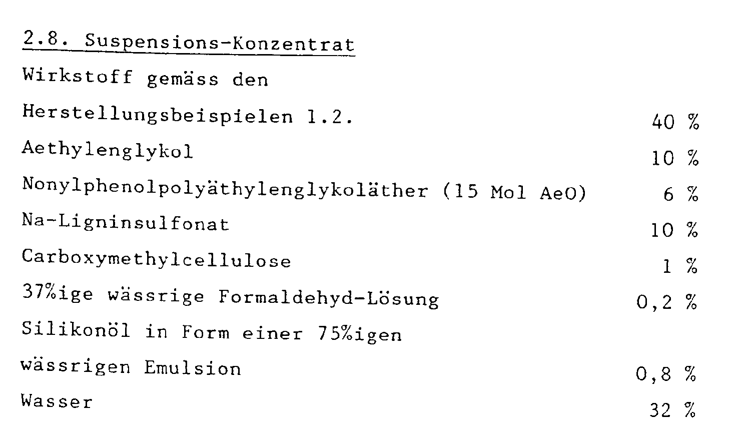

Der fein gemahlene Wirkstoff wird mit den Zusatzstoffen innig vermischt. Man erhält so ein SuspensionsKonzentrat, aus welchem durch Verdünnen mit Wasser Suspensionen jeder gewünschten Konzentration hergestellt werden können.

### Beispiel 3: Biologische Prüfungen

### 3.1. Wirkung gegen Musca domestica

Ein Zuckerwürfel wird derart mit einer Lösung der Testsubstanz befeuchtet, dass die Konzentration des Wirkstoffes im Würfel nach dem Trocknen 500 ppm beträgt. Der so behandelte Würfel wird zusammen mit einem nassen Wattebausch auf eine Schale gelegt und mit einem Becherglas zugedeckt. Unter das Becherglas werden 10 adulte, eine Woche alte und OP-resistente Fliegen gegeben und bei 25°C und 50 % Luftfeuchtigkeit belassen. Nach 24 Stunden wird die insektizide Wirkung durch Ermittlung der Abtötungsrate festgestellt.

Verbindungen gemäss den Beispielen 1.2. zeigen in diesem Test gute Wirkung.

### 3.2. Wirkung gegen Lucilia sericata

Zu 9 ml eines Zuchtmediums wird bei 50°C I ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen gemäss den Beispielen 1.2. zeigen in diesem Test gute Wirkung gegen Lucilia sericata.

### 3.3. Wirkung gegen Zecken in verschiedenen Entwicklungsstadien

10 frische, vollgesogene Boophilus microplus Weibchen werden in einer Reihe dorsal auf einer PVC Platte aufgeklebt und mit einem Wattebausch überdeckt. Danach werden die Testtiere mit 10 ml der wässrigen Testlösung übergossen. Eine Stunde später wird der Wattebausch entfernt und die Zecken über Nacht bei 24°C getrocknet. Nach dem Trocknen werden die Zecken 4 Woche lang bis zum Ende der Eiablage und Beginn des Larvenschlupfes bei 28°C und 80 % Luftfeuchtigkeit gehalten.

Jede Testsubstanz wird in einer Konzentration von 500 ppm getestet. Die akarizide Wirkung zeigt sich entweder beim Weibchen als Mortalität oder Sterilität oder im Eigelege durch Blockierung der Embryogenese oder des Schlüpfaktes. Alle Substanzen werden gegen zwei verschiedene Zeckenstämme, den OP-resistenten Stamm BIARRA und den Amidin-resistenten Stamm ULAM geprüft.

Verbindungen gemäss den Beispielen 1.2. zeigen gute Wirkung im obigen Test.

### 3.4. Frassgift-Wirkung auf Spodoptera littoralis-Larven (L₁)

Baumwollpflanzen im Keimblattstadium werden mit einer wässrigen Wirkstoffemulsion (erhalten aus einem 10%igen emulgierbaren Konzentrat) besprüht, wobei die Wirkstoffemulsion 400 ppm der zu prüfenden Verbindung enthält.

Nach dem Antrocknen des Belages wird jede Baumwollpflanze mit Spodoptera littoralis-Larven im ersten larvelen Stadium besetzt. Der Versuch wird bei 26°C und ca. 50 % relativer Luftfeuchtigkeit durchgeführt. Nach 2 und 3 Tagen wird die Mortalität und nach 5 Tagen werden Entwicklungs- und Häutungsstörungen der angesetzten Larven bestimmt.

Verbindungen gemäss den Beispielen 1.2. zeigen gute Wirkung in diesem Test.

### 3.5. Frassgift-Wirkung auf Spodoptera littoralis- und Heliothis virescens-Larven (L₃)

Eingetopfte Sojapflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen werden die behandelten Sojapflanzen mit je 10 Larven von Spodoptera littoralis und Heliothis virescens im dritten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

### 3.6. Frassgift-Wirkung auf Plutella xylostella- und Crocidolomia binotalis-Larven (L₂)

Eingetopfte Chinakohlpflanzen (Topfgrösse 10 cm Durchmesser) im 4-Blatt-Stadium werden mit wässrigen Wirkstoffemulsionen besprüht, die den Wirkstoff in einer Konzentration von 400 ppm enthalten.

Nach zwei Tagen wird jede behandelte Chinakohlpflanze mit 10 Plutella xylostella- oder Croccidolomia binotalis-Larven im zweiten larvalen Stadium besiedelt. Der Versuch wird bei 26°C und ca. 60 % relativer Luftfeuchtigkeit im Dämmerlicht durchgeführt. Die Bonitur erfolgt nach 2 und 5 Tagen; es wird die Mortalität der Larven in Prozenten bestimmt.

Verbindungen gemäss den Beispielen 1.2. zeigen 80-100%ige Wirkung (Mortalität).

### 3.7. Kontaktwirkung gegen Nilaparvata lugens (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 40 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglaszylinder gestülpt und dieser mit einem Gaze-Deckel abgedeckt. Die Nymphen werden für 6 Tage an der behandelten Pflanze, die mindestens 1 mal nachgegossen werden muss, gehalten. Der Versuch wird bei einer Temperatur von ca. 23°C, bei 55 % relativer Luftfeuchtigkeit und mit einer Beleuchtungsperiode von 16 Stunden durchgeführt.

Verbindungen gemäss den Beispielen 1.2. zeigen in diesem Test gute Wirkung.

### 3.8. Systemische Wirkung auf Nilaparvata lugens

Etwa 10 Tage alte Reispflanzen (ca. 10 cm hoch) werden in einen Plastik-Becher eingestellt, der 20 ml einer wässrigen Emulsions-Zubereitung des zu prüfenden Wirkstoffes in einer Konzentration von 100 ppm enthält und mit einem Löcher aufweisenden Plastikdeckel abgeschlossen ist. Die Wurzel der Reispflanze wird durch ein Loch in dem Plastikdeckel in die wässrige Test-Zubereitung geschoben. Das Loch wird mit Watte abgedichtet, um die Pflanze zu fixieren und den Einfluss der Gasphase aus der Test-Zubereitung auszuschalten. Dann wird die Reispflanze mit 20 Nymphen von Nilaparvata lugens im N₂-bis N₃- Stadium besiedelt und mit einem Plastikzylinder abgedeckt. Der Versuch wird bei 26°C und ca. 60 % relativer Luftfeuchtigkeit mit einer Beleuchtungsperiode von 16 Stunden durchgeführt. Nach zwei und fünf Tagen wird auf die Anzahl der abgetöteten Testtiere, im Vergleich zu unbehandelten Kontrollen, bonitiert. Damit wird festgestellt, ob der über die Wurzel aufgenommene Wirkstoff, die Testtiere an den oberen Pflanzenteilen abtötet.

Verbindungen gemäss den Beispielen 1.2. zeigen im obigen Test 80 - 100%ige Wirkung (Mortalität) gegen Nilaparvata lugens.

### 3.9. Wirkung gegen Bodeninsekten (Diabrotica balteata)

Es werden 350 ml Erde (bestehend aus 95 Vol.-% Sand und 5 Voi.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Duchmeservon ca. 10 cm haben, mit der so behandelten Erde teilweise gefüllt. Pro Becher werden zehn Larven von Diabrotica balteata im dritten Larvalstadium eingesetzt, view Maiskeimlinge eingepflanzt und die Becher mit Erde aufgefüllt. Die gefüllten Becher werden mit Plastikfolie abgedeckt und bei einer Temperatur von etwa 24°C und einer relativen Luftfeuchtigkeit von ca. 50 % gehalten. Sechs Tage nach dem Ansatz wird die in den Bechern enthaltene Erde abgesiebt und die zurückbleibenden Larven werden auf ihre Mortalität geprüft.

Verbindungen gemäss des Beispielen 1.2. zeigen gute Wirkung im obigen Test.

### 3.10. Kontaktwirkung auf Aphis craccivora

In Töpfen angezogene 4-5 Tage alte Erbsenkeimlinge (Pisum sativum) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe direkt besprüht. Man verwendet pro Test-Verbindung zwei Pflanzen. Eine Auswertung der erzielten Abtötungsrate erfolgt nach 3 und 5 Tagen. Der Versuch wird bei ca. 21 °C und einer relativen Luftfeuchtigkeit von etwa 55 % durchgeführt.

Verbindungen gemäss den Beispielen 1.2. zeigen gute Wirkung in diesem Test.

### 3.11. Kontaktwirkung auf Myzus persicae

Etwa 4 bis 5 Tage alte, in Wasser angezogene Erbsenkeimlinge (Pisum sativum) werden vor Versuchsbeginn jeweils mit ca. 200 Individuen der Spezies Myzus persicae besiedelt. Die so behandelten Pflanzen werden 24 Stunden später mit einer wässrigen Suspension enthaltend 100 ppm der zu prüfenden Verbindung bis zur Tropfnässe direkt besprüht. Man verwendet pro Testsubstanz zwei Pflanzen. Eine Auswertung der erzielten Abtötungsrate erfolgt 3 und 5 Tage nach Applikation. Der Versuch wird bei ca. 21°C und etwa 60 % relativer Luftfeuchtigkeit durchgeführt.

Verbindungen gemäss den Beispielen 1.2. zeigen gute Wirkung in diesem Test.

### 3.12. Wirkung gegen Tetranychus urticae (OP-sensibel)

Die Primärblättervon Phaseolus vulgaris-Pflanzen werden 24 Stunden vor dem Versuch auf akarizide Wirkung mit einem infestierten Blattstück aus einer Massenzucht von Tetranychus urticae (OP-sens.) belegt (Mischpopulation). Die Toleranz bezieht sich auf die Verträglichkeit gegenüber Diazinon.

Die so behandelten infestierten Pflanzen werden mit einer Versuchslösung in Emulsionsform enthalten 400 ppm der zu prüfenden Verbindung bis zur Tropfnässe besprüht. Während des Versuchsverlaufs stehen die Pflanzen in Gewächshauskabinen bei ca. 25°C mit etwa 50 % rel. Luftfeuchtigkeit.

Nach 6 Tagen werden Imagines und Larven (alle beweglichen Stadien) sowie abgelegte Eier unter dem Binokular auf lebende und tote Individuen ausgewertet.

Verbindungen gemäss den Beispielen 1.2. zeigen in diesem Versuch gute Wirkung.

### 3.13. Wirkung gegen Panonychus ulmi (OP- und Carbamat-resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 100 ppm der zu prüfenden Verbindung bis zur Tropfnässe besprüht. Die behandelten Pflanzen werden dann während 14 Tagen bei ca. 25°c und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen gemäss den Beispielen 1.2. zeigen gute Wirkung im obigen Test.

### 3.14. Wirkung gegen Anthonomus grandis (Adulte)

Zwei eingetopfte Baumwollpflanzen im 6-Blattstadium werden mit wässrigen benetzungsfähigen Emulsions-Zubereitungen, enthaltend 100 ppm des zu prüfenden Wirkstoffes, besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit Testtieren besiedelten Pflanzen gestülpt, um ein Abwandern der Käfer zu verhindern. Die behandelten Pflanzen werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen gemäss den Beispielen 1.2. zeigen gute Wirkung in diesem Test.

### 3.15. Wirkung gegen Dermanyssus gallinae

In einem nach oben offenen Glasbehälter werden 2 bis 3 ml einer 100 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben durch Auszählen der toten Individuen ermittelt und in Prozenten angegeben.

Verbindungen gemäss den Beispielen 1.2. zeigen 80-100%ige Wirkung (Mortalität).

## Patentansprüche (Patentansprüche für folgende(n) Vertragsstaat(en) : AT, BE, CH, DE, FR, GB, GR, IT, LI, NL)

1. Verbindungen der Formel 1 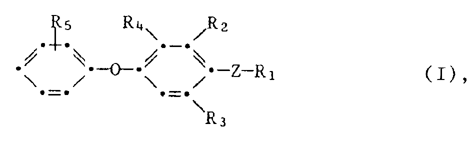 worin n
R1 für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine (CH=CH)₂, (CH₂)₃, 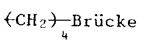 in 2,3- oder 3,4-Stellung;
Z für -NH-CS-NH-, -N=C(SRₛ)-NH- oder -N=C=N- und
R₆ für C₁-C₁₀-Alkyl oder Allyl stehen, sowie deren Salze mit organischen oder anorganischen Säuren.

2. Verbindungen der Formel I gemäss Anspruch 1, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder Cyclopentyl; R₄ für Wasserstoff; R₅ für eine (CH=CH)₂, (CH₂)₃ oder 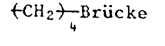 in 2,3-oder 3,4-Stellung; Z für-NH-CS-NH-, -N=C(SR₆)-NH-oder-N=C=N-; und R₆ für C₁-C₅-Alkyl oder Allyl stehen.

3. Verbindungen der Formel gemäss Anspruch 2, worin R₁ für C₁-C₄-Alkyl; R₂ und R₃ je für C₁-C₃-Alkyl; R₄ für Wasserstoff; R₅ für eine (CH=CH)₂ oder 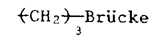 in 3,4-Stellung; und Z für -NH-CS-NH-stehen.

4. Verbindungen der Formel gemäss Anspruch 2, worin R₁ für C₁-C₄-Alkyl; R₂ und R₃ je für C₁-C₃-Alkyl; R₄ für Wasserstoff; R₅ für eine (CH=CH)₂ oder 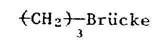 in 3,4-Stellung; Z für -N=C(SR₆)-NH-; und R₆ für C₁-C₃-Alkyl stehen.

5. Verbindungen der Formel gemäss Anspruch 2, worin R₁ für C₁-C₄-Alkyl; R₂ und R₃ je für C₁-C₃-Alkyl; R₄ für Wasserstoff; R₅ für eine (CH=CH)₂ oder 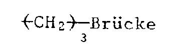 in 3,4-Stellung; und Z für -N=C=N- stehen.

6. Die Verbindungen gemäss einem der Ansprüche 1 bis 3 der Formeln 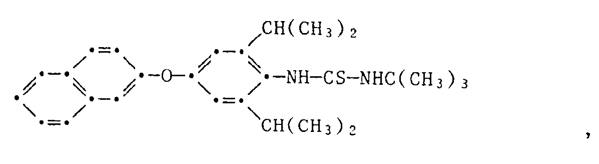 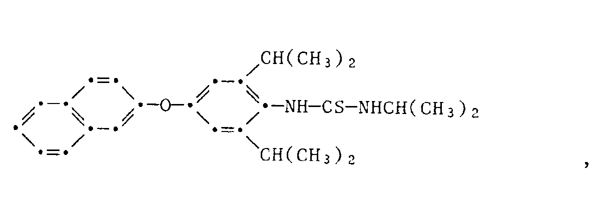 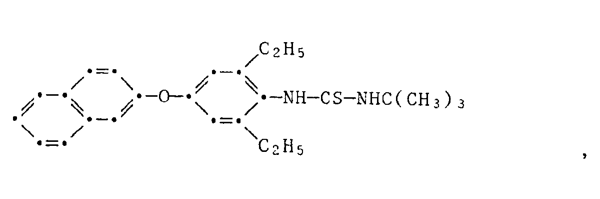 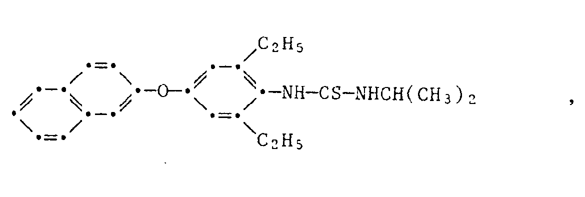 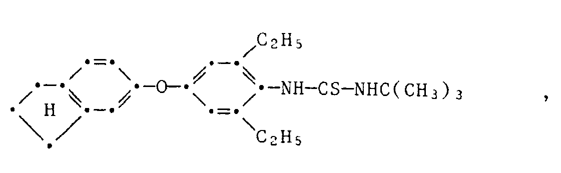 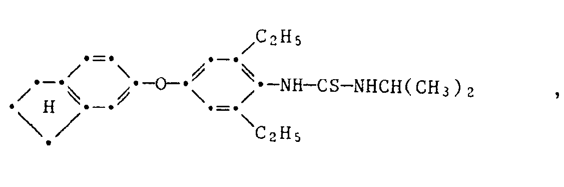 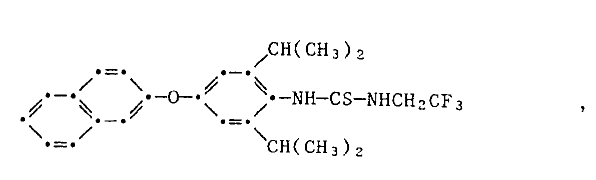 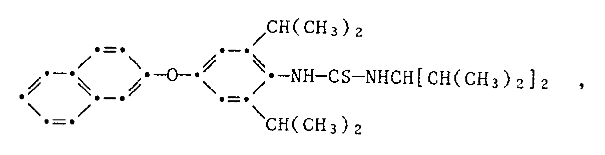 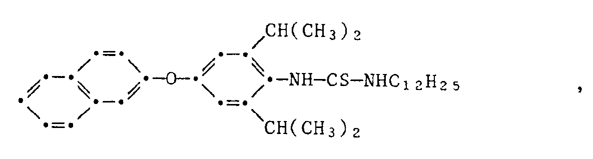 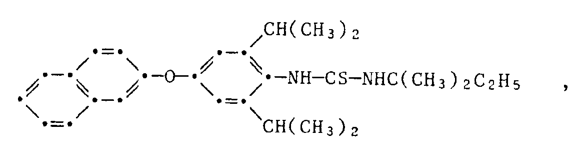 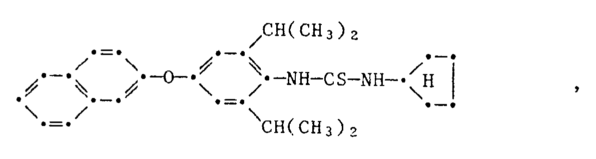 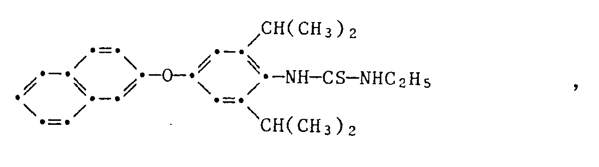 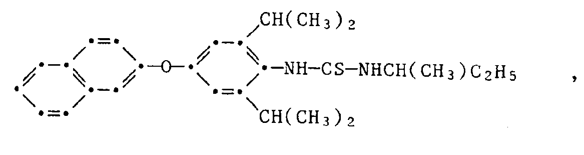 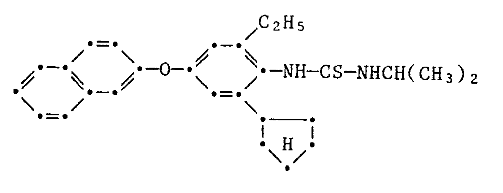 und

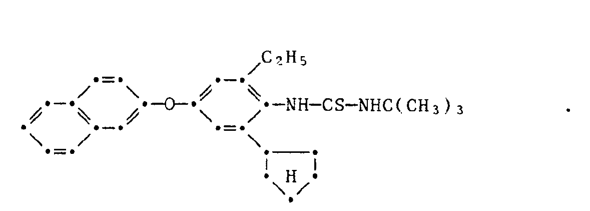

7. Die Verbindungen gemäss einem der Ansprüche 1, 2 oder 4 der Formeln 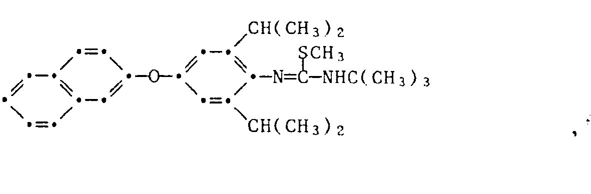 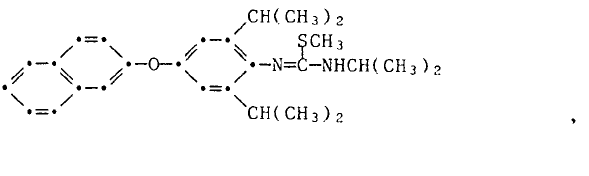 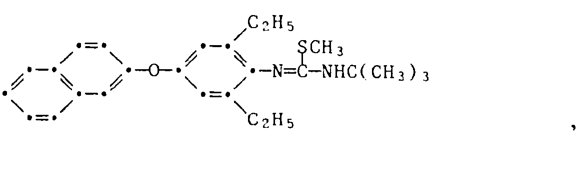 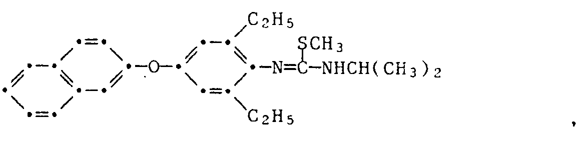 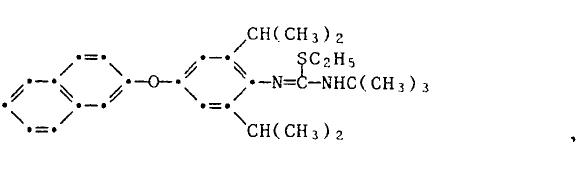 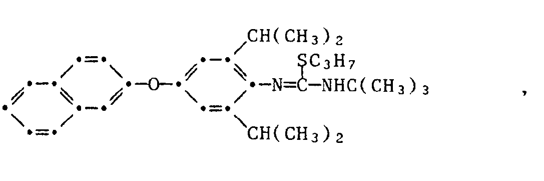 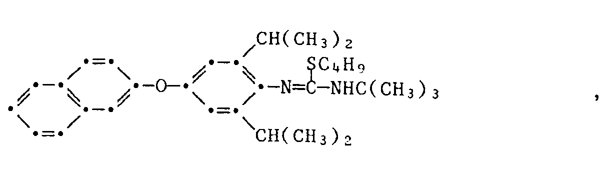 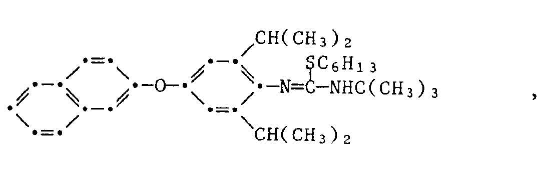 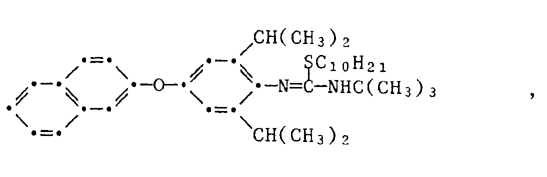 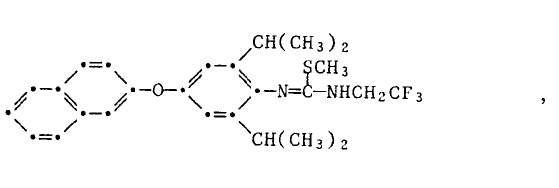 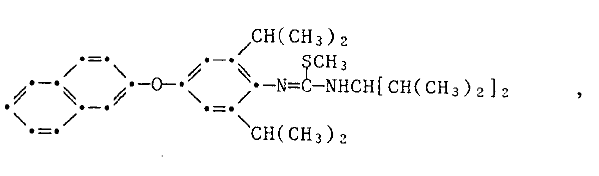 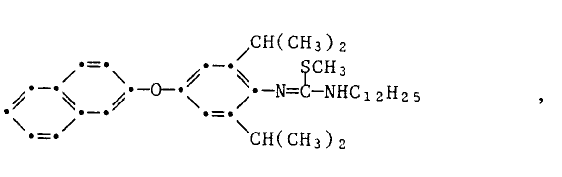 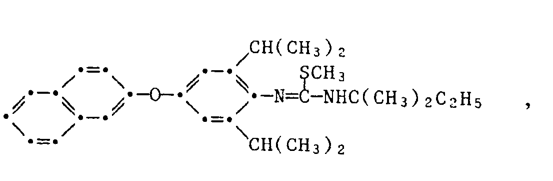 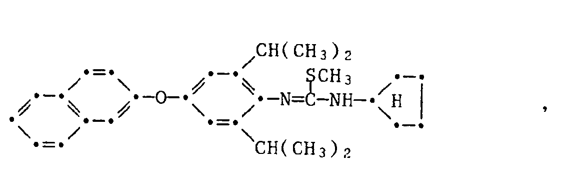 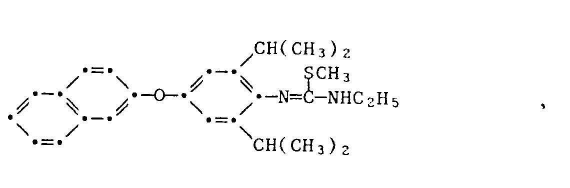 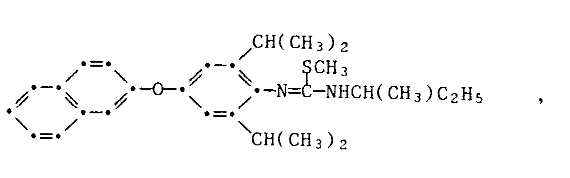 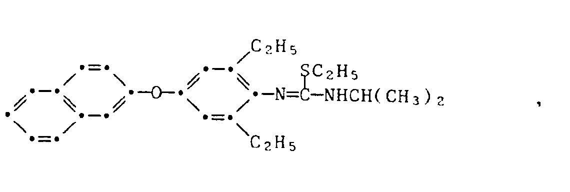 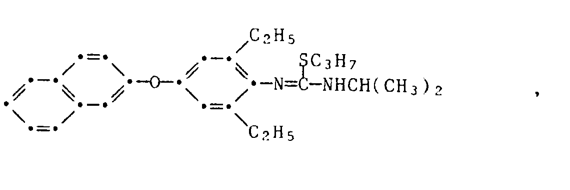 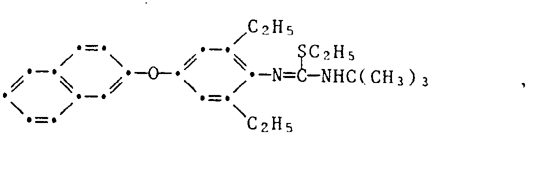 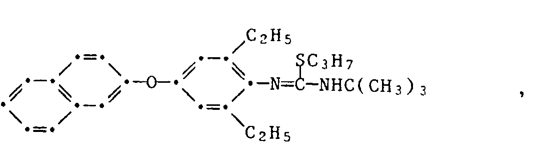 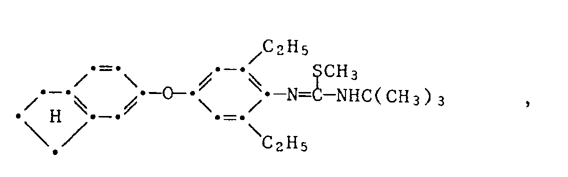 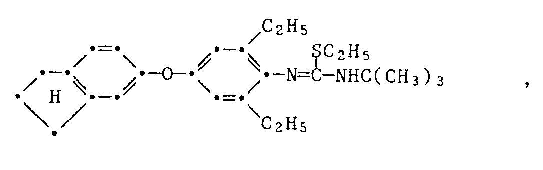 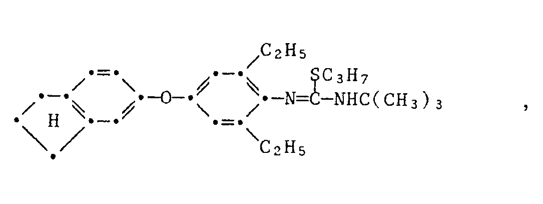 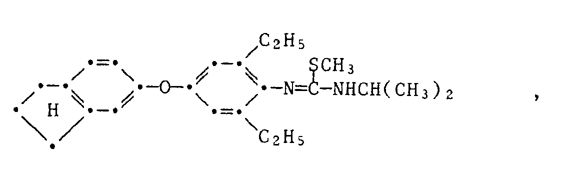 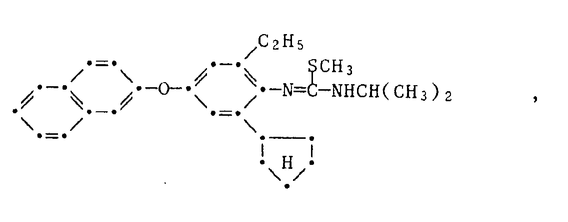 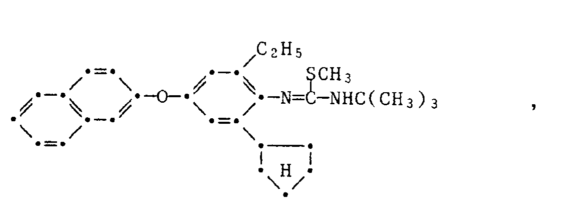 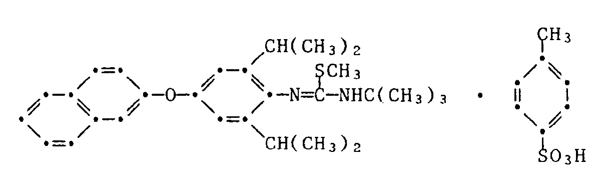 und

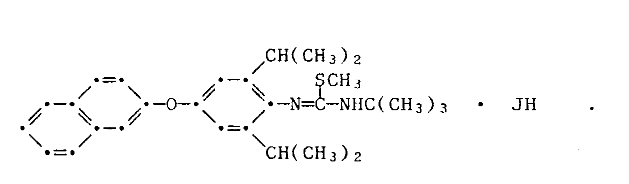

8. Die Verbindungen gemäss einem der Ansprüche 1, 2 oder 5 der Formeln 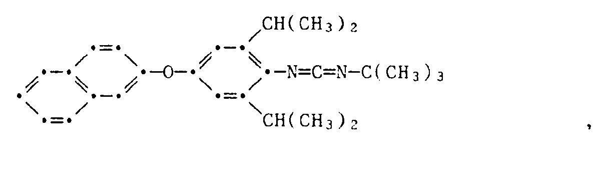 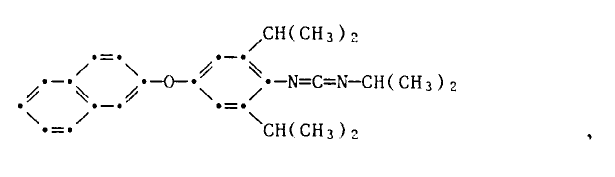 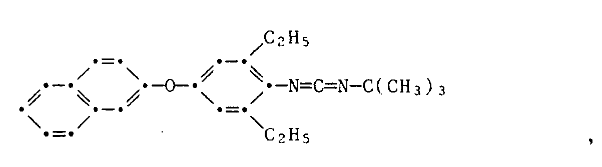 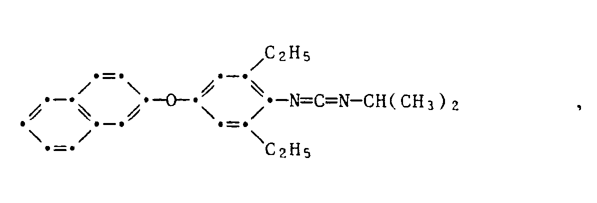 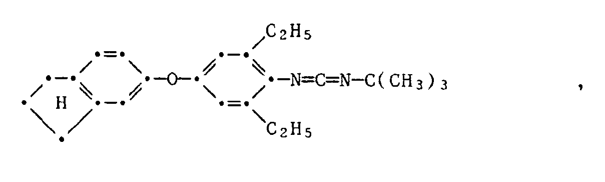 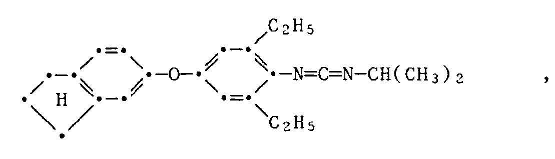 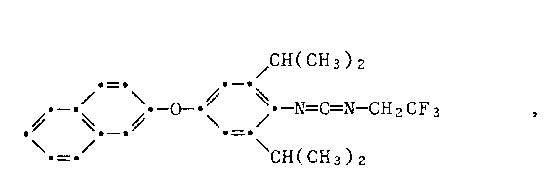 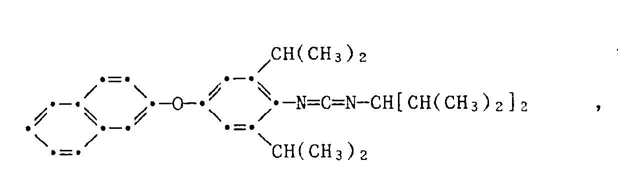 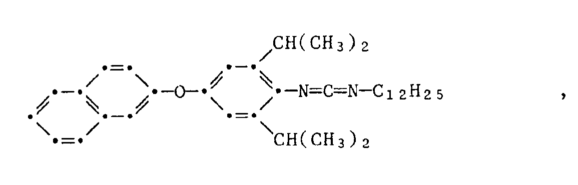 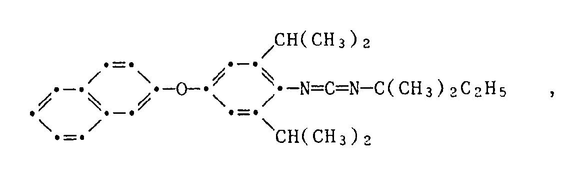 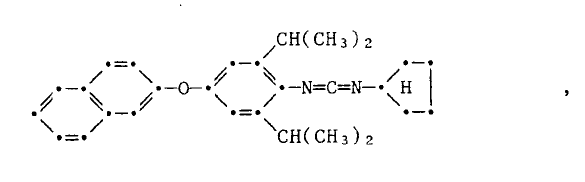 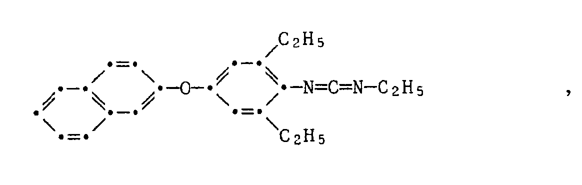 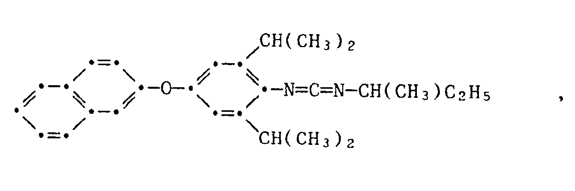 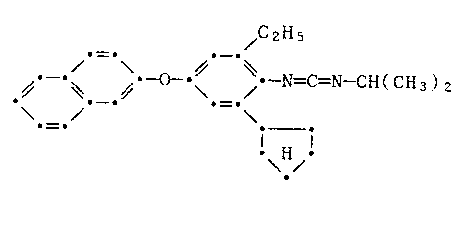 und

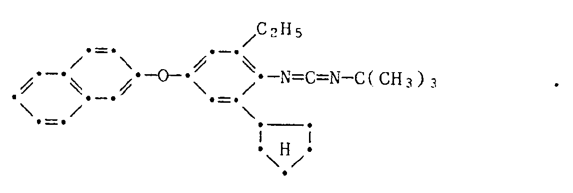

9. Verfahren zur Herstellung einer Verbindung der Formel I 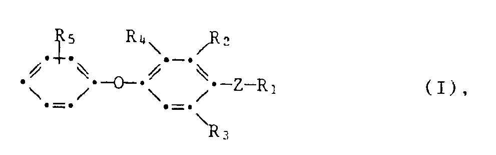 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine (CH=CH)₂, (CH₂)₃, 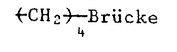 in 2,3- oder 3,4-Stellung;
Z für -NH-CS-NH-, -N=C(SR₆)-NH- oder -N=C=N- und R₆ für C₁-C₁₀-Alkly oder Allyl
stehen, dadurch gekennzeichnet, dass man
A) ein Isothiocyanat der Formel II 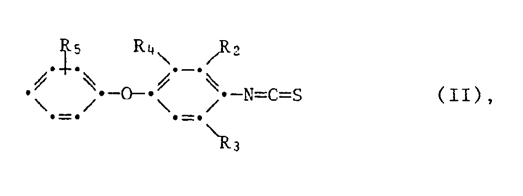 mit einem Amin der Formel III 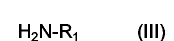 in einem organischen Lösungs- oder Verdünnungsmittel bei Normaldruck und einer Temperatur von 0 bis +150_{°}C zum Thioharnstoff und gegebenenfalls
B) den erhaltenen Thioharnstoff mit einer Verbindung der Formel IV 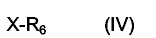 in einem inerten organischen Lösungsmittel unter leicht erhöhtem oder normalem Druck und bei einer Temperatur von +10 bis 250°C zum Isothioharnstoff umsetzt oder
C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150_{°}C in das Carbodiimid überführt, wobei R_{1'} R₂, R₃, R₄, R₅ und R₆ die angegebene Bedeutung haben und X für eine Abgangsgruppe steht.

10. Schädlingsbekämpfungsmittel, welches als aktive Komponente mindestens eine Verbindung der Formel 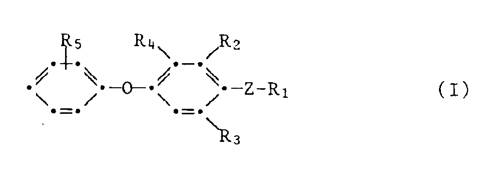 enthält, worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein-oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine (CH=CH)₂, (CH₂)₃, 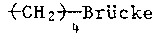 in 2,3- oder 3,4-Stellung;
Z für -NH-CS-NH-, -N=C(SRₛ)-NH- oder -N=C=N- und
R₆ für C₁-C₁₀-Alkyl oder Allyl stehen, oder eines ihrer Salze mit einer organischen oder anorganischen Säure zusammen mit geeigneten Trägern und/oder Zuschlagstoffen.

11. Schädlingsbekämpfungsmittel gemäss Anspruch 10, welches als aktive Komponente mindestens eine Verbindung der Formel enthält, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder Cyclopentyl; R₄ für Wasserstoff; R₅ für eine (CH=CH)₂, (CH₂)₃ oder 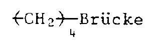 in 2,3- oder 3,4-Stellung; Z für -NH-CS-NH-, -N=C(SR₆)-NH- oder -N=C=N-; und R₆ für C₁-C₅-Alkyl oder Allyl stehen.

12. Schädlingsbekämpfungsmittel gemäss Anspruch 11, worin R₁ für C₁-C₄-Alkyl; R₂ und R₃ je für C₁-C₃-Alkyl; R₄ für Wasserstoff; R₅ für eine -(CH=CH)₂ oder 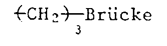 in 3,4-Stellung; und Z für -NH-CS-NH- stehen.

13. Schädlingsbekämpfungsmittel gemäss Anspruch 11, worin R₁ für C₁-C₄-Alkyl; R₂ und R₃ je für C₁-C₃-Alkyl; R₄ für Wasserstoff; R₅ für eine (CH=CH)₂ oder 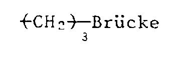 in 3,4-Stellung; Z für -N=C(SR₆)-NH-; und R₆ für C₁-C₃-Alkyl stehen.

14. Schädlingsbekämpfungsmittel gemäss Anspruch 11, worin R₁ für C₁-C₄-Alkyl; R₂ und R₃ je für C₁-C₃-Alkyl; R₄ für Wasserstoff; R₅ für eine (CH=CH)₂ oder 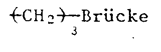 in 3,4-Stellung; und Z für -N=C=N- stehen.

15. Verwendung einer Verbindung der Formel I 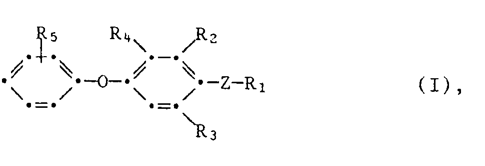 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine (CH=CH)₂, (CH₂)₃, 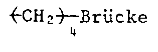 in 2,3- oder 3,4-Stellung;
Z für -NH-CS-NH-, -N=C(SR₆)-NH- oder -N=C=N- und
R₆ für C₁-C₁₀-Alkyl oder Allyl,
stehen, oder eines ihrer Salze mit einer organischen oder anorganischen Säure zur Bekämpfung von Schädlingen an Tieren und Pflanzen.

16. Verwendung gemäss Anspruch 15 zur Bekämpfung von Insekten und Arachniden.

17. Verfahren zum Bekämpfen von Schädlingen an Tieren und Pflanzen, dadurch gekennzeichnet, dass man die Schädlinge in ihren verschiedenen Entwicklungsstadien mit einer Verbindung der Formel 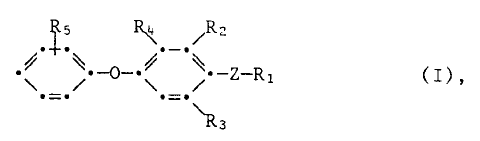 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine -(CH=CH)₂, -(CH₂)₃. 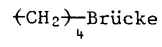
in 2,3- oder 3,4-Stellung;
Z für -NH-CS-NH-, -N=C(SR₆)-NH- oder -N=C=N- und
R₆ für C₁-C₁₀-Alkyl oder Allyl,
stehen, oder einem ihrer Salze mit einer organischen oder anorganischen Säure in Kontakt bringt.

18. Verbindung der Formel V 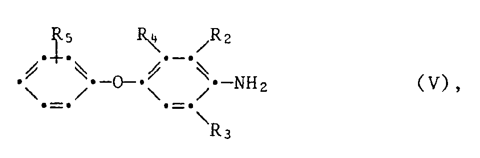 worin n
R₂ für Wasserstoff oder C₁-C₅-Alkyl;
R₃ für C₁-C₅-Alkyl oder C₅-C₆-Cycloalkyl;
R₄ für Wasserstoff oder Methyl; und
R₅ für eine -(CH=CH)₂, -(CH₂)₃ oder 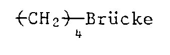 in 2,3- oder 3,4-Stellung; stehen.

19. Verbindungen der Formel Vgemäss Anspruch 18, worin R₂ für C₁-C₅-Alkyl; R₃ für C₁-C₅-Alkyl oder Cyclopentyl; R₄ für Wasserstoff; und R₅ für eine (CH=CH)₂, (CH₂)₃ oder 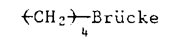 in 2,3- oder 3,4-Stellung stehen.

20. Verbindungen der Formel V gemäss Anspruch 19, worin R₂ und R₃ je für C₁-C₃-Alkyl; R₄ für Wasserstoff; und R₅ für eine -(CH=CH)₂ oder 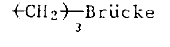 in 3,4-Stellung stehen.

21. Die Verbindungen gemäss Anspruch 20 der Formeln 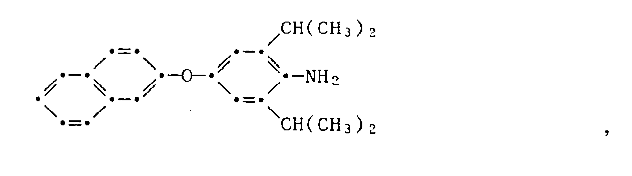 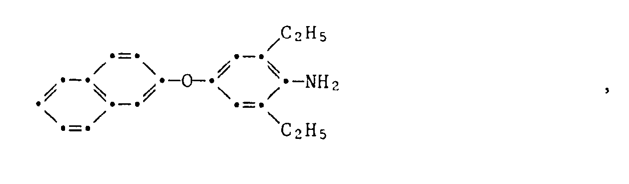 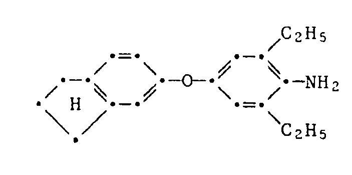 und

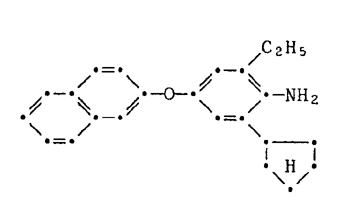

22. Verbindungen der Formel II 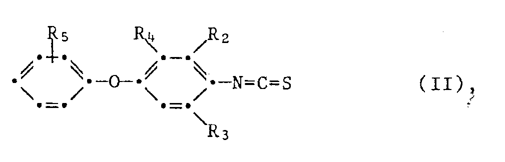 worin n
R₂ für Wasserstoff oder C₁-C₅-Alkyl;
R₃ für C₁-C₅-Alkyl oder C₅-C₆-Cycloalkyl;
R₄ für Wasserstoff oder Methyl; und
R₅ für eine (CH=CH)₂, (CH₂)₃ oder 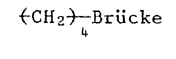 in 2,3- oder 3,4-Stellung stehen.

23. Verbindungen der Formel II gemäss Anspruch 22, worin R₂ für C₁-C₅-Alkyl; R₃ für C₁-C₅-Alkyl oder Cyclopentyl; R₄ für Wasserstoff; und R₅ für eine -(CH=CH)₂, -(CH₂)₃ oder 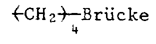 in 2,3- oder 3,4-Stellung stehen.

24. Verbindungen der Formel II gemäss Anspruch 23, worin R₂ und R₃ je für C₁-C₃-Alkyl; R₄ für Wasserstoff; und R₅ für eine (CH=CH)₂ oder 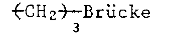 in 3,4-Stellung stehen.

25. Die Verbindungen gemäss Anspruch 24 der Formeln 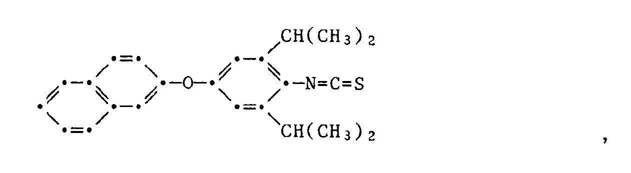 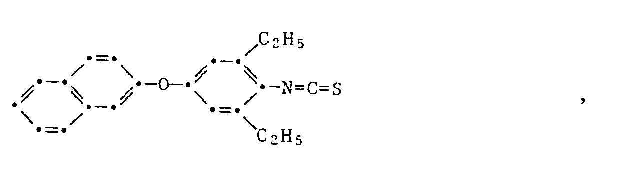 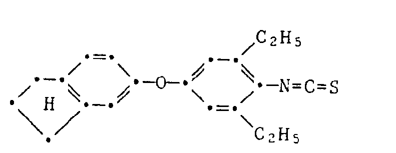 und

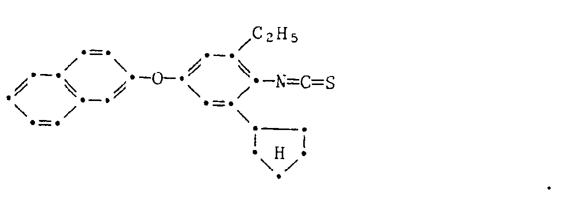

## Patentansprüche (Patentansprüche für folgende(n) Vertragsstaat(en) : ES)

1. Verfahren zur Herstellung einer Verbindung der Formel I 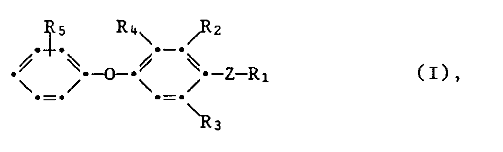 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine -(CH=CH)₂, -(CH₂)₂, 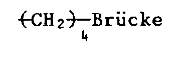 in 2,3- oder 3,4-Stellung;
Z für -NH-CS-NH-, -N=C(SR₆)-NH- oder -N=C=N- und R₆ für C₁-C₁₀-Alkyl oder Allyl stehen, dadurch gekennzeichnet, dass man
A) ein Isothiocyanat der Formel II 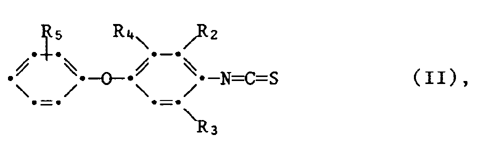 mit einem Amin der Formel III 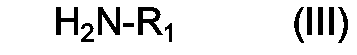 in einem organischen Lösungs- oder Verdünnungsmittel bei Normaldruck und einer Temperatur von 0 bis +150_{°}C zum Thioharnstoff und gegebenenfalls
B) den erhaltenen Thioharnstoff mit einer Verbindung der Formel IV 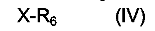 in einem inerten organischen Lösungsmittel unter leicht erhöhtem oder normalem Druck und bei einer Temperatur von +10 bis 250°C zum Isothioharnstoff umsetzt oder
C) den erhaltenen Thioharnstoff durch Abspalten von Schwefelwasserstoff in einem aprotischen organischen Lösungs- oder Verdünnungsmittel unter Normaldruck und bei einer Temperatur von 0 bis +150_{°}C in das Carbodiimid überführt, wobei R_{1'} R₂, R₃, R₄, R₅ und R₆ die angegebene Bedeutung haben und X für eine Abgangsgruppe steht.

2. Verfahren gemäss Anspruch 1, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder Cyclopentyl; R₄ für Wasserstoff; R₅ für eine (CH=CH)₂, -(CH₂)₃ oder 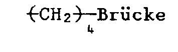 in 2,3-oder 3,4-Stellung; Z für-NH-CS-NH-, -N=C(SR₆)-NH-oder-N=C=N-; und R₆ für C₁-C₅-Alkyl oder Allyl stehen.

3. Verfahren gemäss Anspruch 2, worin R₁ für C₁-C₄-Alkyl; R₂ und R₃ je für C₁-C₃-Alkyl; R₄ für Wasserstoff; R₅ für eine -(CH=CH)₂ oder 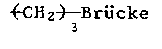 in 3,4-Stellung; und Z für -NH-CS-NH-stehen.

4. Verfahren gemäss Anspruch 2, worin R₁ für C₁-C₄-Alkyl; R₂ und R₃ je für C₁-C₃-Alkyl; R₄ für Wasserstoff; R₅ für eine -(CH=CH)₂ oder 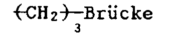 in 3,4-Stellung; Z für-N=C(SR₆)-NH-; und R₆ für C₁-C₃-Alkyl stehen.

5. Verfahren gemäss Anspruch 2, worin R₁ für C₁-C₄-Alkyl; R₂ und R₃ je für C₁-C₃-Alkyl; R₄ für Wasserstoff; R₅ für eine (CH=CH)₂ oder 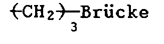 in 3,4-Stellung; und Z für -N=C=N- stehen.

6. Verfahren gemäss einem der Ansprüche 1 bis 3 zur Herstellung der Verbindungen der Formeln 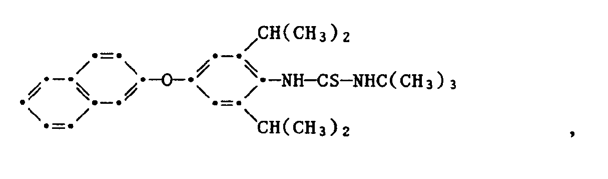 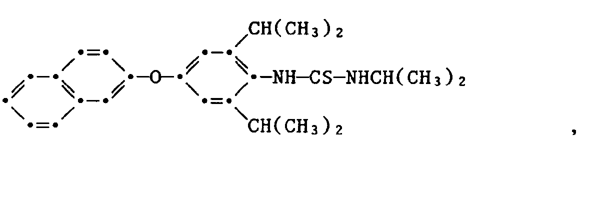 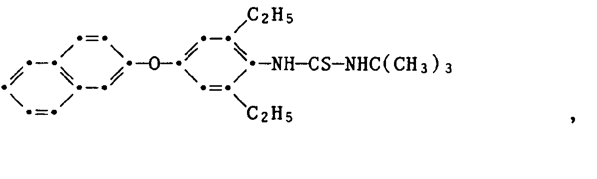 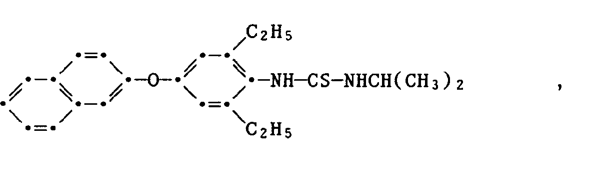 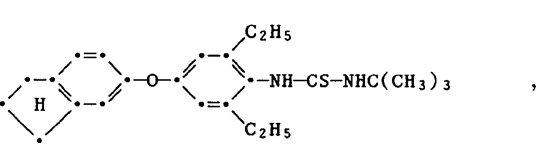 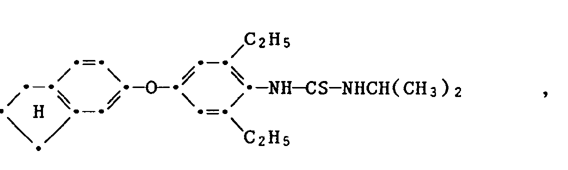 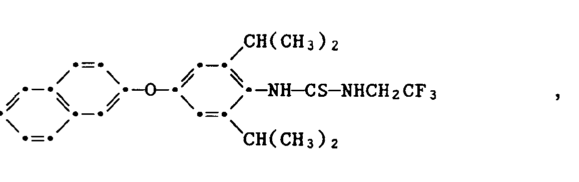 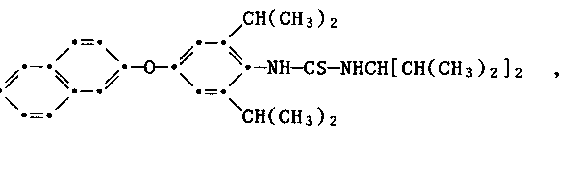 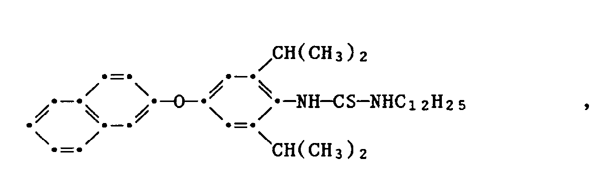 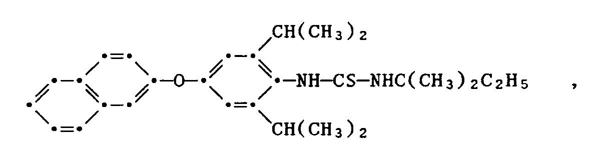 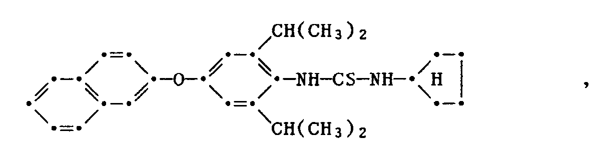 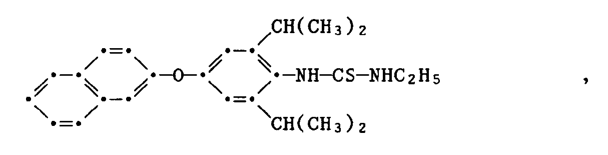 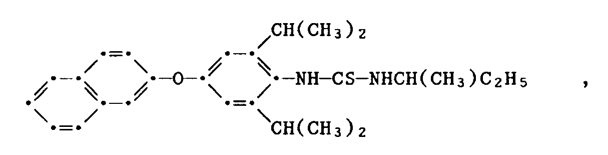 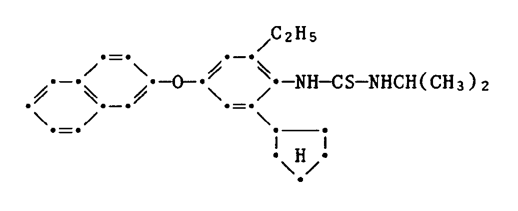 und

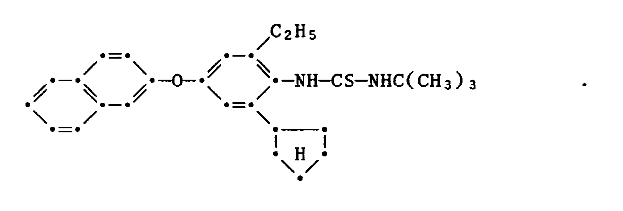

7. Verfahren gemäss einem der Ansprüche 1, 2 oder 4 zur Herstellung der Verbindungen der Formeln 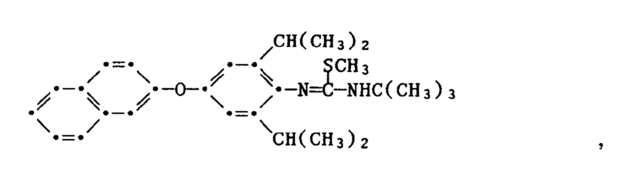 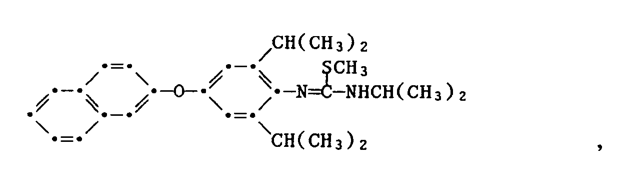 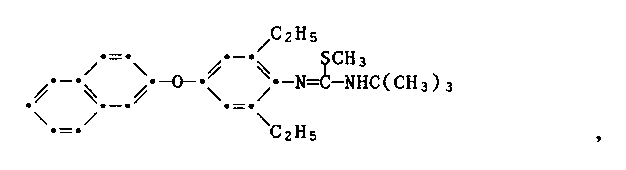 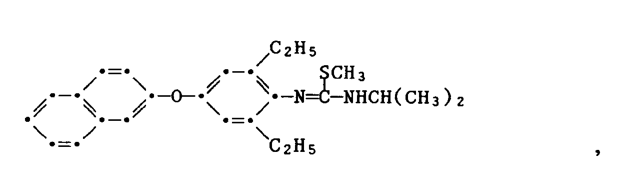 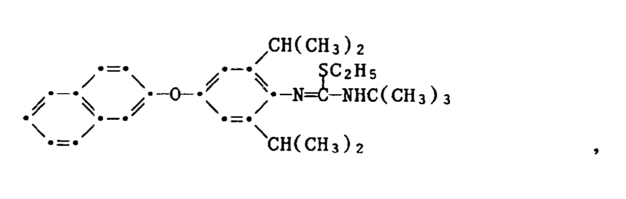 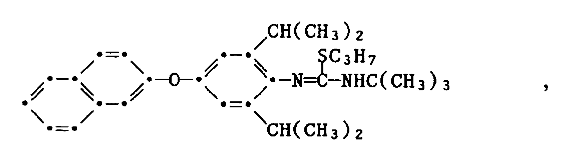 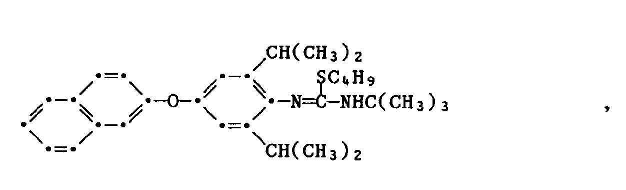 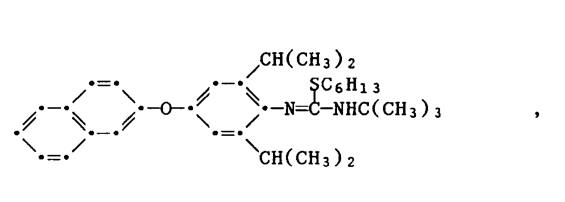 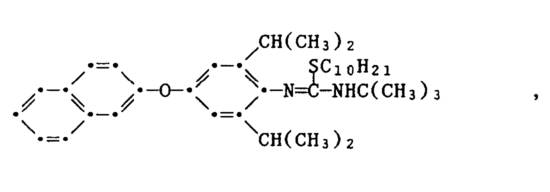 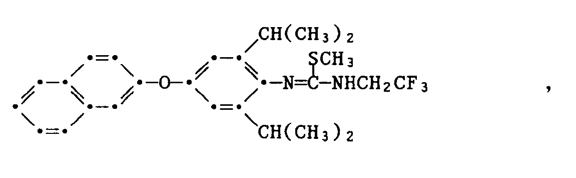 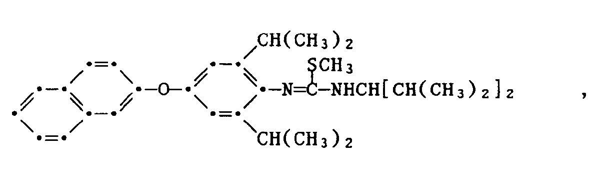 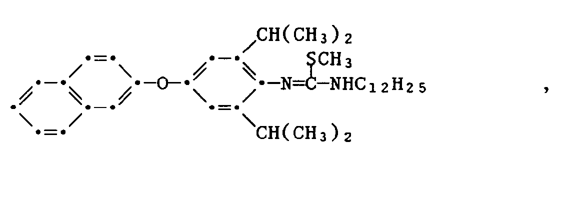 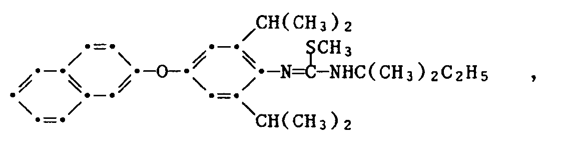 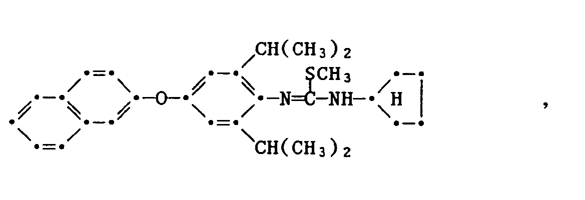 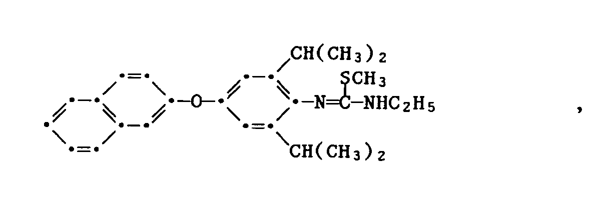 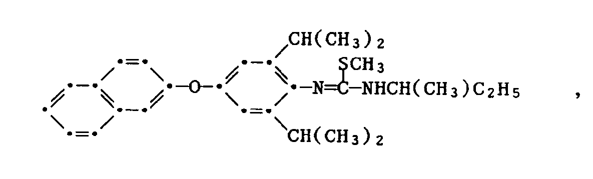 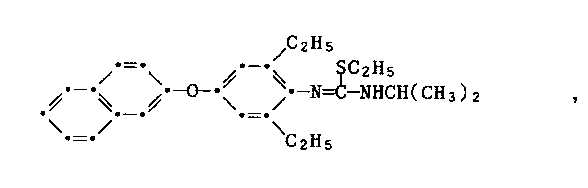 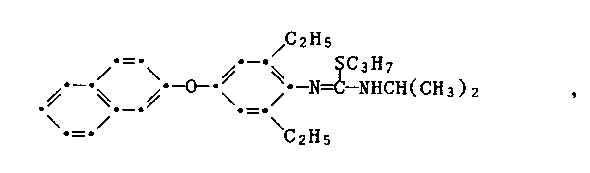 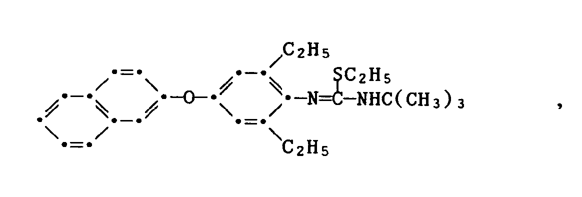 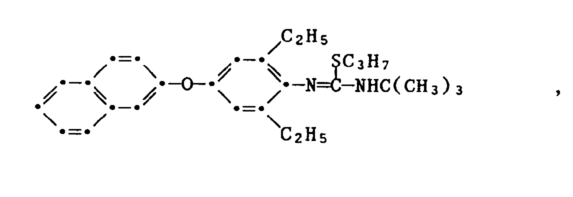 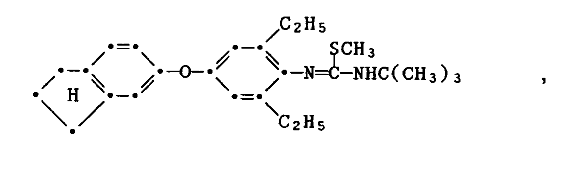 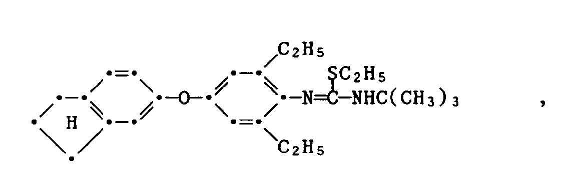 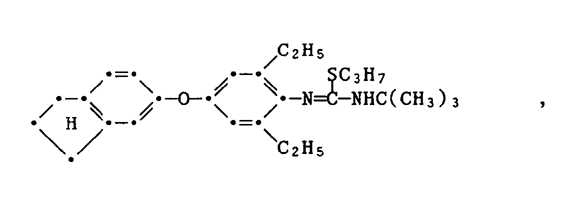 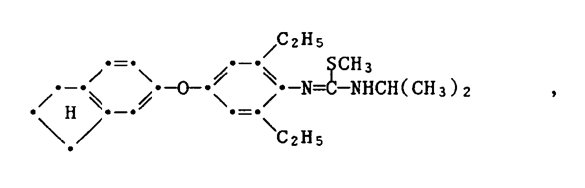 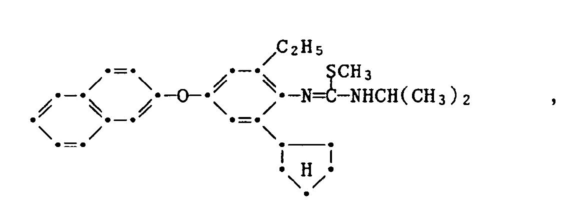 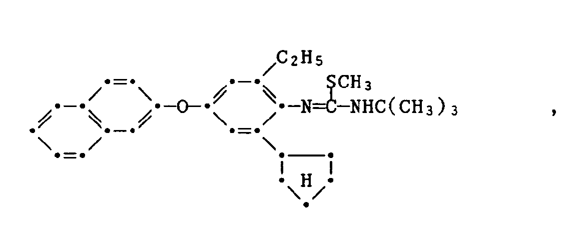 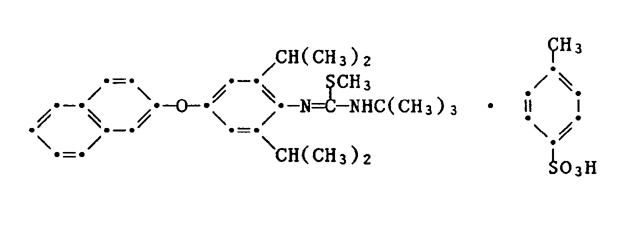 und

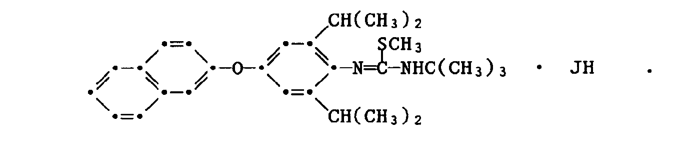

8. Verfahren gemäss einem der Ansprüche 1, 2 oder 5 zur Herstellung der Verbindungen der Formeln 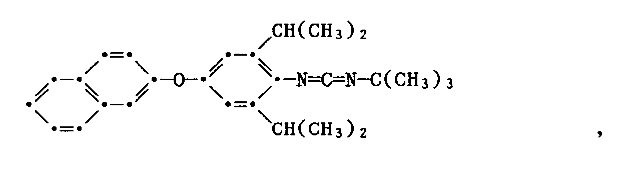 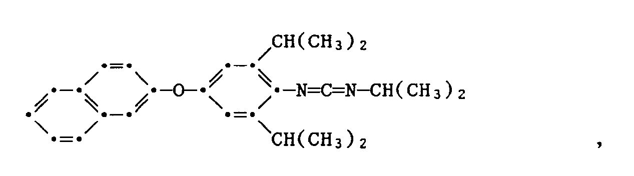 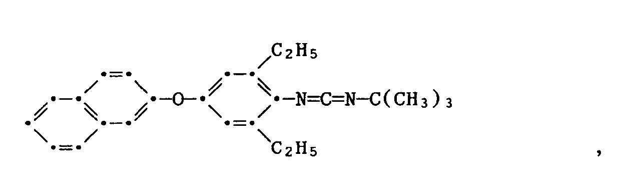 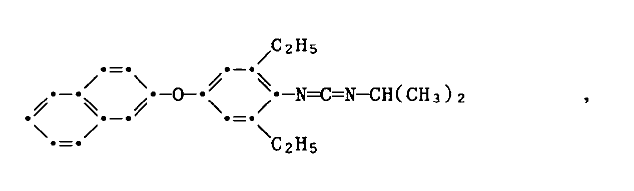 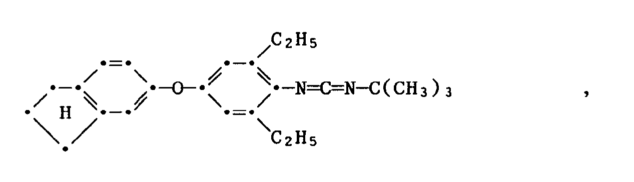 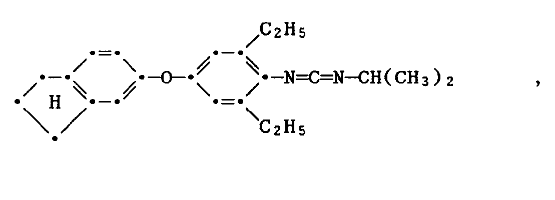 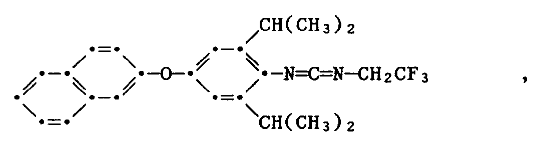 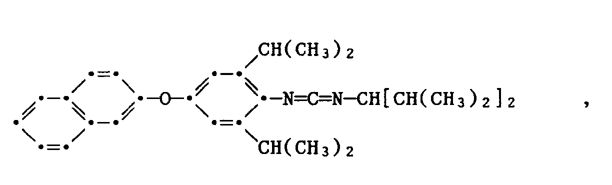 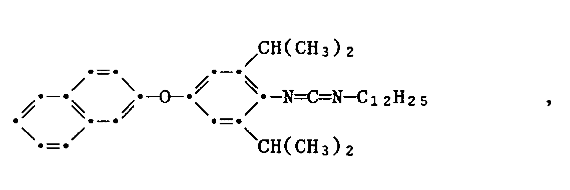 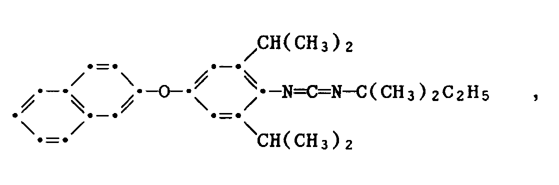 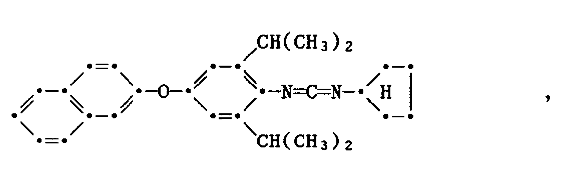 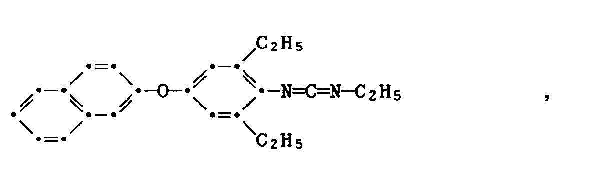 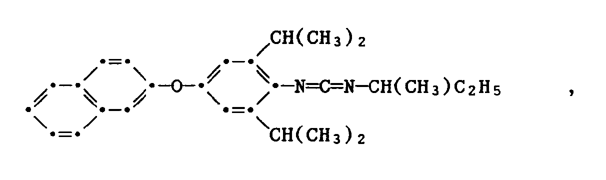 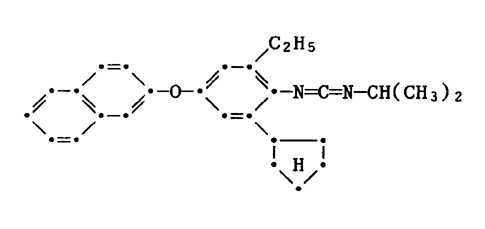 und

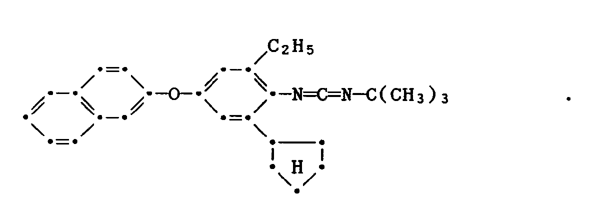

9. Schädlingsbekämpfungsmittel, welches als aktive Komponente mindestens eine Verbindung der Formel I 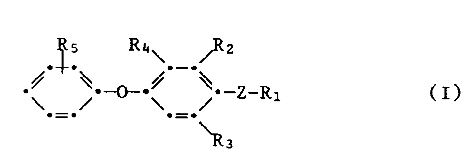 enthält, worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein-oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine -(CH=CH)₂, -(CH₂)₃. 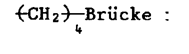 in 2,3- oder 3,4-Stellung;
Z für -NH-CS-NH-, -N=C(SR₆)-NH- oder -N=C=N- und
R₆ für C₁-C₁₀-Alkyl oder Allyl
stehen, oder eines ihrer Salze mit einer organischen oder anorganischen Säure zusammen mit geeigneten Trägern und/oder Zuschlagstoffen.

10. Schädlingsbekämpfungsmittel gemäss Anspruch 9, welches als aktive Komponente mindestens eine Verbindung der Formel enthält, worin R₁ für C₁-C₈-Alkyl, ein- oder mehrfach durch Halogen und/oder C₁-C₅-Alkoxy substituiertes C₁-C₈-Alkyl oder C₃-C₈-Cycloalkyl; R₂ für C₁-C₅-Alkyl; R₃ für C₁-C₅-Alkyl oder Cyclopentyl; R₄ für Wasserstoff; R₅ für eine (CH=CH)₂, (CH₂)₃ oder 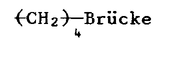 in 2,3- oder 3,4-Stellung; Z für -NH-CS-NH-, -N=C(SR₆)-NH- oder -N=C=N-; und R₆ für C₁-C₅-Alkyl oder Allyl stehen.

11. Schädlingsbekämpfungsmittel gemäss Anspruch 10, worin R₁ für C₁-C₄-Alkyl; R₂ und R₃ je für C₁-C₃-Alkyl; R₄ für Wasserstoff; R₅ für eine (CH=CH)₂ oder 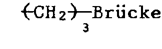 in 3,4-Stellung; und Z für -NH-CS-NH- stehen.

12. Schädlingsbekämpfungsmittel gemäss Anspruch 10, worin R₁ für C₁-C₄-Alkyl; R₂ und R₃ je für C₁-C₃-Alkyl; R₄ für Wasserstoff; R₅ für eine (CH=CH)₂ oder in 3,4-Stellung; Z für-N=C(SR₆)-NH-; und R₆ für C₁-C₃-Alkyl s

13. Schädlingsbekämpfungsmittel gemäss Anspruch 10, Alkyl; R₄ für Wasserstoff; R₅ für eine (CH=CH)₂ oder tehen. vorin R₁ für C₁-C₄-Alkyl; R₂ und R₃ je für C₁-C₃- 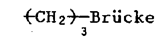 in 3,4-Stellung; und Z für -N=C=N- stehen.

14. Verwendung einer Verbindung der Formel I 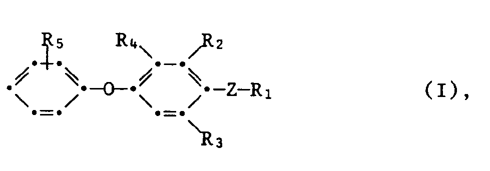 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cylcloalkyl-C₁-C₄-alkyl;
R₂ für Wasserstoff oder C₁-C₅-Alkyl;
R₃ für C₁-C₅-Alkyl oder C₅-Cₛ-Cycloalkyl;
R₄ für Wasserstoff oder Methyl;
R₅ für eine (CH=CH)₂, (CH₂)₃, 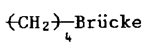 in 2,3- oder 3,4-Stellung;
Z für -NH-CS-NH-, -N=C(SR₆)-NH- oder -N=C=N- und
R₆ für C₁-C₁₀-Alkyl oder Allyl,
stehen, oder eines ihrer Salze mit einer organischen oder anorganischen Säure zur Bekämpfung von Schädlingen an Tieren und Pflanzen.

15. Verwendung gemäss Anspruch 14 zur Bekämpfung von Insekten und Arachniden.

16. Verfahren zum Bekämpfen von Schädlingen an Tieren und Pflanzen, dadurch gekennzeichnet, dass man die Schädlinge in ihren verschiedenen Entwicklungsstadien mit einer Verbindung der Formel I 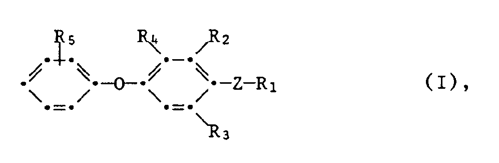 worin
R₁ für C₁-C₁₂-Alkyl, ein- oder mehrfach durch Halogen und/oder C₁-C₆-Alkoxy substituiertes C₁-C₁₂-Alkyl, C₃-C₈-Cycloalkyl, ein- oder mehrfach durch C₁-C₃-Alkyl substituiertes C₃-C₈-Cycloalkyl oder C₃-C₈-Cycloalkyl-C₁-C₄-alkyl;
R₂ für Wasserstoff oder C₁-C₅-Alkyl;
R₃ für C₁-C₅-Alkyl oder C₅-C₆-Cycloalkyl;
R₄ für Wasserstoff oder Methyl;
R₅ für eine -(CH=CH)₂, (CH₂)₃, 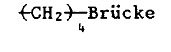 in 2,3- oder 3,4-Stellung;
Z für -NH-CS-NH-, -N=C(SR₆)-NH- oder -N=C=N- und
R₆ für C₁-C₁₀-Alkyl oder Allyl stehen, oder einem ihrer Salze mit einer organischen oder anorganischen Säure in Kontakt bringt.

17. Verfahren zur Herstellung einer Verbindung der Formel V 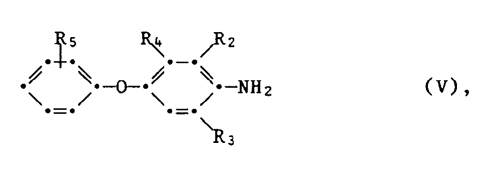 worin n
R₂ für Wasserstoff oder C₁-C₅-Alkyl;
R₃ für C₁-C₅-Alkyl oder C₅-C₆-Cycloalkyl;
R₄ für Wasserstoff oder Methyl; und
R₅ für eine -(CH=CH)₂, (CH₂)₃ oder 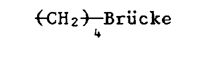 in 2,3- oder 3,4-Stellung
stehen, dadurch gekennzeichnet, dass man ein Anilin der Formel VI 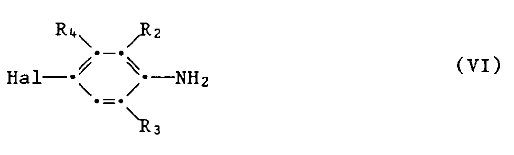 mit einem Phenol der Formel VII 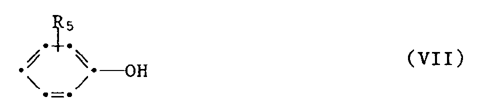 umsetzt, wobei R₂, R₃, R₄ und R₅ die für Formel V angegebene Bedeutung haben und Hal für Halogen, insbesondere für Chlor oder Brom steht.

18. Verfahren gemäss Anspruch 17, worin R₂ für C₁-C₅-Alkyl; R₃ für C₁-C₅-Alkyl oder Cyclopentyl; R₄ für Wasserstoff; und R₅ für eine (CH=CH)₂, (CH₂)₃ oder 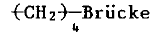 in 2,3- oder 3,4-Stellung stehen.

19. Verfahren gemäss Anspruch 18, worin R₂ und R₃je für C₁-C₃-Alkyl; R₄ für Wasserstoff; und R₅ für eine (CH=CH)₂ oder 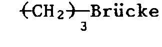 in 3,4-Stellung stehen.

20. Verfahren gemäss Anspruch 19 zur Herstellung der Verbindungen der Formeln 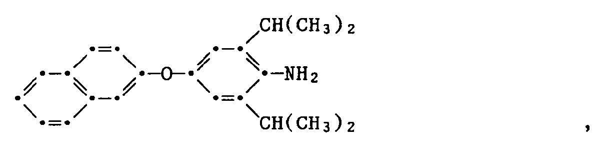 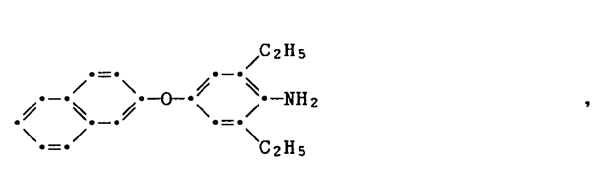 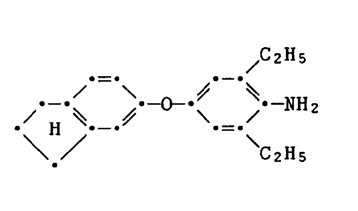 und

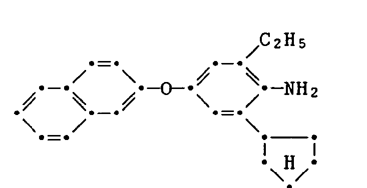

21. Verfahren zur Herstellung der Verbindungen der Formel II 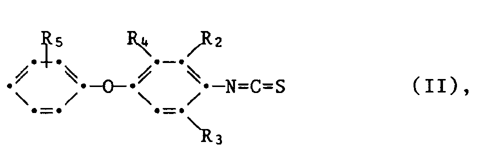 worin n
R₂ für Wasserstoff oder C₁-C₅-Alkyl;
R₃ für C₁-C₅-Alkyl oder C₅-C₆-Cycloalkyl;
R₄ für Wasserstoff oder Methyl; und
R₅ für eine (CH=CH)₂, (CH₂)₃ oder 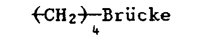 in 2,3- oder 3,4-Stellung stehen, dadurch gekennzeichnet, dass man ein Phenoxyanilin der Formel V 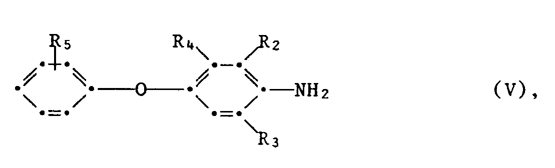 worin R₂, R₃, R₄ und R₅ die für Formel II angegebene Bedeutung haben, mit Thiophosgen umsetzt

22. Verfahren gemäss Anspruch 21, worin R₂ für C₁-C₅-Alkyl; R₃ für C₁-C₅-Alkyl oder Cyclopentyl; R₄ für Wasserstoff; und R₅ für eine (CH=CH)₂, (CH₂)₃ oder 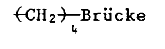 in 2,3- oder 3,4-Stellung stehen.

23. Verfahren gemäss Anspruch 22, worin R₂ und R₃je für C₁-C₃-Alkyl; R₄ für Wasserstoff; und R₅ für eine (CH=CH)₂ oder 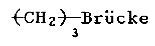 in 3,4-Stellung stehen.

24. Verfahren gemäss Anspruch 23 zur Herstellung von Verbindungen der Formeln 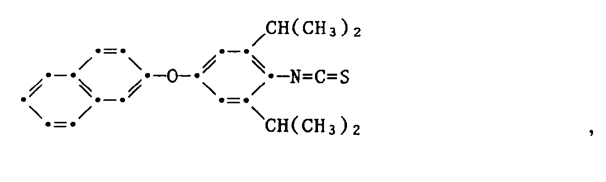 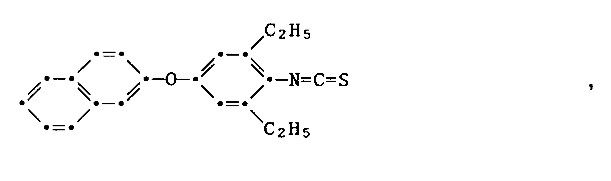 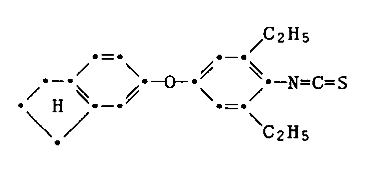 und

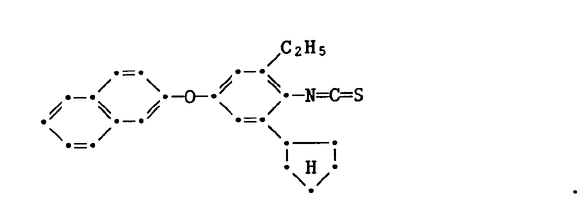

## Claims (Claims for the following Contracting State(s) : AT, BE, CH, DE, FR, GB, GR, IT, LI, NL)

1. Compounds of formula I 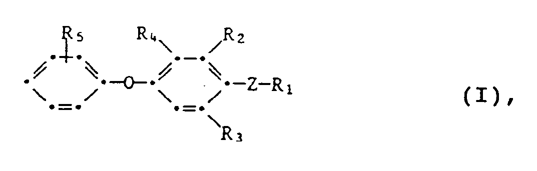 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-C₆cycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, and the salts thereof with organic or inorganic acids.

2. Compounds of formula I according to claim 1, in which R₁ represents C₁-C₈alkyl, C₁-C₈alkyl mono- or poly-substituted by halogen and/or by C₁-C₅alkoxy, or C₃-C₈-cycloalkyl; R₂ represents C₁-C₅alkyl; R₃ represents C₁-C₅alkyl or cyclopentyl; R₄ represents hydrogen; R₅ represents a (CH=CH)₂, (CH₂)₃ or (CH₂)₄ bridge in the 2,3- or 3,4-position; Z represents -NH-CS-NH-, -N=C(SR₆)-NH- or -N=C=N-; and R₆ represents C₁-C₅alkyl or allyl.

3. Compounds of formula I according to claim 2, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents -NH-CS-NH-.

4. Compounds of formula I according to claim 2, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; Z represents -N=C(SR₆)-NH-; and R₆ represents C₁-C₃alkyl.

5. Compounds of formula I according to claim 2, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents -N=C=N-.

6. The compounds according to any one of claims 1 to 3 of the formulae 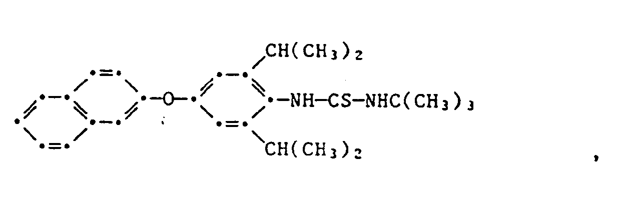 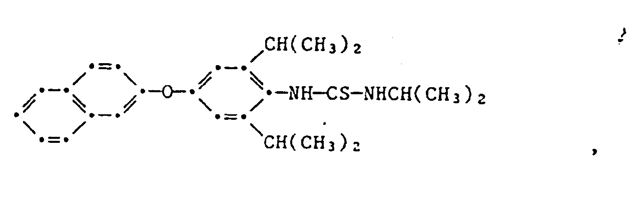 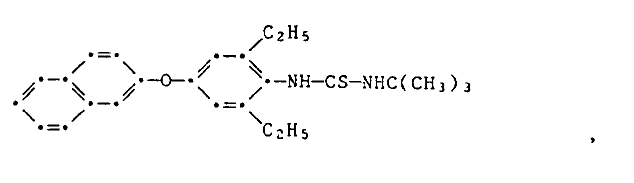 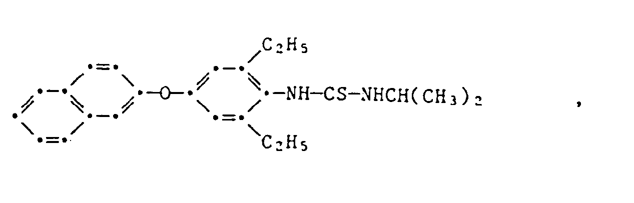 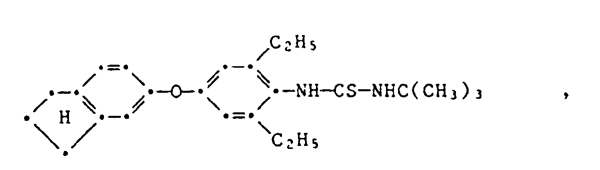 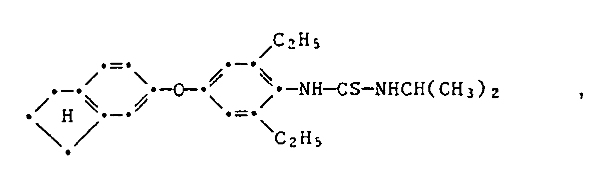 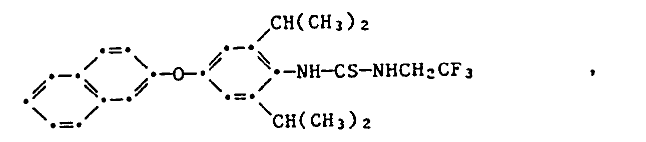 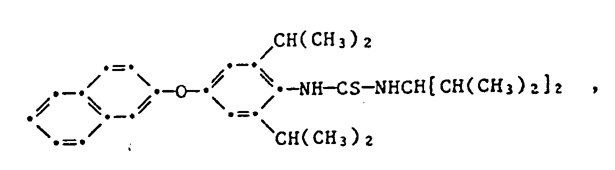 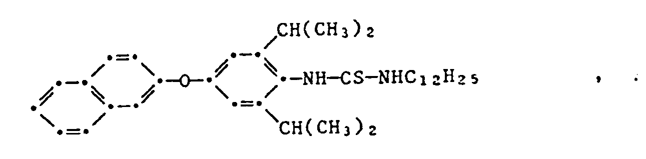 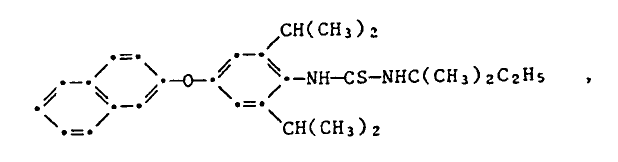 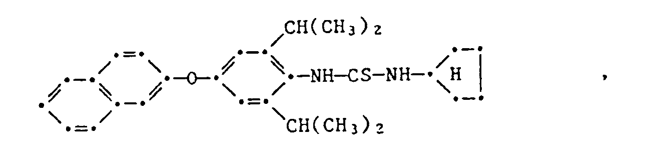 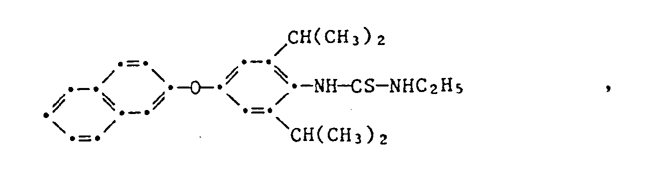 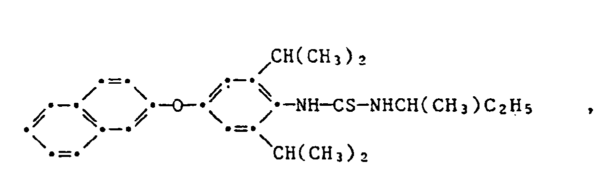 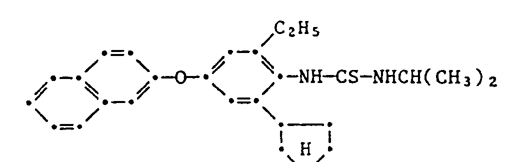 and

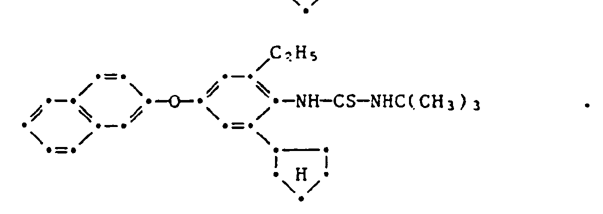

7. The compounds according to any one of claims 1, 2 and 4 of the formulae 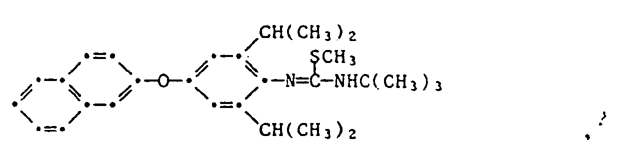 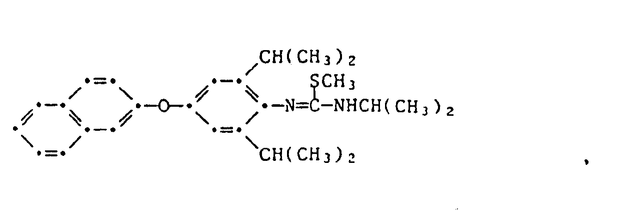 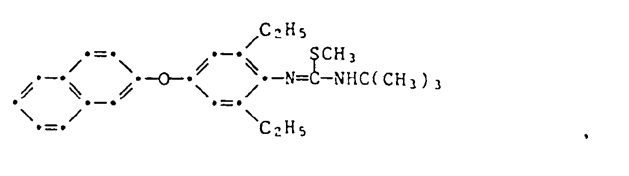 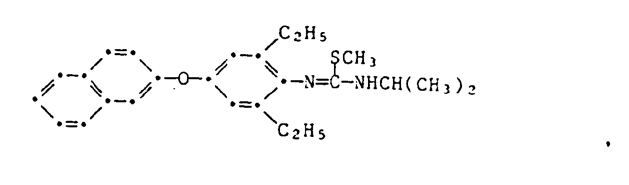 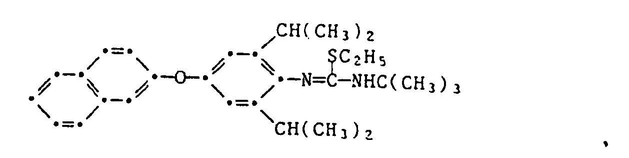 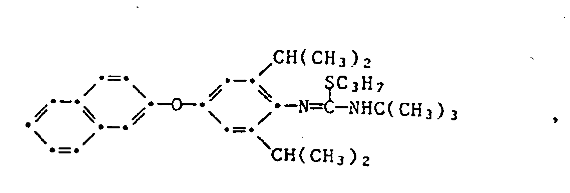 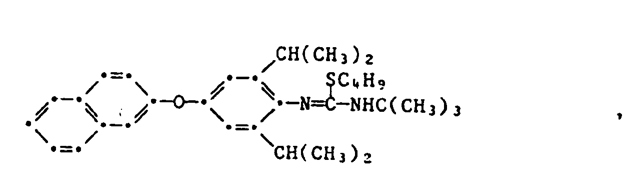 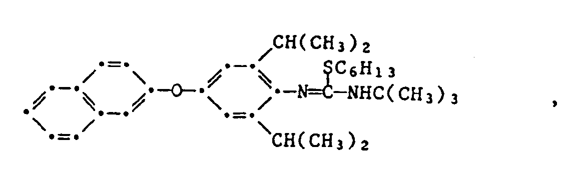 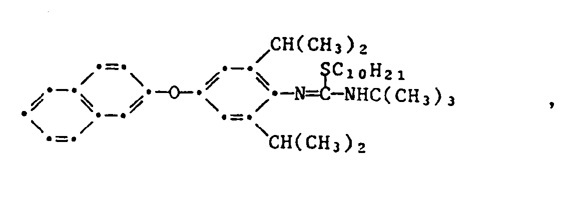 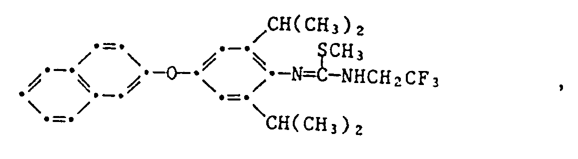 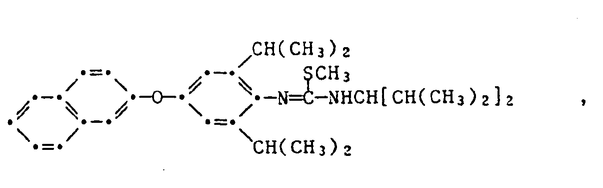 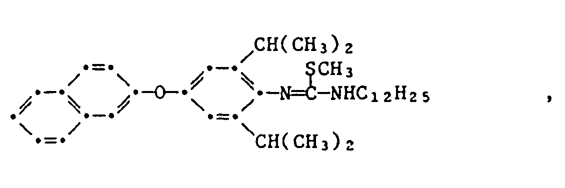 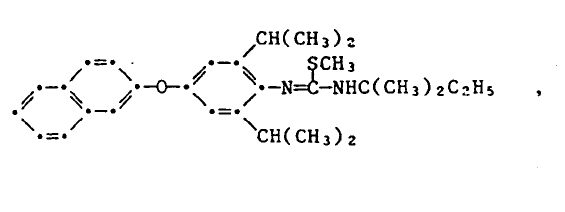 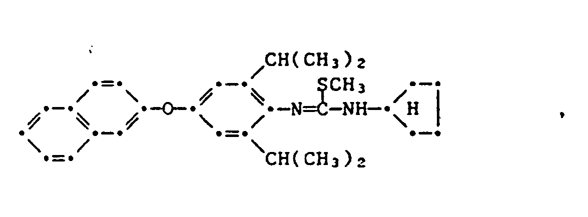 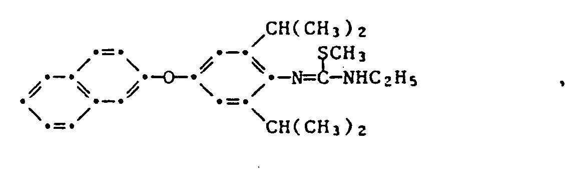 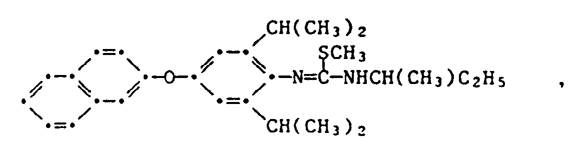 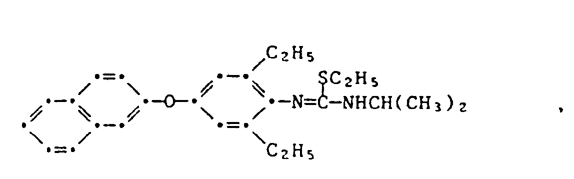 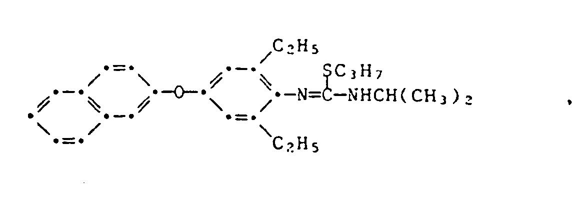 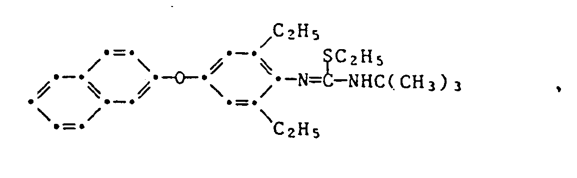 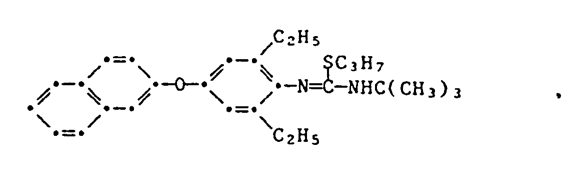 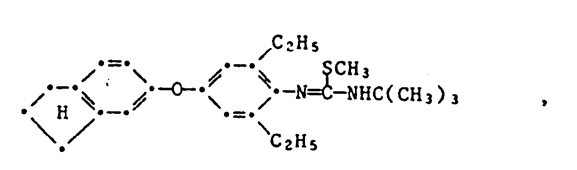 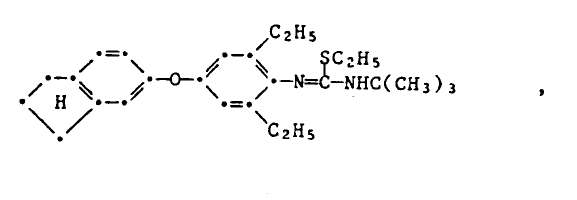 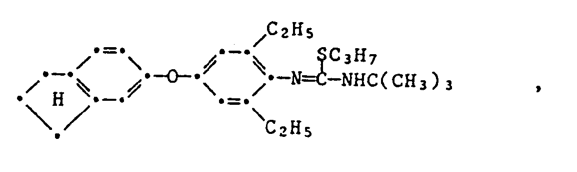 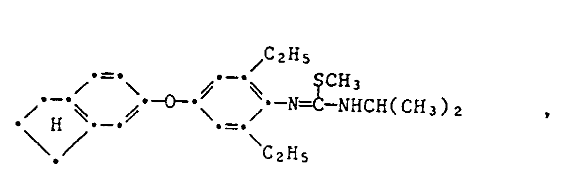 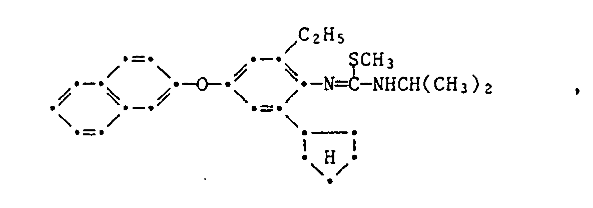 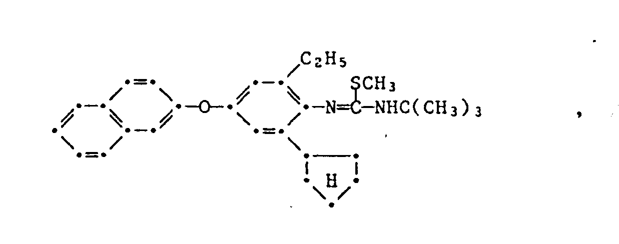 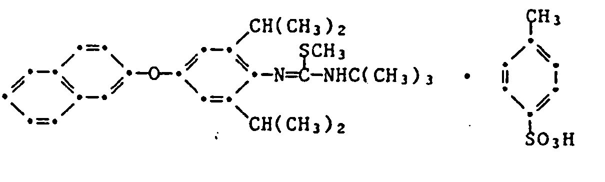 and

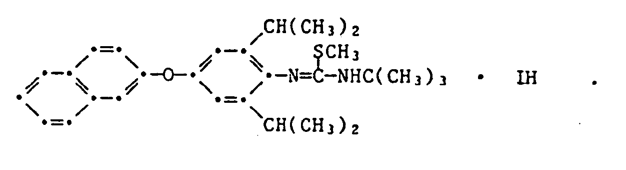

8. The compounds according to any one of claims 1, 2 and 5 of the formulae 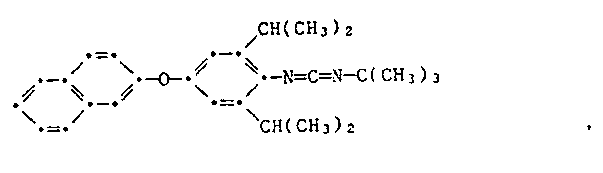 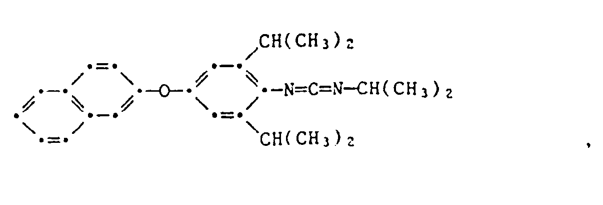 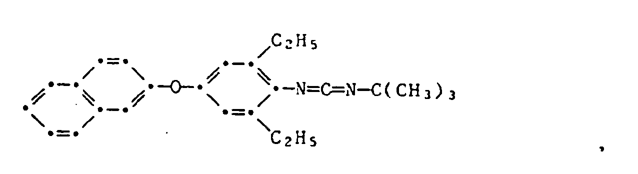 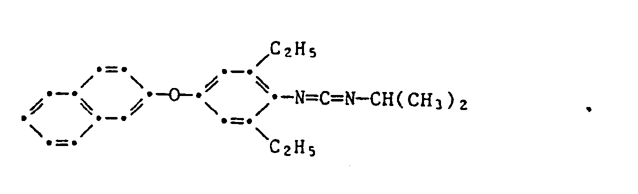 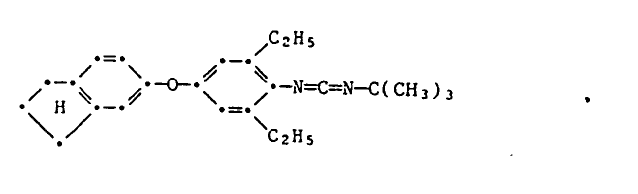 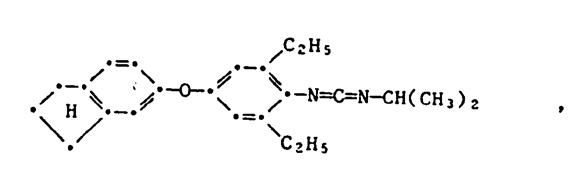 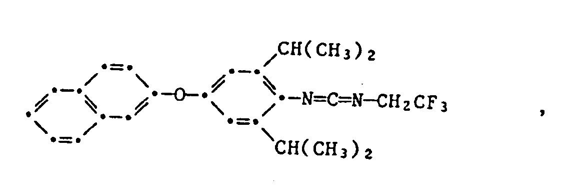 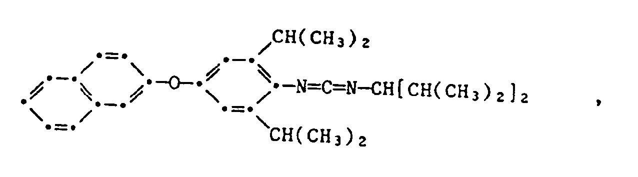 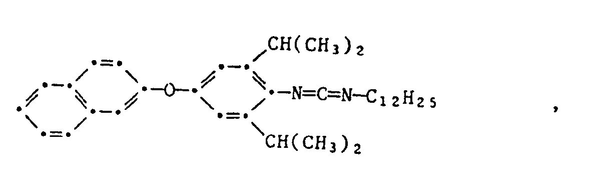 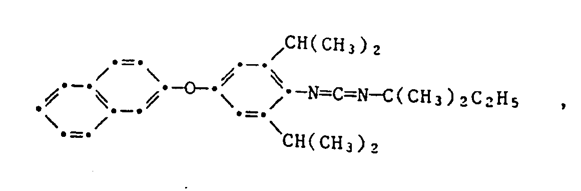 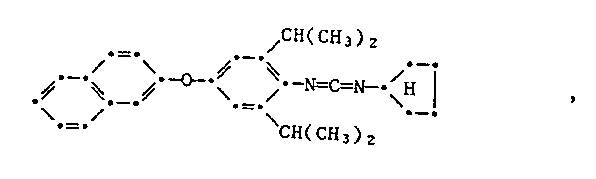 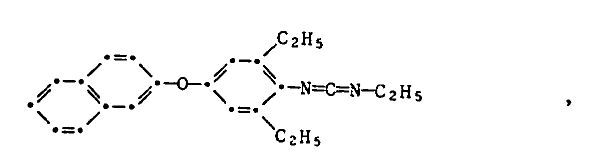 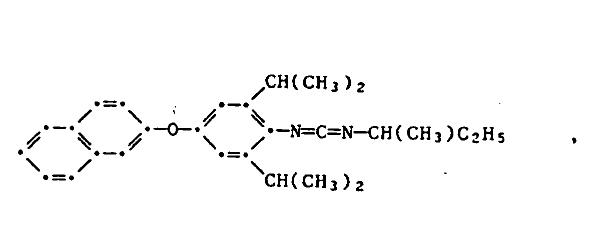 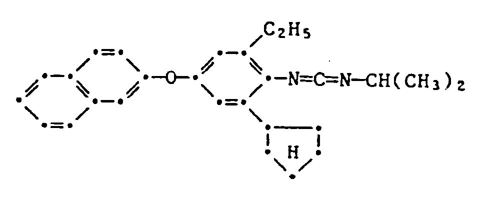 and

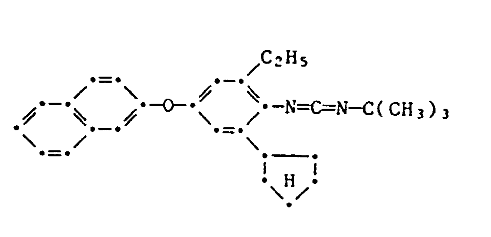

9. A process for the preparation of a compound of formula I 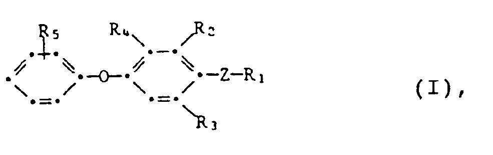 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅-alkyl;
R₃ represents C₁-C₅-alkyl or C₅-Cₛcycloalkyl;
R₄ represents hydrogen or methyl;
R₅ represents a (CH=CH)₂, (CH₂)3 or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, characterised in that
A) an isothiocyanate of formula II 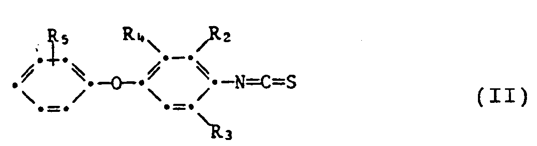 is reacted with an amine of formula III 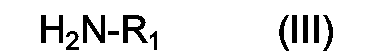 in an organic solvent or diluent at normal pressure and at a temperature of from 0 to +150_{°}C to form the thiourea and optionally
B) the resulting thiourea is reacted with a compound of formula IV 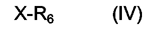 in an inert organic solvent under slightly elevated or normal pressure and at a temperature of from +10 to 250°C to form the isothiourea, or
C) the resulting thiourea is converted into the carbodiimide by removal of hydrogen sulfide in an aprotic organic solvent or diluent under normal pressure and at a temperature of from 0 to +150°C, R₁, R₂, R₃, R₄, R₅ and R₆ having the meanings given and X representing a leaving group.

10. A pesticidal composition comprising as active ingredient at least one compound of formula I 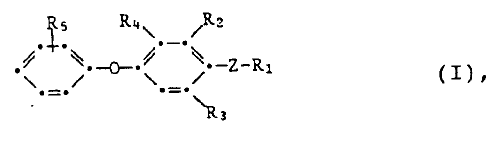 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-Cₛcycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, or one of the salts thereof with an organic or inorganic acid, together with suitable carriers and/or adjuvants.

11. A pesticidal composition according to claim 10 comprising as active ingredient at least one compound of formula in which R₁ represents C₁-C₈alkyl, C₁-C₈alkyl mono- or poly-substituted by halogen and/or by C₁-C₅-alkoxy, or C₃-C₈cycloalkyl; R₂ represents C₁-C₅alkyl; R₃ represents C₁-C₅alkyl or cyclopentyl; R₄ represents hydrogen; R₅ represents a (CH=CH)₂, (CH₂)₃ or (CH₂)₄ bridge in the 2,3- or 3,4-position; Z represents -NH-CS-NH-, -N=C(SR₆)-NH- or -N=C=N- and R₆ represents C₁-C₅alkyl or allyl.

12. A pesticidal composition according to claim 11, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents -NH-CS-NH-.

13. A pesticidal composition according to claim 11, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; Z represents -N=C(SR₆)-NH-; and R₆ represents C₁-C₃alkyl.

14. A pesticidal composition according to claim 11, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents -N=C=N-.

15. The use of a compound of formula I 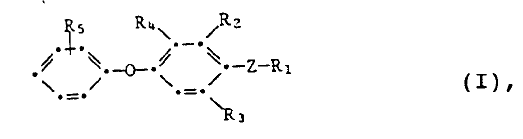 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-Cₛcycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, or one of the salts thereof with an organic or inorganic acid, for controlling pests in and on animals and plants.

16. The use according to claim 15 for controlling insects and arachnids.

17. A method of controlling pests in and on animals and plants, characterised in that the pests in their various stages of development are brought into contact with a compound of formula I 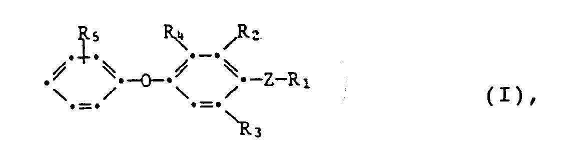 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-Cₛcycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, or with one of the salts thereof with an organic or inorganic acid.

18. A compound of formula V 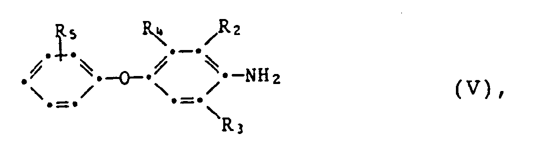 in which R₂ represents hydrogen or C₁-C₅alkyl;
R₃ represents C₁-C₅alkyl or C₅-Cₛcycloalkyl;
R₄ represents hydrogen or methyl; and R₅ represents a (CH=CH)₂, (CH₂)₃ or (CH₂)₄ bridge in the 2,3- or 3,4-position.

19. Compounds of formula V according to claim 18, in which R₂ represents C₁-C₅alkyl; R₃ represents C₁-C₅alkyl or cyclopentyl; R₄ represents hydrogen; and R₅ represents a (CH=CH)₂, (CH₂)₃ or (CH₂)₄ bridge in the 2,3- or 3,4-position.

20. Compounds of formula V according to claim 19, in which R₂ and R₃ each represents C₁-C₃alkyl; R₄ represents hydrogen; and R₅ represents a (CH=CH)₂ or (CH₂)₃ bridge in the 3,4-position.

21. The compounds according to claim 20 of the formulae 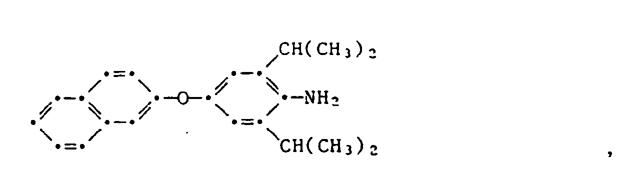 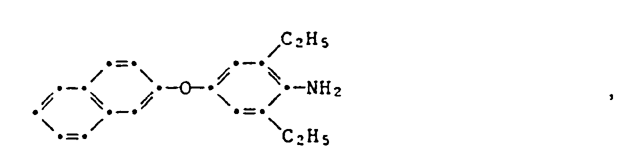 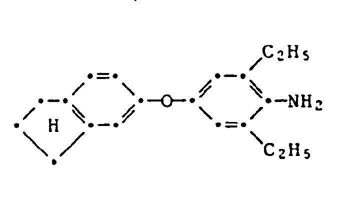 and

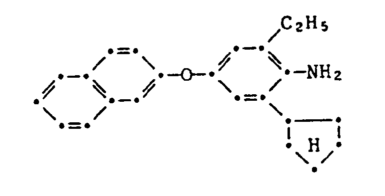

22. Compounds of formula II 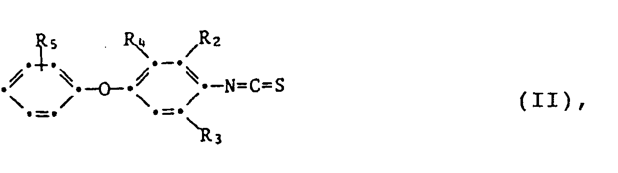 in which R₂ represents hydrogen or C₁-C₅alkyl;
R₃ represents C₁-C₅alkyl or C₅-C₆cycloalkyl;
R₄ represents hydrogen or methyl; and R₅ represents a (CH=CH)₂, (CH₂)₃ or (CH₂)₄ bridge in the 2,3- or 3,4-position.

23. Compounds of formula II according to claim 22, in which R₂ represents C₁-C₅alkyl; R₃ represents C₁-C₅alkyl or cyclopentyl; R₄ represents hydrogen; and R₅ represents a (CH=CH)₂, (CH₂)₃ or (CH₂)₄ bridge in the 2,3- or 3,4-position.

24. Compounds of formula II according to claim 23, in which R₂ and R₃ each represents C₁-C₃alkyl; R₄ represents hydrogen; and R₅ represents a (CH=C)₂ or (CH₂)₃ bridge in the 3,4-position.

25. The compounds according to claim 24 of the formulae 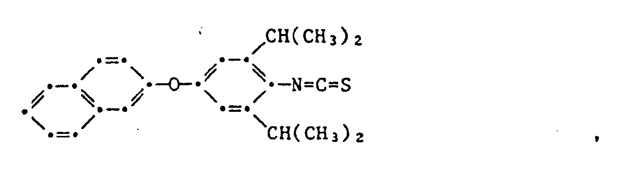 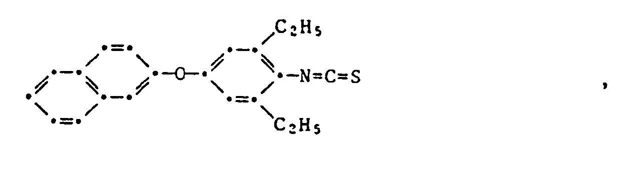 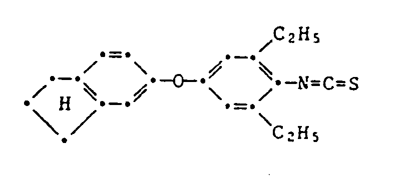 and

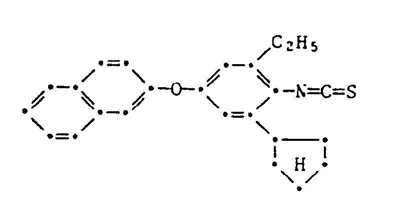

## Claims (Claims for the following Contracting State(s) : ES)

1. A process for the preparation of a compound of formula I 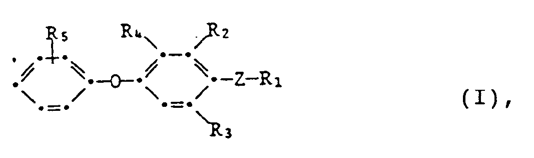 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-Cₛcycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, characterised in that
A) an isothiocyanate of formula II 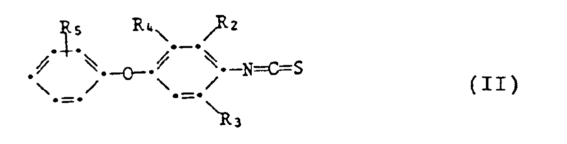 is reacted with an amine of formula III 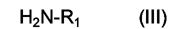 in an organic solvent or diluent at normal pressure and at a temperature of from 0 to +150_{°}C to form the thiourea and optionally
B) the resulting thiourea is reacted with a compound of formula IV 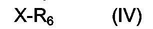 in an inert organic solvent under slightly elevated or normal pressure and at a temperature of from +10 to 250°C to form the isothiourea, or
C) the resulting thiourea is converted into the carbodiimide by removal of hydrogen sulfide in an aprotic organic solvent or diluent under normal pressure and at a temperature of from 0 to +150°C, R₁, R₂, R₃, R₄, R₅ and R₆ having the meanings given and X representing a leaving group.

2. A process according to claim 1, in which R₁ represents C₁-C₈alkyl, C₁-C₈alkyl mono- or poly-substituted by halogen and/or by C₁-C₅alkoxy, or C₃-C₈cycloalkyl; R₂ represents C₁-C₅alkyl; R₃ represents C₁-C₅alkyl or cyclopentyl; R₄ represents hydrogen; R₅ represents a (CH=CH)₂, (CH₂)₃ or (CH₂)₄ bridge in the 2,3- or 3,4- position; Z represents -NH-CS-NH-, -N=C(SR₆)-NH- or -N=C=N-; and R₆ represents C₁-C₅alkyl or allyl.

3. A process according to claim 2, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents - NH-CS-NH-.

4. A process according to claim 2, in which R₁ represents C₁-C₄alkyl; R₂ and R₃ each represents C₁-C₃alkyl; R₄ represents hydrogen, R₅ represents a (CH=CH)₂ or (CH₂)₃ bridge in the 3,4-position; Z represents - N=C(SR₆)-NH-; and R₆ represents C₁-C₃alkyl.

5. A process according to claim 2, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents - N=C=N-.

6. A process according ,to any one of claims 1 to 3 for the preparation of the compounds of the formulae 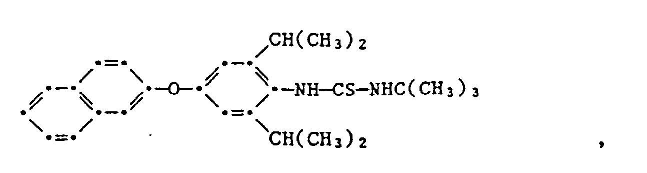 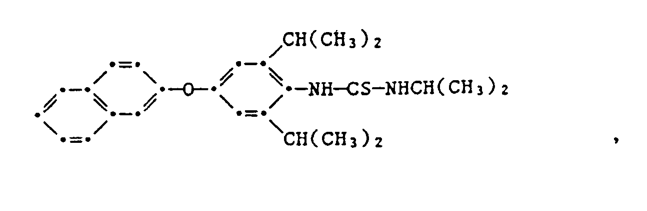 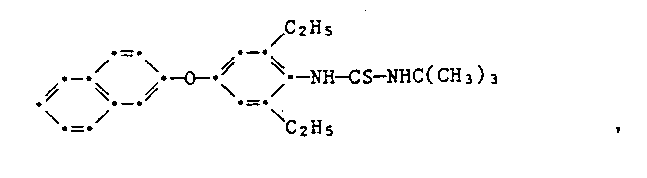 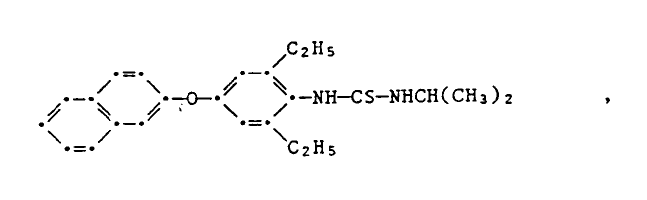 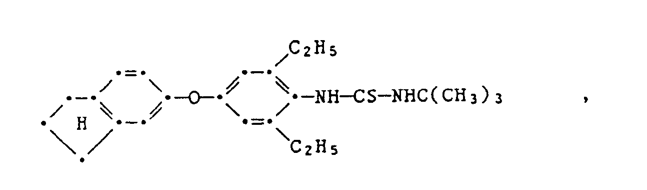 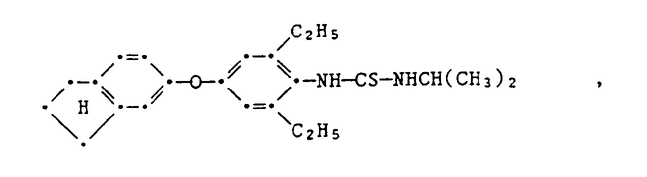 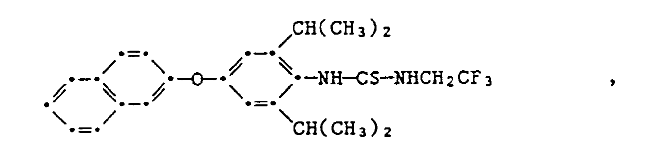 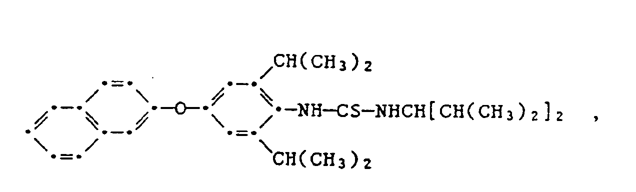 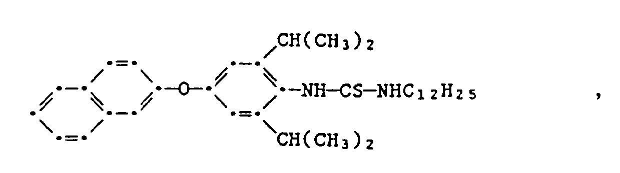 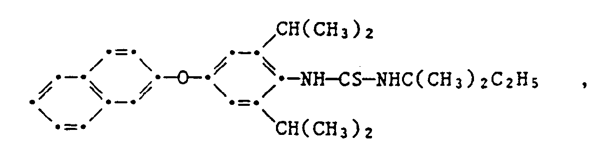 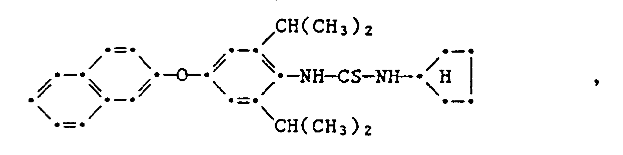 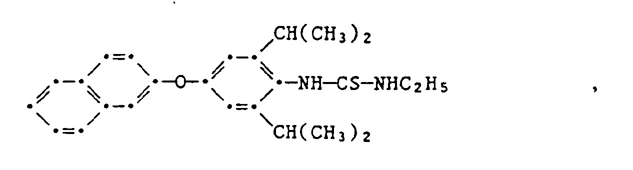 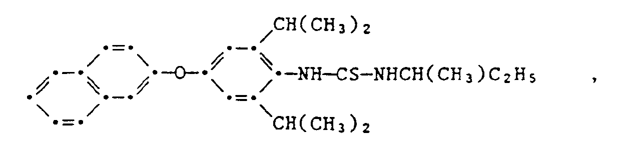 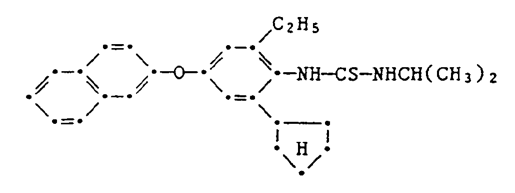 and

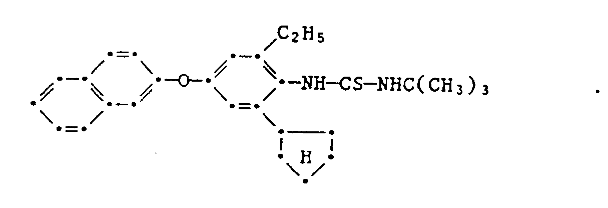

7. A process according to any one of claims 1, 2 and 4 for the preparation of the compounds of the formulae 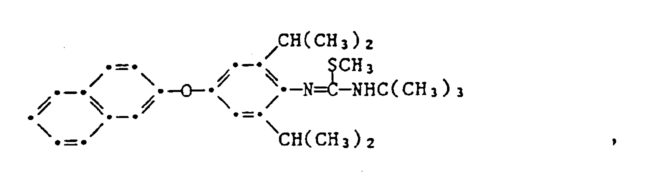 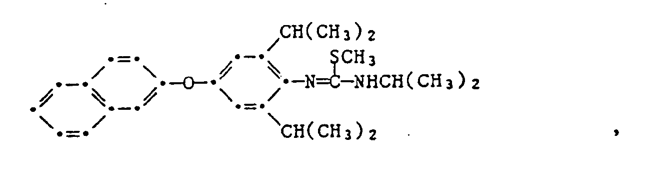 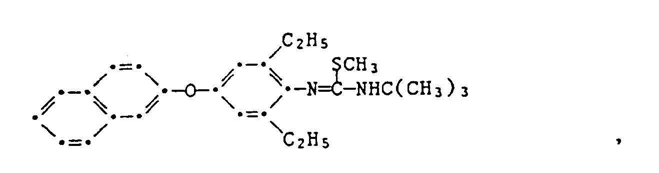 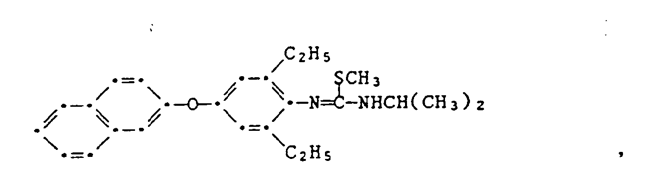 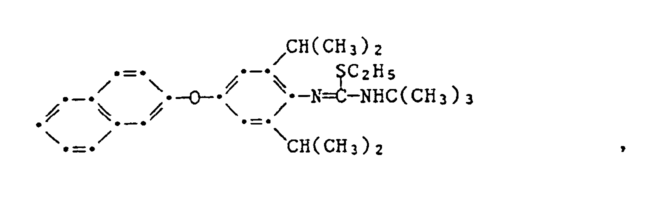 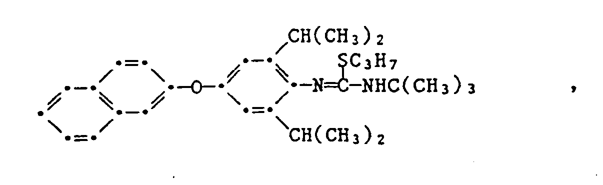 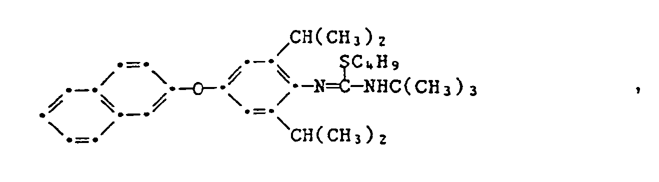 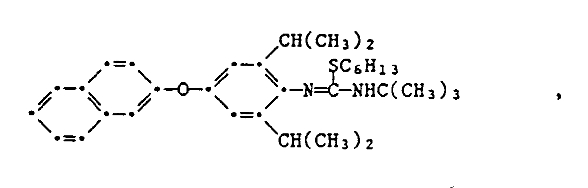 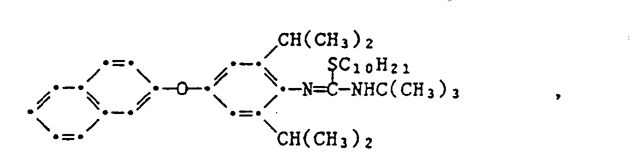 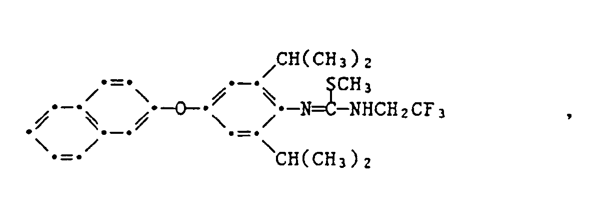 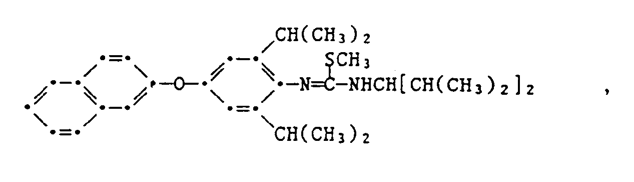 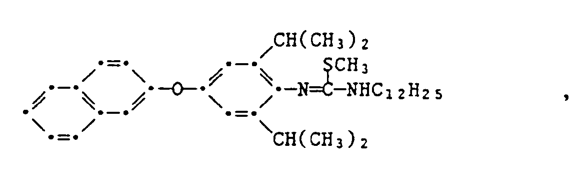 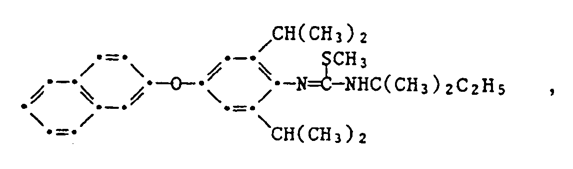 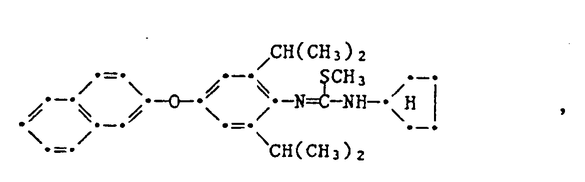 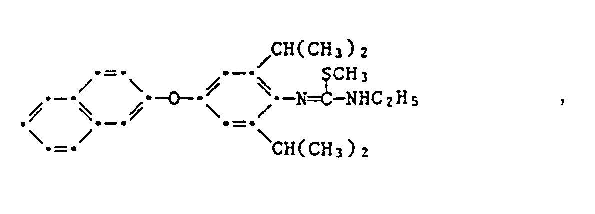 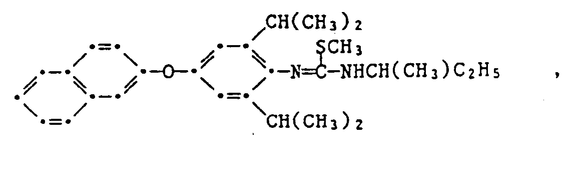 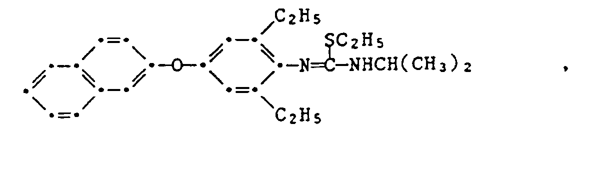 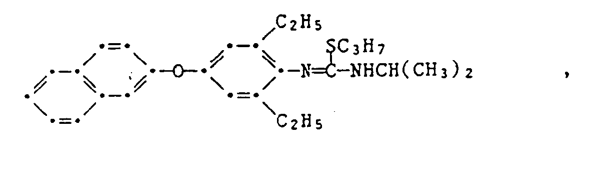 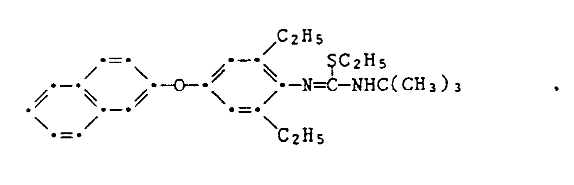 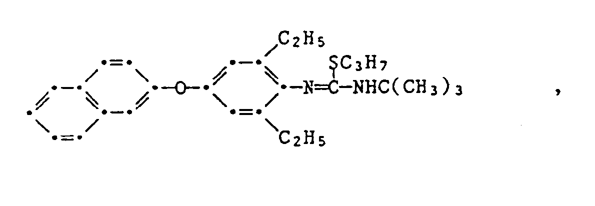 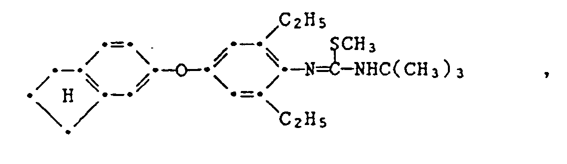 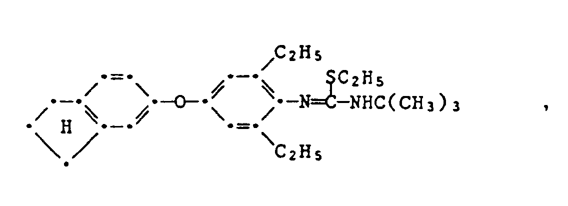 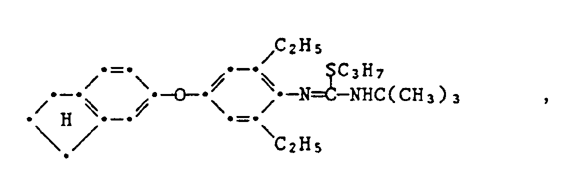 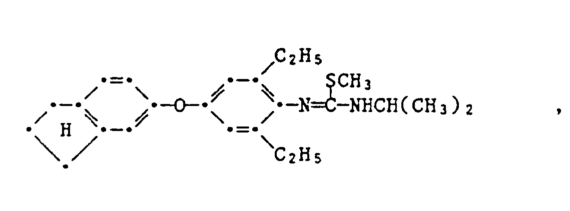 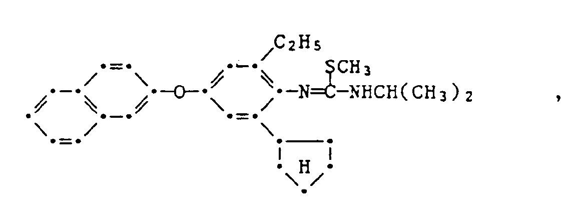 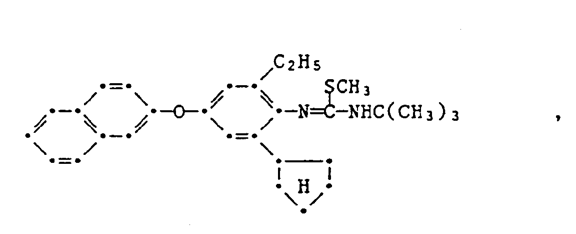 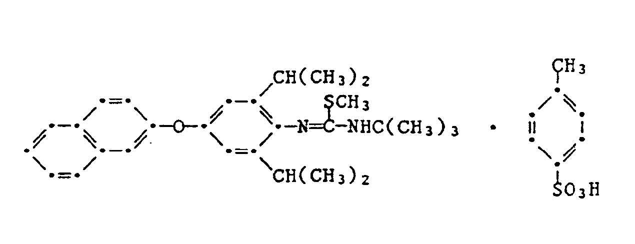 and

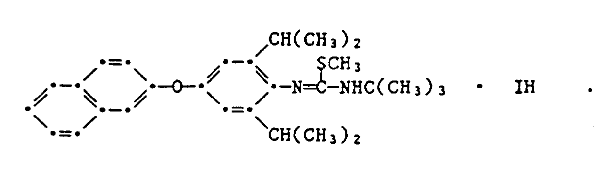

8. A process according to any one of claims 1, 2 and 5 for the preparation of the compounds of the formulae 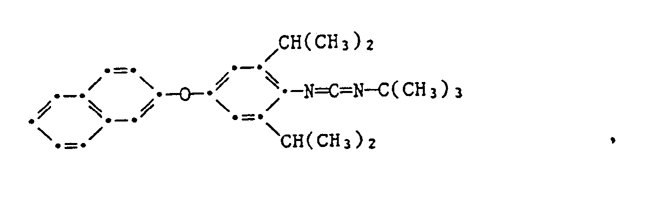 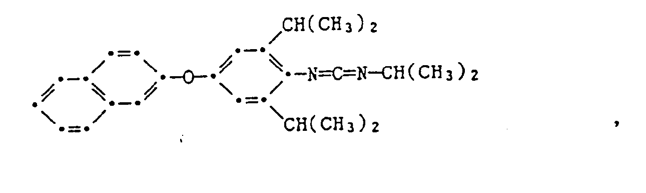 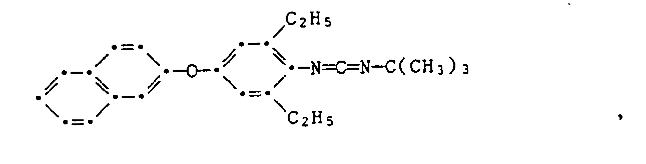 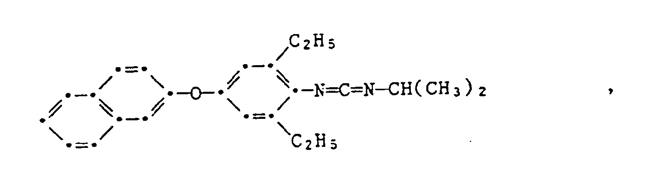 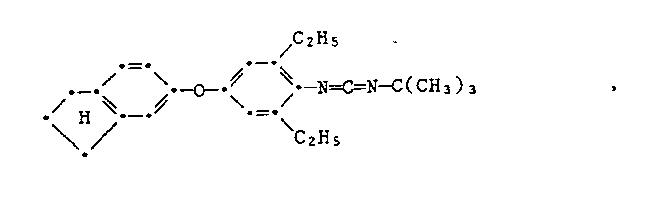 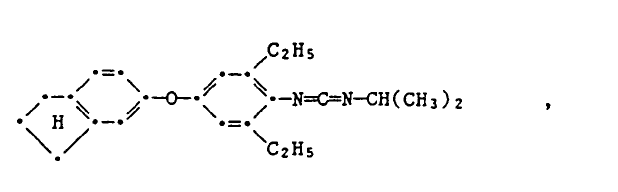 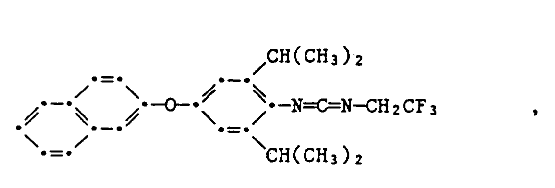 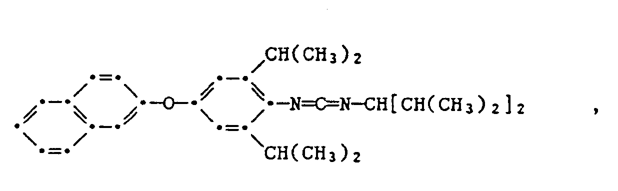 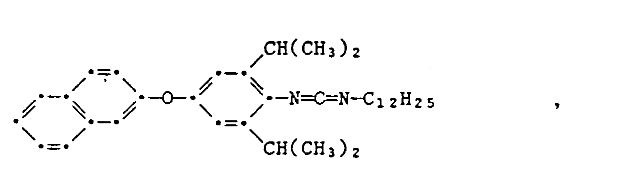 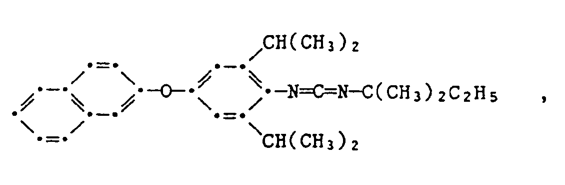 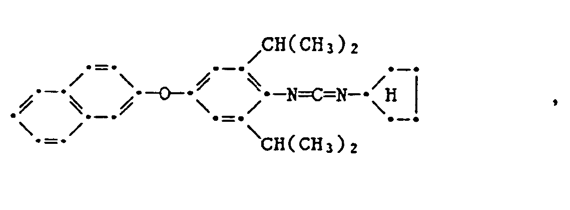 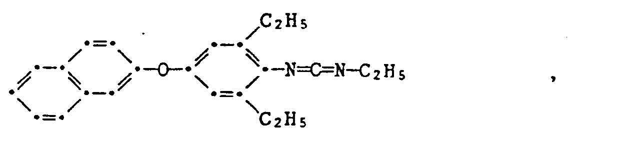 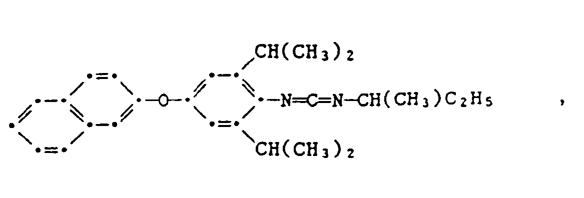 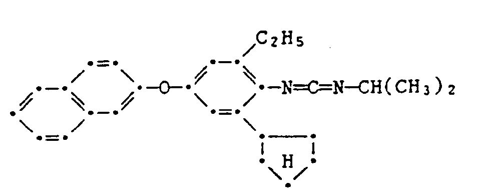 and

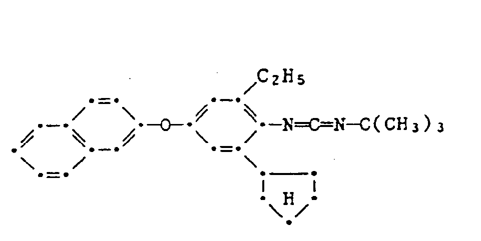

9. A pesticidal composition comprising as active ingredient at least one compound of formula I 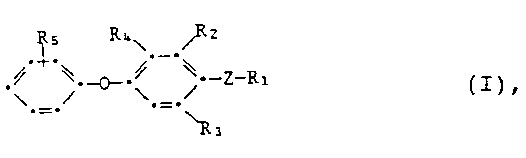 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-Cₛcycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, or one of the salts thereof with an organic or inorganic acid, together with suitable carriers and/or adjuvants.

10. A pesticidal composition according to claim 9 comprising as active ingredient at least one compound of formula I in which R₁ represents C₁-C₈alkyl, C₁-C₈alkyl mono- or poly-substituted by halogen and/or by C₁-C₅-alkoxy, or C₃-C₈cycloalkyl; R₂ represents C₁-C₅alkyl; R₃ represents C₁-C₅alkyl or cyclopentyl; R₄ represents hydrogen; R₅ represents a (CH=CH)₂, (CH₂)₃ or (CH₂)₄ bridge in the 2,3- or 3,4-position; Z represents -NH-CS-NH-, -N=C(SR₆)-NH- or-N=C=N-; and R₆ represents C₁-C₅alkyl or allyl.

11. A pesticidal composition according to claim 10, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents -NH-CS-NH-.

12. A pesticidal composition according to claim 10, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; Z represents -N=C(SR₆)-NH-; and R₆ represents C₁-C₃alkyl.

13. A pesticidal composition according to claim 10, in which R₁ represents C₁-C₄alkyl; R₂ and R₃ each represents C₁-C₃alkyl; R₄ represents hydrogen; R₅ represents a (CH=CH)₂ or (CH₂)₃ bridge in the 3,4-position; and Z represents -N=C=N-.

14. The use of a compound of formula I 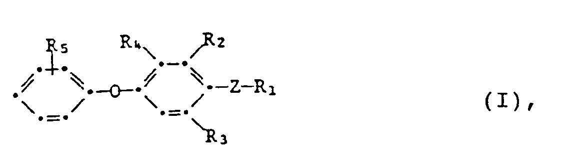 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-Cₛcycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, or one of the salts thereof with an organic or inorganic acid, for controlling pests in and on animals and plants.

15. The use according to claim 14 for controlling insects and arachnids.

16. A method of controlling pests in and on animals and plants, characterised in that the pests in their various stages of development are brought into contact with a compound of formula I 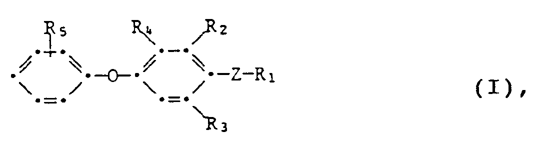 in which
R₁ represents C₁-C₁₂alkyl, C₁-C₁₂alkyl mono- or poly-substituted by halogen and/or by C₁-C₆alkoxy, C₃-C₈cycloalkyl, C₃-C₈cycloalkyl mono- or poly-substituted by C₁-C₃alkyl, or C₃-C₈cycloalkyl-C₁-C₄alkyl;
R₂ represents hydrogen or C₁-C₅alkyl;
R₃ represents C₁-C₅-alkyl or C₅-Cₛcycloalkyl;
R₄ represents hydrogen or methyl;
R₅ represents a (CH=CH)₂, (CH₂)₃ or (CH₂)₄ bridge in the 2,3- or 3,4-position;
Z represents -NH-CS-NH-, -N=C(SR₆)-NH- or -N=C=N- and
R₆ represents C₁-C₁₀alkyl or allyl, or with one of the salts thereof with an organic or inorganic acid.

17. A process for the preparation of a compound of formula V 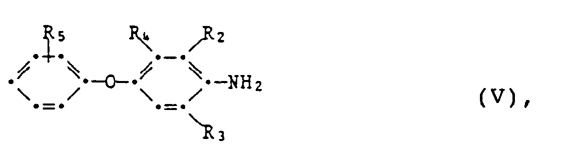 in which
R₂ represents hydrogen or C₁-C₅alkyl;
R₃ represents C₁-C₅alkyl or C₅-Cₛcycloalkyl;
R₄ represents hydrogen or methyl;
and R₅ represents a (CH=CH)₂, (CH₂)₃ or (CH₂)₄ bridge in the 2,3- or 3,4-position, characterised in that an aniline of formula VI 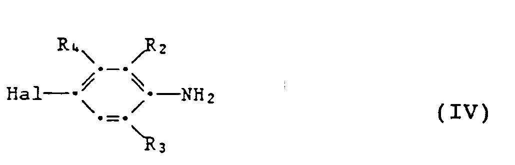 is reacted with a phenol of formula VII 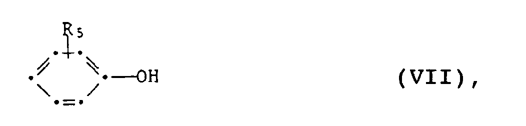
R₂, R₃, R₄ and R₅ having the meanings given for formula V and Hal representing halogen, especially chlorine or bromine.

18. A process according to claim 17, in which R₂ represents C₁-C₅alkyl; R₃ represents C₁-C₅alkyl or cyclopentyl; R₄ represents hydrogen; and R₅ represents a (CH=CH)₂, (CH₂)₃ or (CH₂)₄ bridge in the 2,3- or 3,4- position.

19. A process according to claim 18, in which R₂ and R₃ each represents C₁-C₃alkyl; R₄ represents hydrogen; and R₅ represents a (CH=CH)₂ or (CH₂)₃ bridge in the 3,4-position.

20. A process according to claim 19 for the preparation of the compounds of the formulae 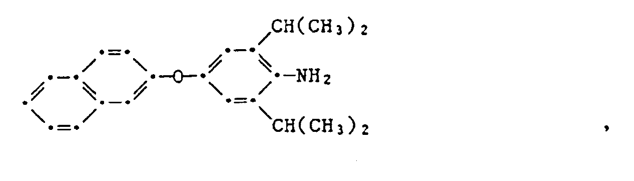 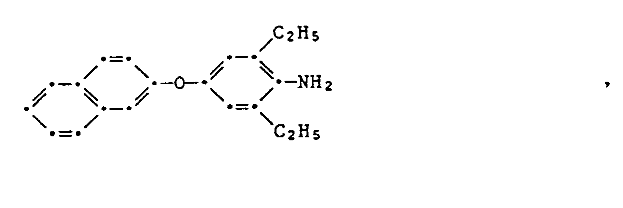 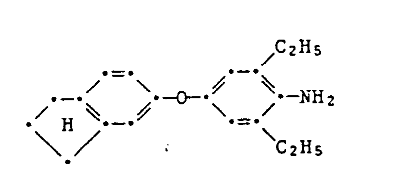 and

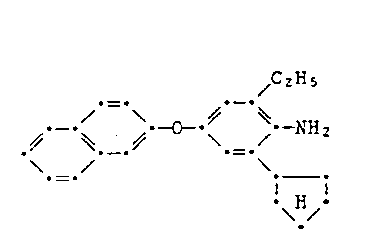

21. A process for the preparation of the compounds of formula II 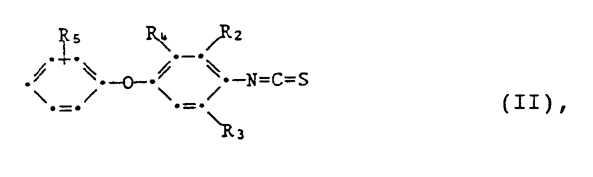 in which
R₂ represents hydrogen or C₁-C₅alkyl;
R₃ represents C₁-C₅alkyl or C₅-Cₛcycloalkyl;
R₄ represents hydrogen or methyl;
and R₅ represents a (CH=CH)₂, (CH₂)₃ or (CH₂)₄ bridge in the 2,3- or 3,4-position, characterised in that a phenoxyaniline of formula V 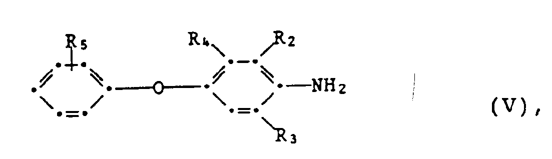 in which R₂, R₃, R₄ and R₅ have the meanings given for formula II, is reacted with thiophosgene.

22. A process according to claim 21, in which R₂ represents C₁-C₅alkyl; R₃ represents C₁-C₅alkyl or cyclopentyl; R₄ represents hydrogen; and R₅ represents a (CH=CH)₂, (CH₂)₃ or (CH₂)₄ bridge in the 2,3- or 3,4- position.

23. A process according to claim 22, in which R₂ and R₃ each represents C₁-C₃alkyl; R₄ represents hydrogen; and R₅ represents a (CH=CH)₂ or (CH₂)₃ bridge in the 3,4-position.

24. A process according to claim 23 for the preparation of compounds of the formulae 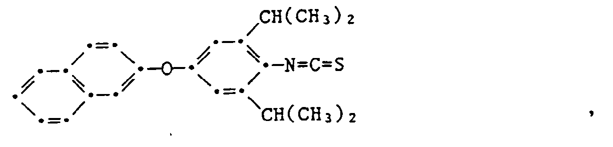 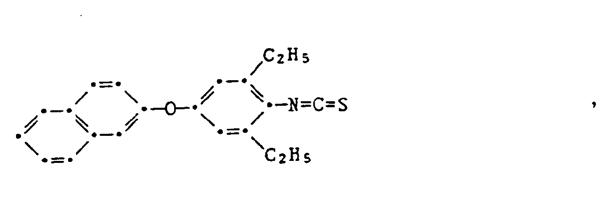 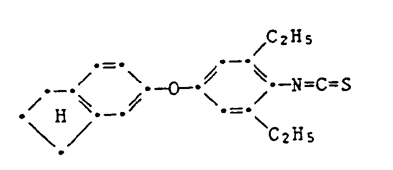 and

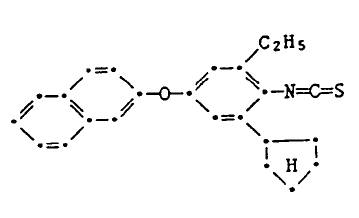

## Revendications (Revendications pour l'(les) Etat(s) contractant(s) suivant(s) : suivants : AT, BE, CH, DE, FR, GB, GR, IT, LI, NL)

1. Composés de formule I 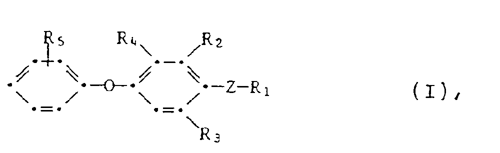 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ;
R₅ représente un pont (CH=CH)₂, (CH₂)₃, (CH₂)₄ en position 2,3 ou 3,4 ;
Z représente -NH-CS-NH-, -N=C(SR₆)-NH- ou -N=C=N-, et
R₆ représente un groupe alkyle en C 1-C 10 ou allyle, et leurs sels d'acides organiques ou minéraux.

2. Composés de formule 1 de la revendication 1, dans laquelle R₁ représente un groupe alkyle en C 1-C 8, un groupe alkyle en C 1-C 8 portant un ou plusieurs substituants choisis parmi les halogènes et/ou les groupes alcoxy en C 1-C 5, ou un groupe cycloalkyle en C 3-C 8 ; R₂ représente un groupe alkyle en C 1-C 5 ; R₃ représente un groupe alkyle en C 1-C 5 ou cyclopentyle ; R₄ représente l'hydrogène ; R₅ représente un pont (CH=CH)₂, (CH₂)₃ ou (CH₂)₄ en position 2,3 ou 3,4 ; Z représente -NH-CS-NH-, -N=C(SR₆)-NH- ou -N=C=N- ; et R₆ représente un groupe alkyle en C 1-C 5 ou allyle.

3. Composés de formule I de la revendication 2, dans laquelle R₁ représente un groupe alkyle en C 1-C 4 ; R₂ et R₃ représentent chacun un groupe alkyle en C 1-C 3 ; R₄ représente l'hydrogène ; R₅ représente un pont (CH=CH)₂ ou (CH₂)₃ en position 3,4 ; et Z représente -NH-CS-NH-.

4. Composés de formule I de la revendication 2, dans laquelle R₁ représente un groupe alkyle en C 1-C 4 ; R₂ et R₃ représentent chacun un groupe alkyle en C 1-C 3 ; R₄ représente l'hydrogène ; R₅ représente un pont (CH=CH)₂ ou (CH₂)₃ en position 3,4 ; Z représente -N=C(SRₛ)-NH- ; et R₆ représente un groupe alkyle en C 1-C 3.

5. Composés de formule I de la revendication 2, dans laquelle R₁ représente un groupe alkyle en C 1-C 4 ; R₂ et R₃ représentent chacun un groupe alkyle en C 1-C 3 ; R₄ représente l'hydrogène ; R5 représente un pont (CH=CH)₂ ou (CH₂)₃ en position 3,4 ; et Z représente -N=C=N-.

6. Les composés selon l'une des revendications 1 à 3, de formules 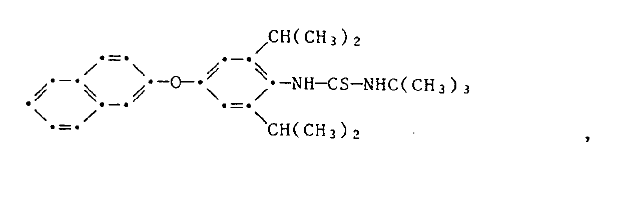 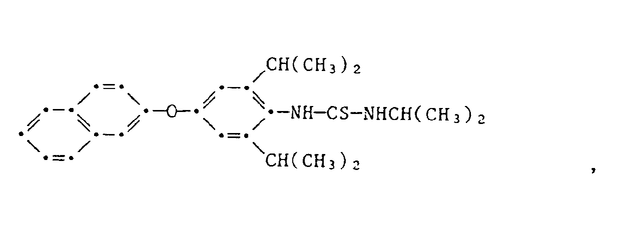 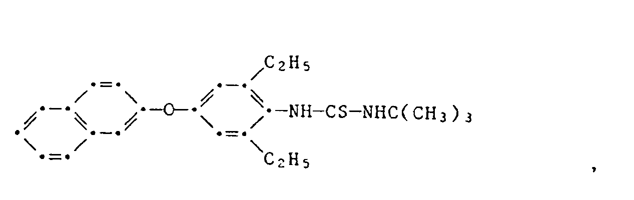 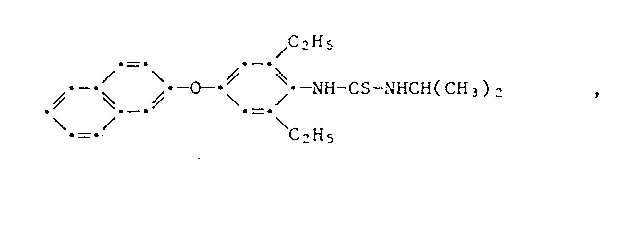 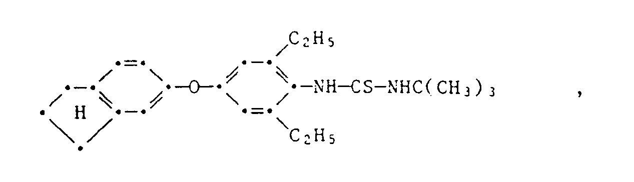 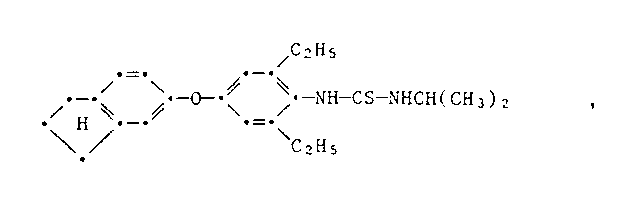 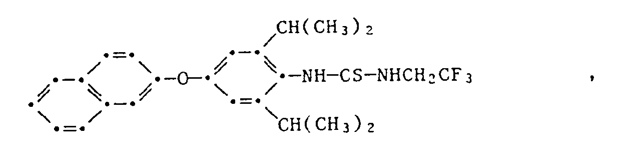 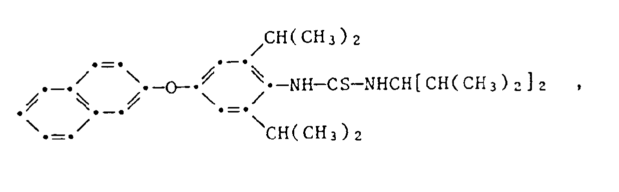 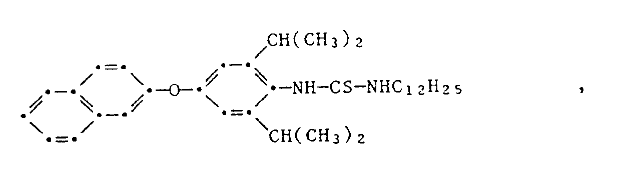 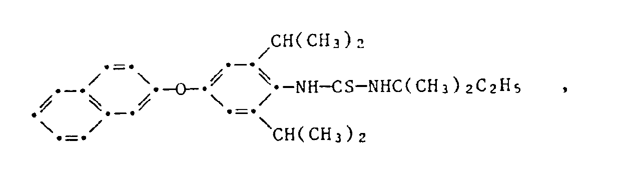 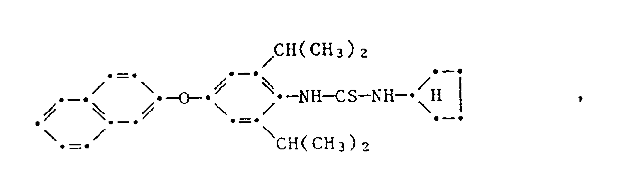 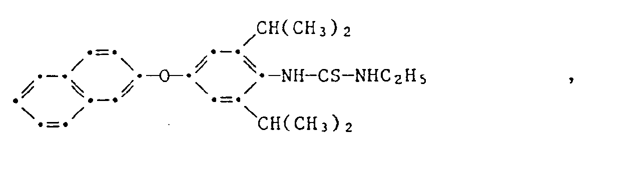 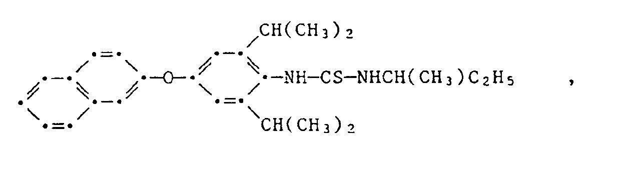 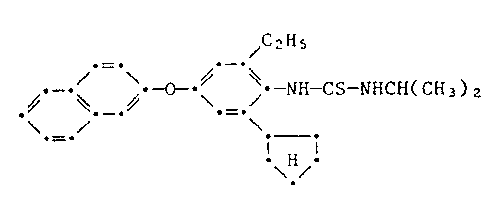 et

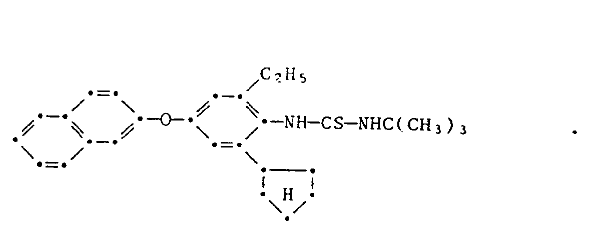

7. Les composés selon l'une des revendications 1, 2 ou 4, de formules 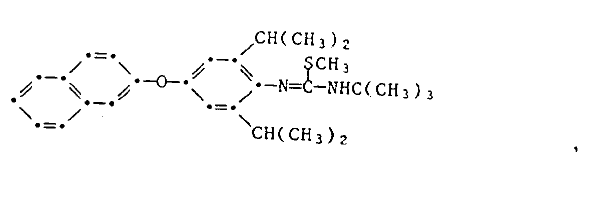 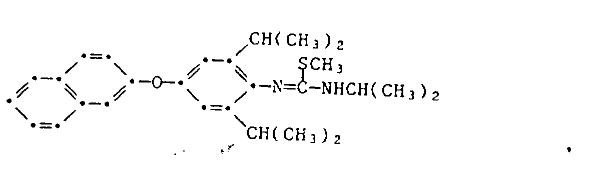 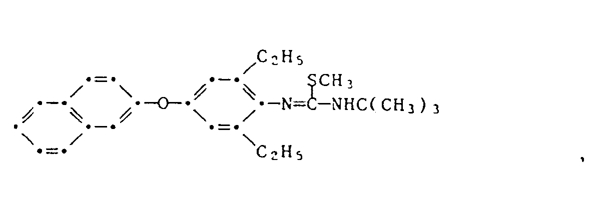 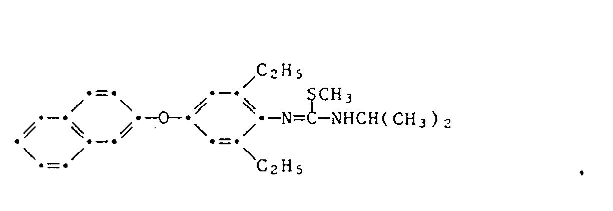 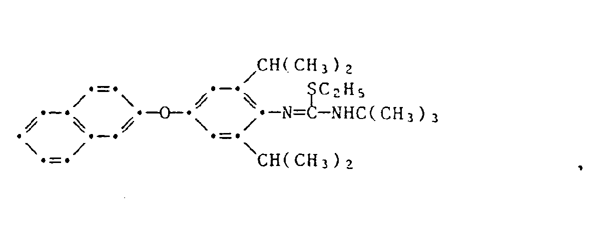 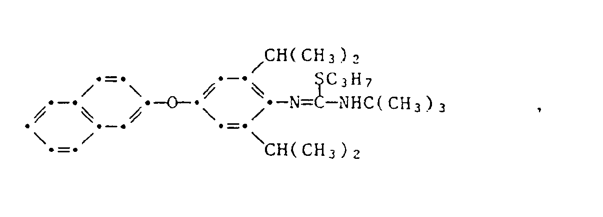 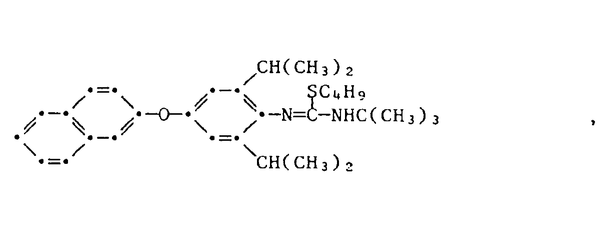 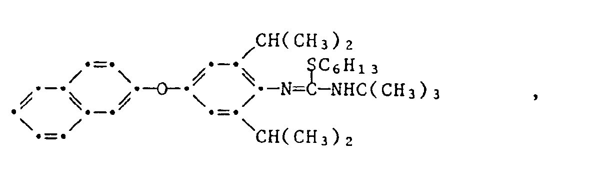 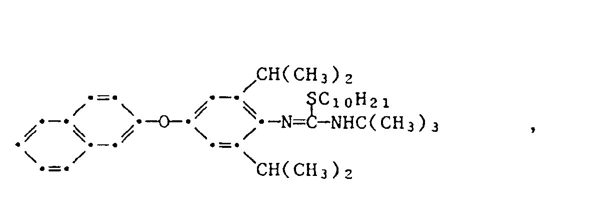 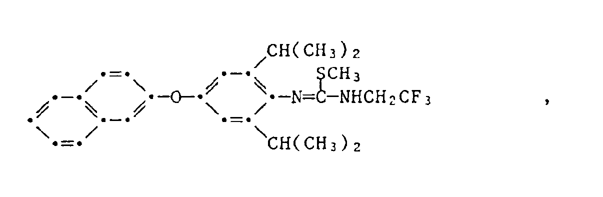 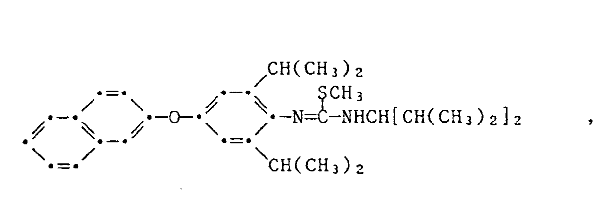 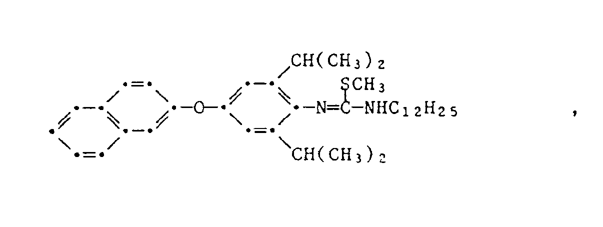 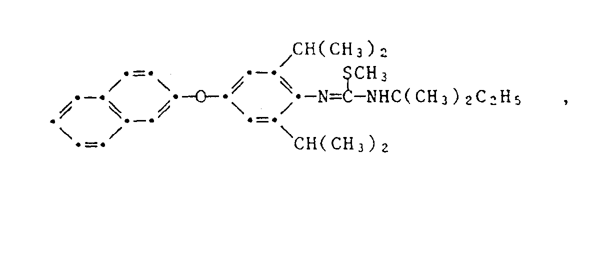 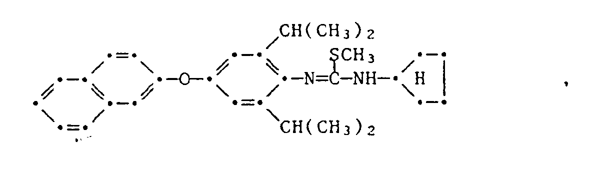 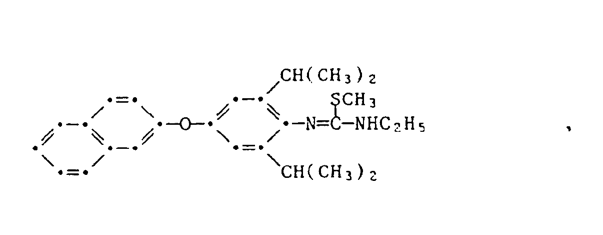 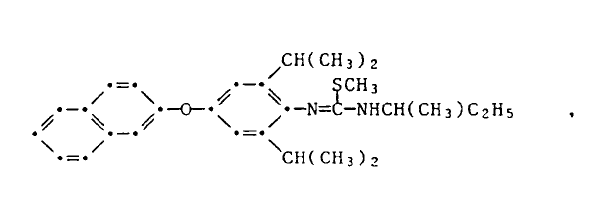 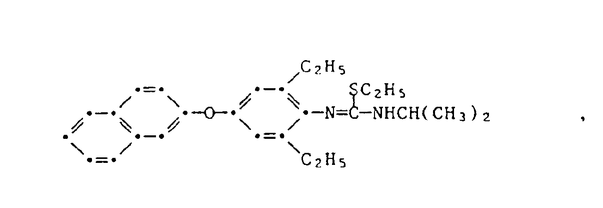 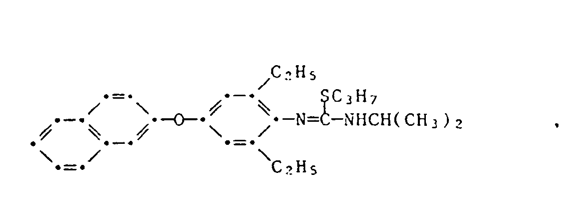 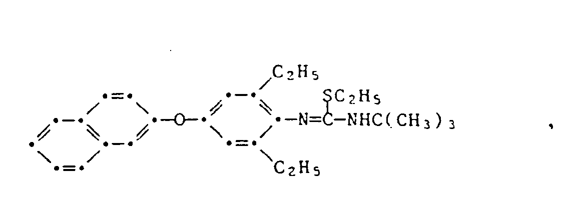 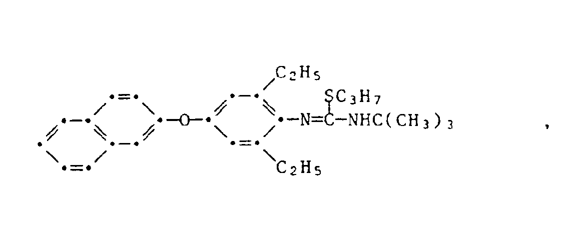 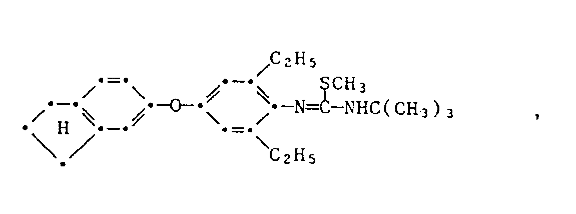 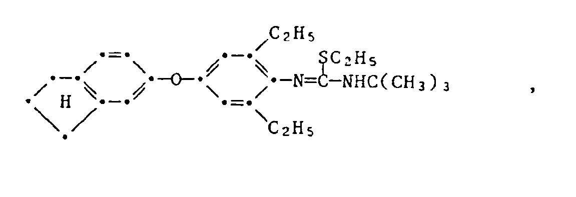 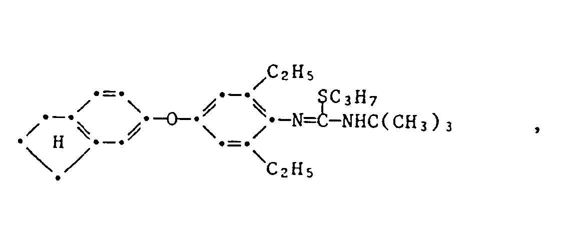 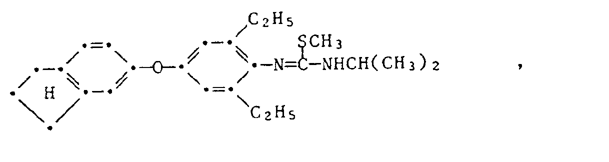 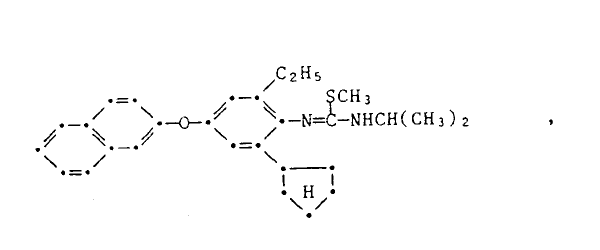 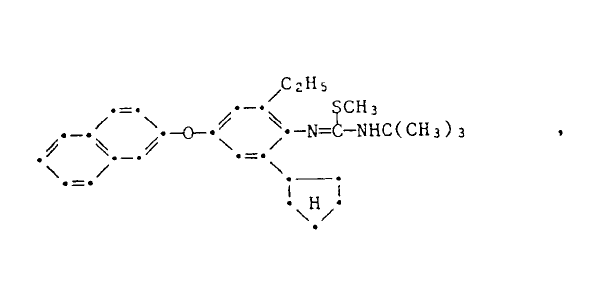 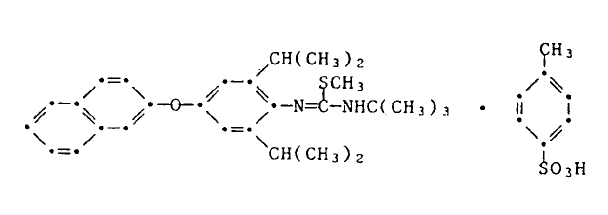 et

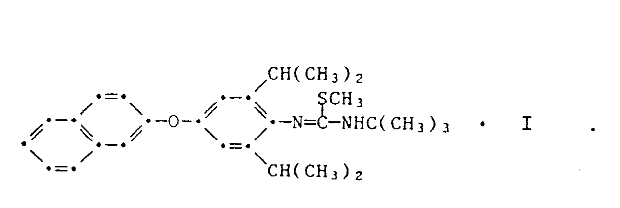

8. Les composés selon l'une des revendications 1, 2 ou 5, de formules 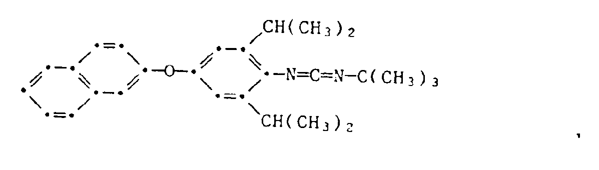 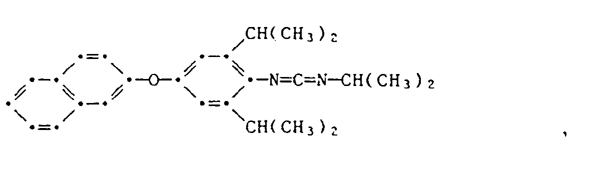 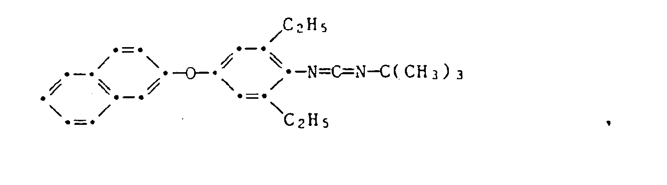 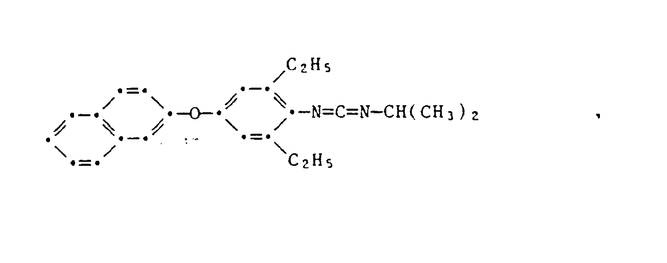 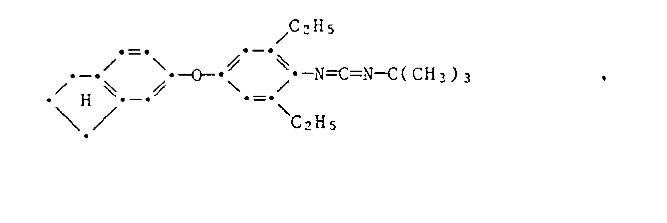 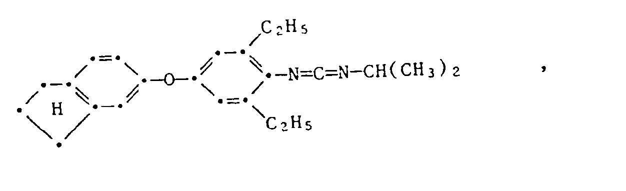 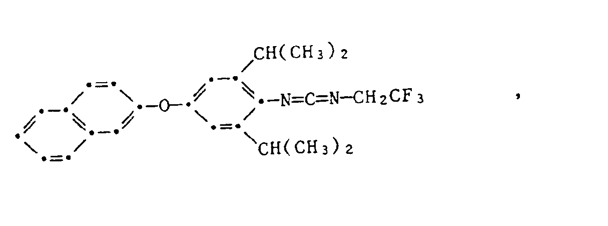 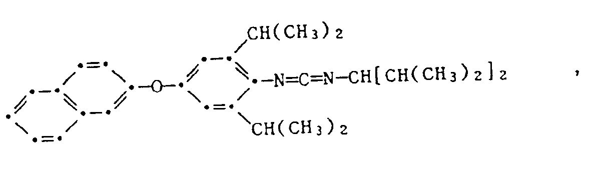 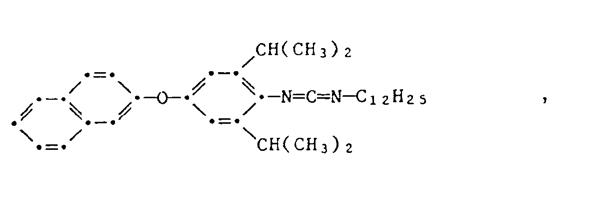 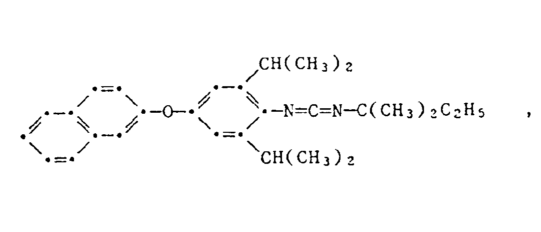 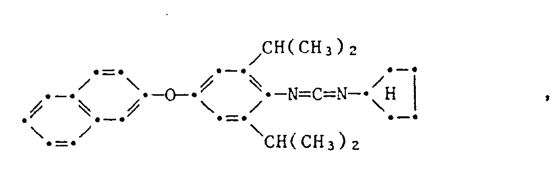 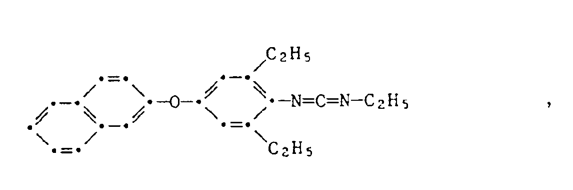 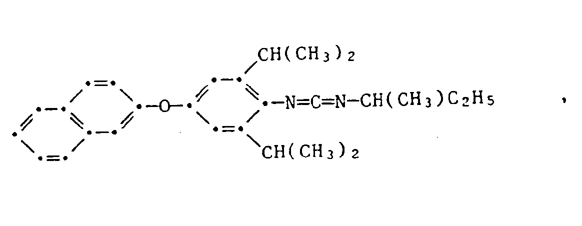 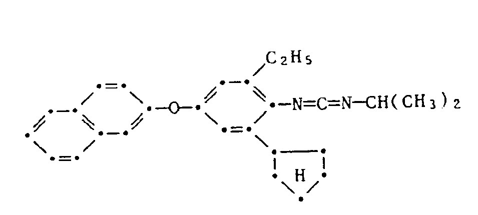 et

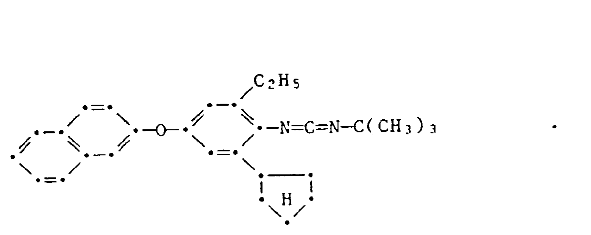

9. Procédé de préparation d'un composé de formule 1 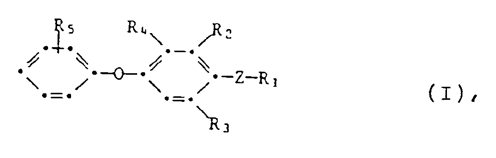 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ;
R₅ représente un pont (CH=CH)₂, (CH₂)₃, (CH₂)₄ en position 2,3 ou 3,4 ;
Z représente -NH-CS-NH-, -N=C(SR₆)-NH- ou -N=C=N-, et
R₆ représente un groupe alkyle en C 1-C 10 ou allyle, caractérisé en ce que :
A) on fait réagir un isothiocyanate de formule II 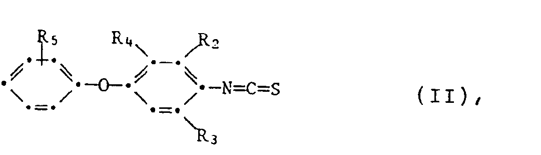 avec une amine de formule III 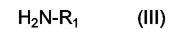 dans un solvant ou diluant organique, à pression normale et à une température de 0 à +150_{°}C, la réaction donnant une thiourée, et le cas échéant
B) on fait réagir cette thiourée avec un composé de formule IV 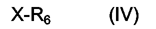 dans un solvant organique inerte à pression normale ou légèrement supérieure à la normale et à une température de +10 à +250_{°}C, ce qui donne une isothiourée, ou bien
C) on convertit la thiourée, par scission de sulfure d'hydrogène dans un solvant ou diluant organique apro- tonique, à pression normale et à une température de 0 à +150°C, en le carbodiimide, les symboles R₁, R₂, R₃, R₄, R₅ et R₆ ayant les significations indiquées ci-dessus et X représentant un substituant éliminable.

10. Produit pesticide contenant au moins un composant actif consistant en un composé de formule 1 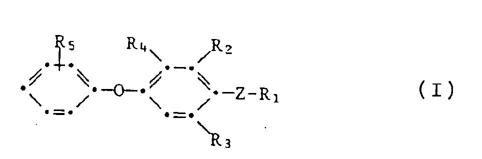 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ;
R₅ représente un pont (CH=CH)₂, (CH₂)₃, (CH₂)₄ en position 2,3 ou 3,4 ;
Z représente -NH-CS-NH-, -N=C(SR₆)-NH- ou -N=C=N-, et
R₆ représente un groupe alkyle en C 1-C 10 ou allyle, ou l'un de ses sels d'acide organique ou minéral, avec des véhicules et/ou additifs appropriés.

11. Produit pesticide selon la revendication 10, contenant au moins un composant actif consistant en un composé de formule I dans laquelle R₁ représente un groupe alkyle en C 1-C 8, un groupe alkyle en C 1-C 8 portant un ou plusieurs substituants choisis parmi les halogènes et/ou les groupes alcoxy en C 1-C 5, ou un groupe cycloalkyle en C 3-C 8 ; R₂ représente un groupe alkyle en C 1-C 5 ; R₃ représente un groupe alkyle en C 1-C 5 ou cyclopentyle ; R₄ représente l'hydrogène ; R₅ représente un pont -(CH=(̵CH)̵₂, -(̵CH₂)̵₃ ou (̵CH₂)̵₄ en position 2,3 ou 3,4 ; Z représente -NH-CS-NH-, -N=C(SR₆)-NH- ou -N=C=N- ; et R₆ représente un groupe alkyle en C 1-C 5 ou allyle.

12. Produit pesticide selon la revendication 11, pour lequel R₁ représente un groupe alkyle en C 1-C 4 ; R₂ et R₃ représentent chacun un groupe alkyle en C 1-C 3 ; R₄ représente l'hydrogène ; R₅ représente un pont (̵CH=CH)̵₂ ou (̵CH_{2)̵3} en position 3,4 ; et Z représente -NH-CS-NH-.

13. Produit pesticide selon la revendication 11, pour lequel R₁ représente un groupe alkyle en C 1-C 4 ; R₂ et R₃ représentent chacun un groupe alkyle en C 1-C 3 ; R₄ représente l'hydrogène ; R₅ représente un pont (̵CH=CH)̵₂ ou (̵CH₂)̵₃ en position 3,4 ; Z représente -NH=C(SRₛ)-NH- ; et R₆ représente un groupe alkyle en C 1-C 3.

14. Produit pesticide selon la revendication 11, pour lequel R₁ représente un groupe alkyle en C 1-C 4 ; R₂ et R₃ représentent chacun un groupe alkyle en C 1-C 3 ; R₄ représente l'hydrogène ; R₅ représente un pont (̵H=CH)̵₂ ou (̵CH₂)̵₃ en position 3,4 ; et Z représente -N=C=N-.

15. Utilisation d'un composé de formule 1 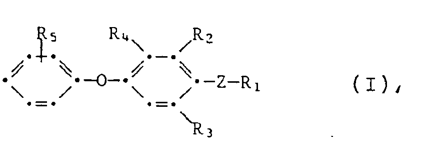 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4 ; R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ;
R₅ représente un pont (̵CH=CH)̵₂, {CH₂)̵₃ ou (̵CH₂)̵₄ en position 2,3 ou 3,4 ;
Z représente -NH-CS-NH-, -N=C(SR₆)-NH- ou -N=C=N-, et
R₆ représente un groupe alkyle en C 1-C 10 ou allyle,
ou de l'un de ses sels d'acides organiques ou minéraux pour la lutte contre les parasites des animaux et des végétaux.

16. Utilisation selon la revendication 15, pour la lutte contre les insectes et les arachnides.

17. Procédé pour combattre les parasites des animaux et des végétaux, caractérisé en ce que l'on met les parasites, dans leurs divers stades de développement, en contact avec un composé de formule 1 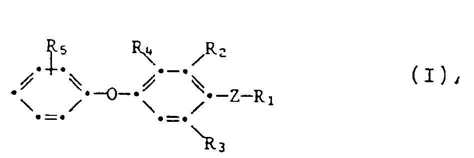 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3, ou un groupe cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ;
R₅ représente un pont -(̵CH=CH_{)̵2}, (̵CH₂)̵₃ ou (̵CH₂)̵₄ en position 2,3 ou 3,4 ;
Z représente -NH-CS-NH-, -N=C(SR₆)-NH-, -N=C=N-, et
R₆ représente un groupe alkyle en C 1-C 10 ou allyle, ou avec l'un de ses sels d'acide organique ou minéral.

18. Composé de formule V 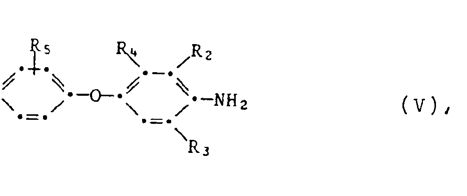 dans laquelle
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ; et
R₅ représente un pont (̵CH=CH)̵₂, (̵CH₂)̵₃, (̵CH₂)̵₄ en position 2,3 ou 3,4.

19. Composés de formule V de la revendication 18, dans laquelle R₂ représente un groupe alkyle en C 1-C 5 ; R₃ représente un groupe alkyle en C 1-C 5 ou cyclopentyle; R₄ représente l'hydrogène ; R₅ représente un pont (̵CH=CH)̵₂, (̵CH₂)̵₃, (̵CH₂)̵₄ en position 2,3 ou 3,4.

20. Composés de formule V de la revendication 19, dans laquelle R₂ et R₃ représentent chacun un groupe alkyle en C 1-C 3 ; R₄ représente l'hydrogène et R₅ représente un pont (̵CH=CH)̵₂ ou (̵CH₂)̵₃ en position 3,4.

21. Les composés de la revendication 20, de formules 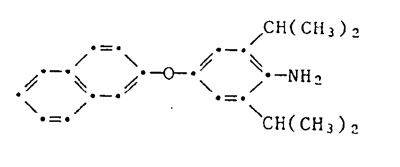 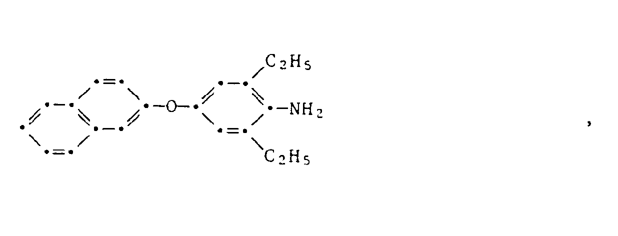 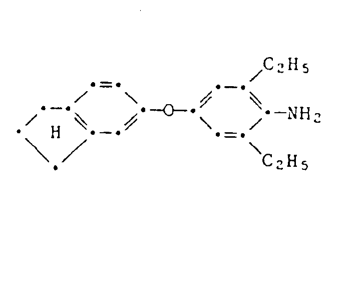 et

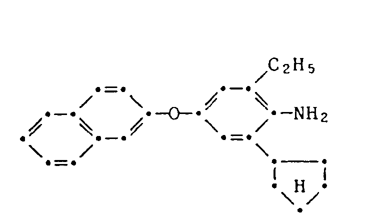

22. Composés de formule Il 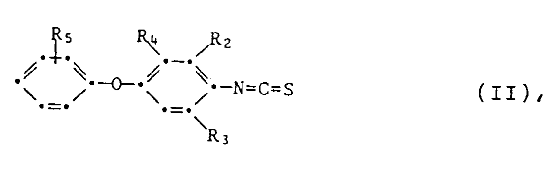 dans laquelle
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ; et
R₅ représente un pont (̵CH=CH)̵₂, (̵CH₂)̵₃, (̵CH₂)̵₄ en position 2,3 ou 3,4.

23. Composés de formule II de la revendication 22, dans laquelle R₂ représente un groupe alkyle en C 1-C 5 ; R₃ représente un groupe alkyle en C 1-C 5 ou cyclopentyle ; R₄ représente l'hydrogène et R₅ représente un pont (̵CH=CH)̵₂, (̵CH₂)̵₃, (̵CH₂)̵₄ en position 2,3 ou 3,4.

24. Composés de formule II de la revendication 23, dans laquelle R₂ et R₃ représentent chacun un groupe alkyle en C 1-C 3 ; R₄ représente l'hydrogène et R₅ un pont (̵CH=CH)̵₂ ou (̵CH₂)̵₃ en position 3,4.

25. Les composés de la revendication 24, de formules

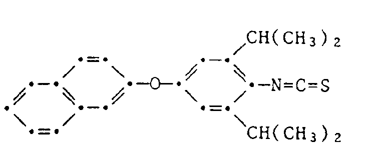

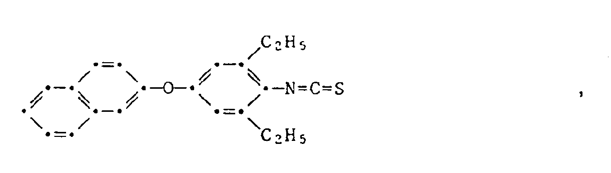

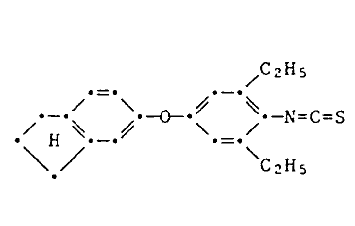

et

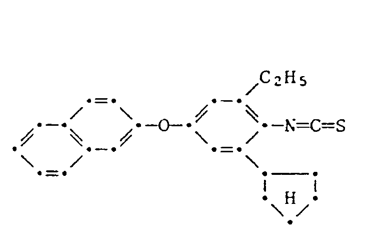

## Revendications (Revendications pour l'(les) Etat(s) contractant(s) suivant(s) : suivant : ES)

1. Procédé de préparation d'un composé de formule 1 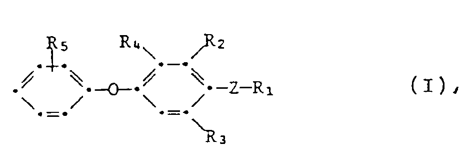 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3, ou un groupe (cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ;
R₅ représente un pont -(CH=CH)̵₂, (̵CH₂)̵₃, (̵CH₂)̵₄ en position 2,3 ou 3,4 ;
Z représente -NH-CS-NH-, -N=C(SR₆)-NH-, -N=C=N-, et
R₆ représente un groupe alkyle en C 1-C 10 ou allyle,
caractérisé en ce que :
A) on fait réagir un isothiocyanate de formule II 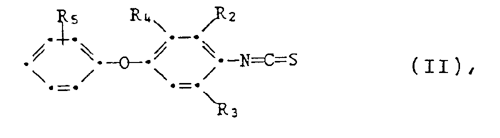 avec une amine de formule III 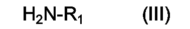 dans un solvant ou diluant organique à pression normale et à une température de 0 à +150_{°}C, la réaction donnant une thiourée, et le cas échéant
B) on fait réagir cette thiourée avec un composé de formule IV 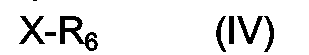 dans un solvant organique inerte, à pression normale ou légèrement supérieure à la normale et à une température de +10 à 250°C, la réaction donnant une isothiourée, ou bien
C) on convertit la thiourée, par scission de sulfure d'hydrogène dans un solvant ou diluant organique apro- tonique à pression normale et à une température de 0 à +150°C, en le carbodiimide, les symboles R_{1'} R₂, R₃, R₄, R₅ et R₆ ayant les significations indiquées ci-dessus et X représentant un substituant éliminable.

2. Procédé selon la revendication 1, dans lequel R₁ représente un groupe alkyle en C 1-C 8, un groupe alkyle en C 1-C 8 portant un ou plusieurs substituants choisis parmi les halogènes et/ou les groupes alcoxy en C 1-C 5, ou un groupe cycloalkyle en C 3-C 8 ; R₂ représente un groupe alkyle en C 1-C 5 ; R₃ représente un groupe alkyle en C 1-C 5 ou cyclopentyle ; R₄ représente l'hydrogène ; R₅ représente un pont (̵CH=CH)̵₂, (̵CH₂)̵₃, (̵CH₂)̵₄ en position 2,3 ou 3,4 ; Z représente -NH-CS-NH-, -N=C(SRₛ)-NH-, -N=C=N-, et R₆ représente un groupe alkyle en C 1-C 5 ou allyle.

3. Procédé selon la revendication 2, dans laquelle R₁ représente un groupe alkyle en C 1-C 4 ; R₂ et R₃ représentent chacun un groupe alkyle en C 1-C 3 ; R₄ représente l'hydrogène ; R₅ représente un pont (̵CH=CH)̵₂ ou (̵CH₂)̵₃ en position 3,4 ; et Z représente -NH-CS-NH-.

4. Procédé selon la revendication 2, dans laquelle R₁ représente un groupe alkyle en C 1-C 4 ; R₂ et R₃ représentent chacun un groupe alkyle en C 1-C 3 ; R₄ représente l'hydrogène ; R₅ représente un pont (̵CH=CH)̵₂ ou (̵CH₂)̵₃ en position 3,4 ; Z représente -N=C(SR₆)-NH-, et R₆ représente un groupe alkyle en C 1-C 3.

5. Procédé selon la revendication 2, dans lequel R₁ représente un groupe alkyle en C 1-C 4 ; R₂ et R₃ représentent chacun un groupe alkyle en C 1-C 3 ; R₄ représente l'hydrogène ; R₅ représente un pont (̵CH=CH)̵₂ ou (̵CH₂)̵₃ en position 3,4 ; et Z représente -N=C=N-.

6. Procédé selon l'une des revendications 1 à 3, pour la préparation des composés de formules

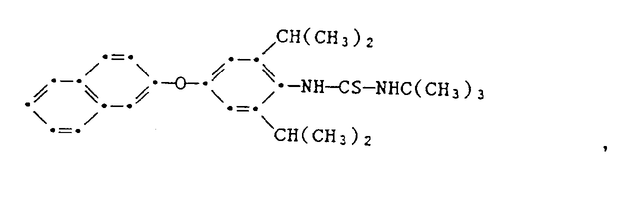

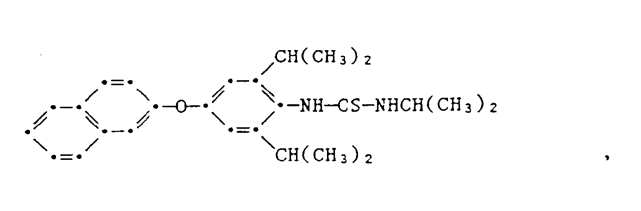

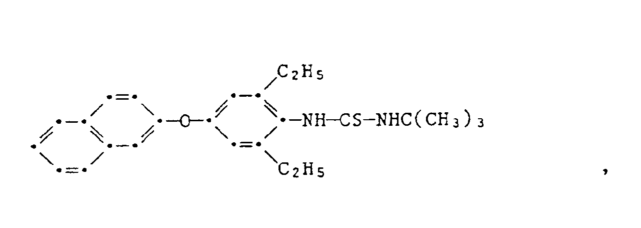

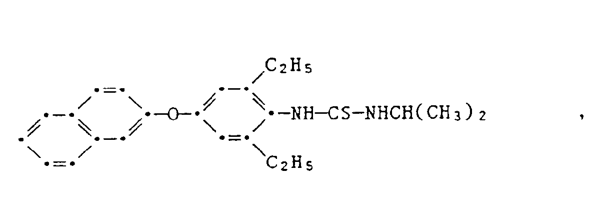

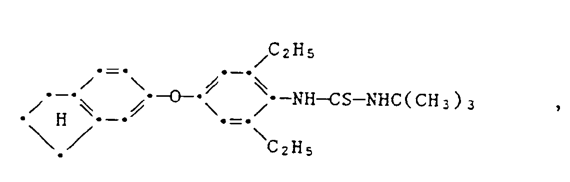

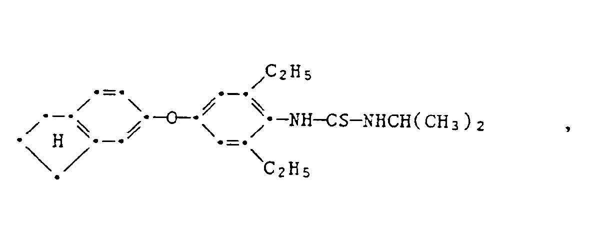

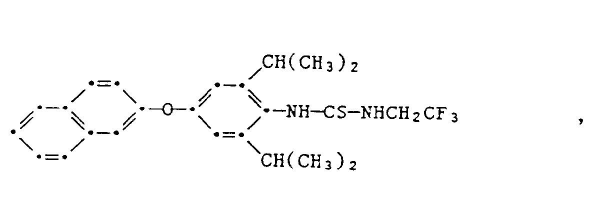

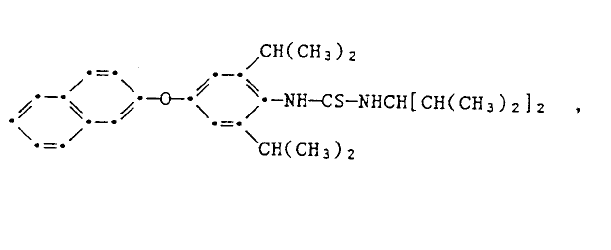

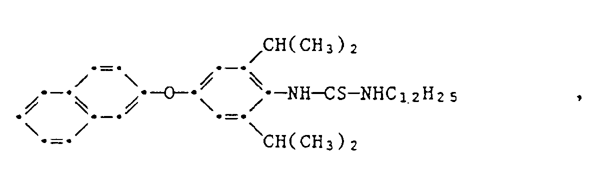

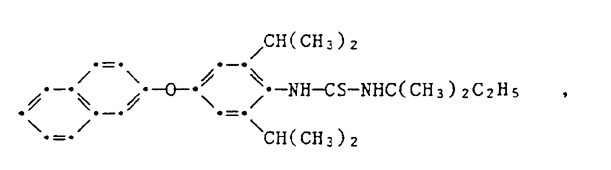

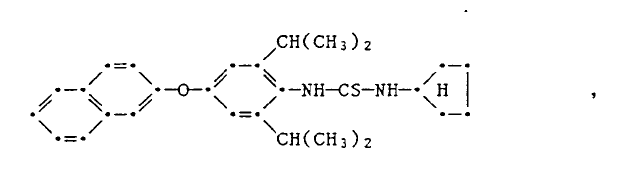

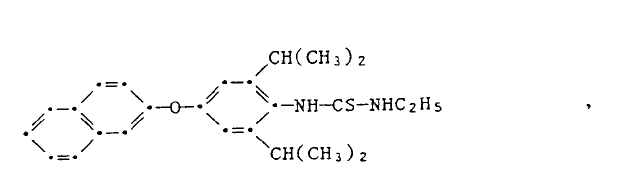

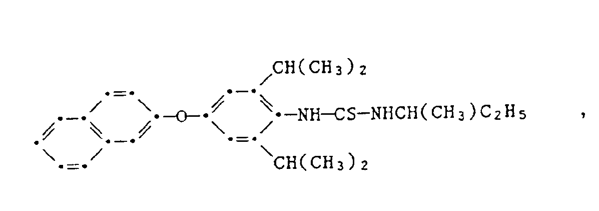

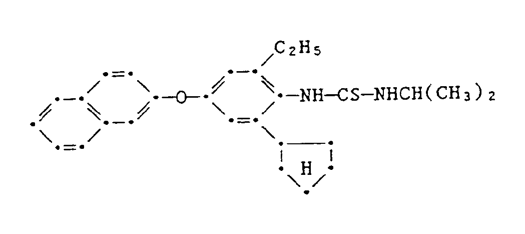

et

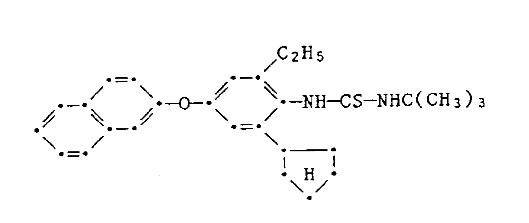

7. Procédé selon l'une des revendications 1, 2 ou 4, pour la préparation des composés de formules 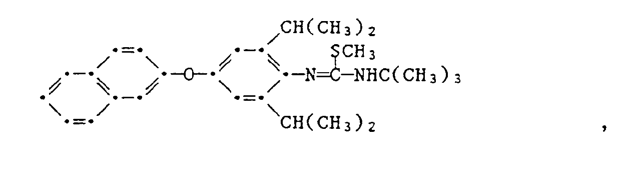 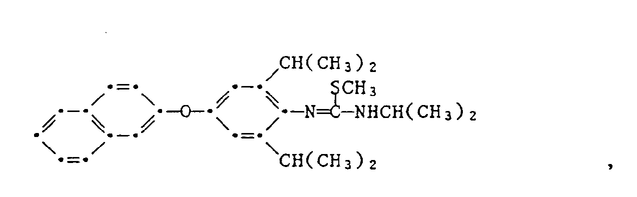 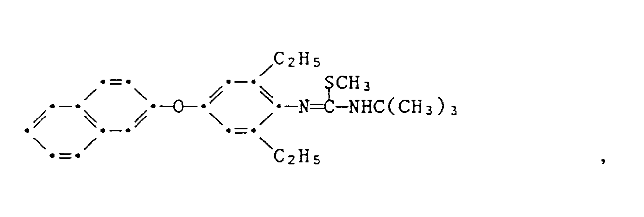 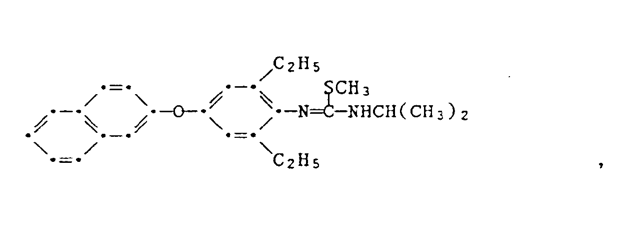 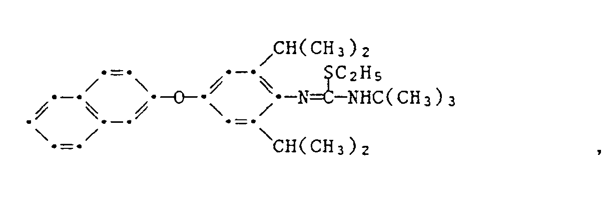 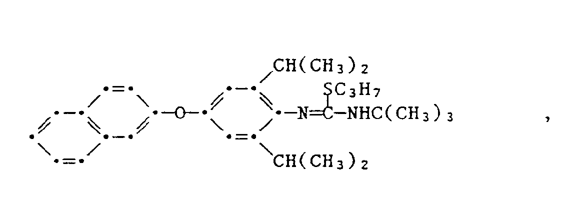 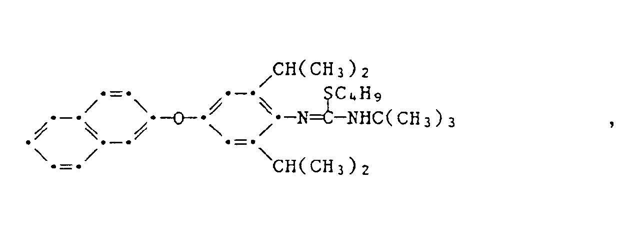 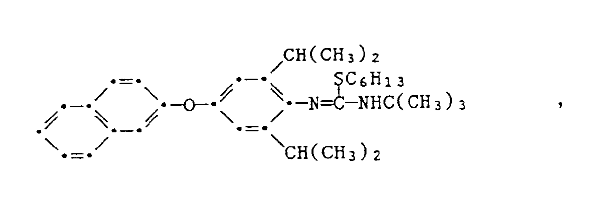 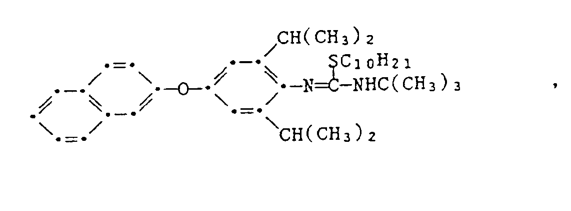 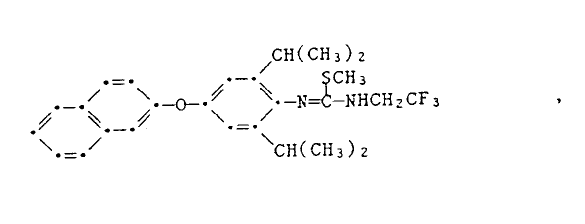 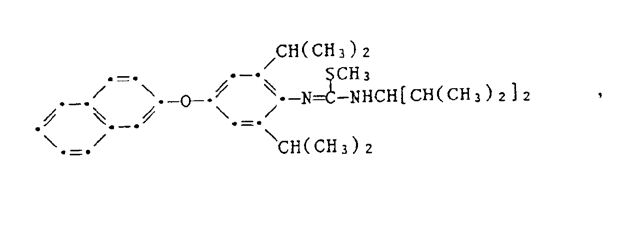 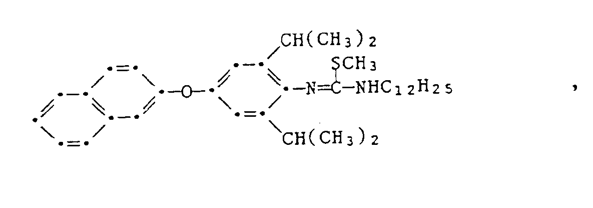 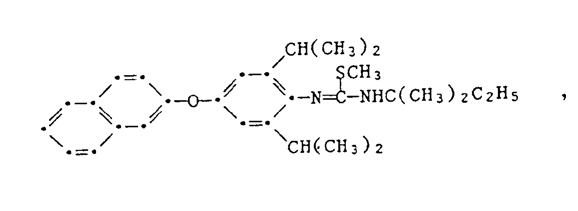 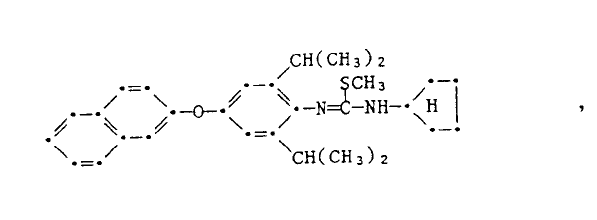 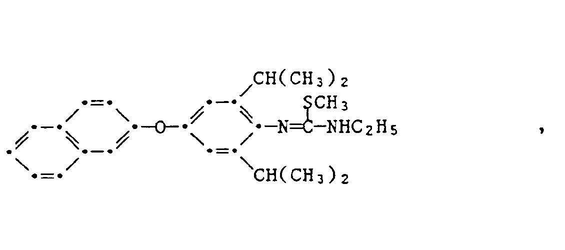 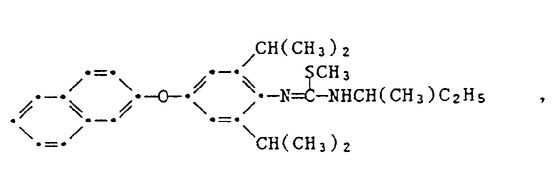 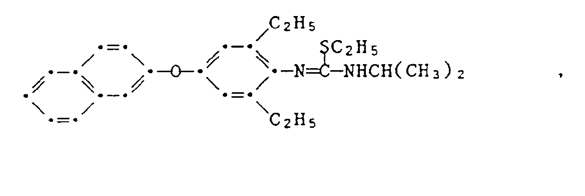 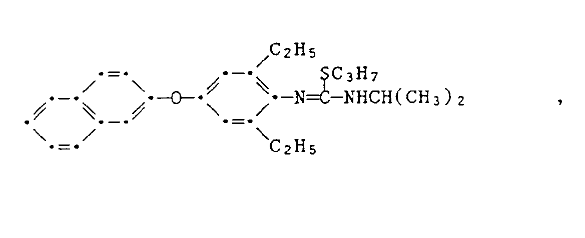 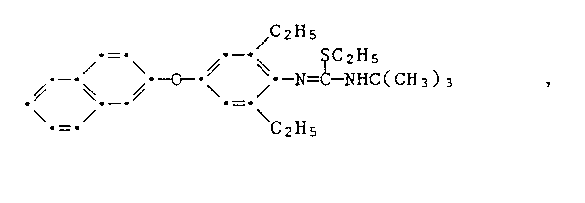 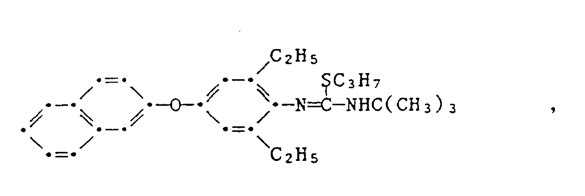 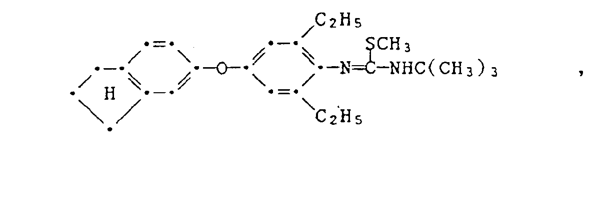 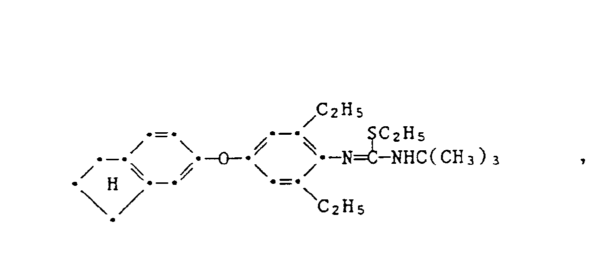 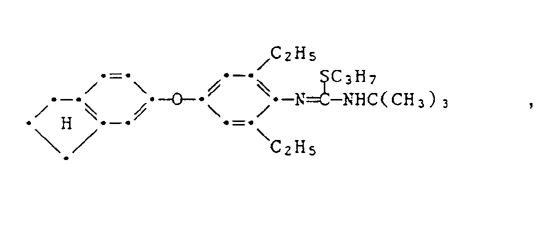 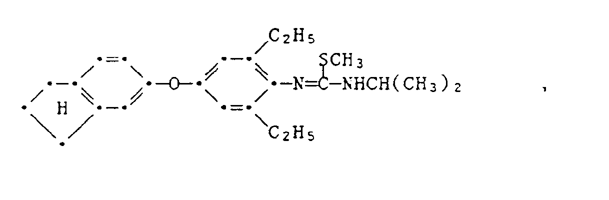 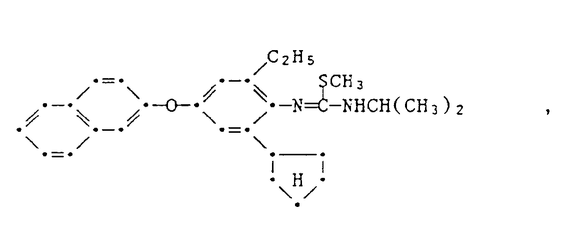 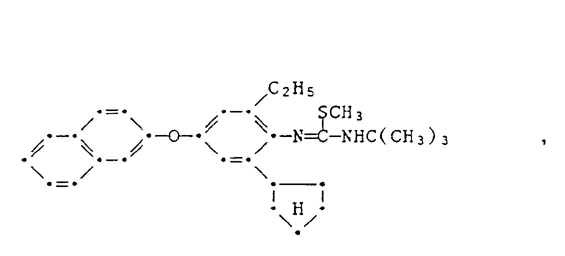 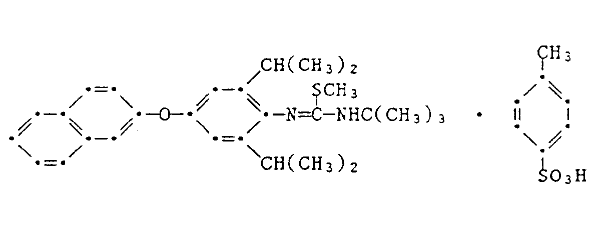 et

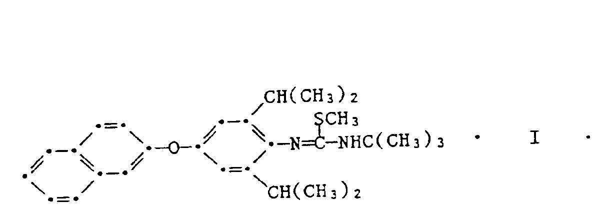

8. Procédé selon l'une des revendications 1, 2 ou 5, pour la préparation des composés de formules 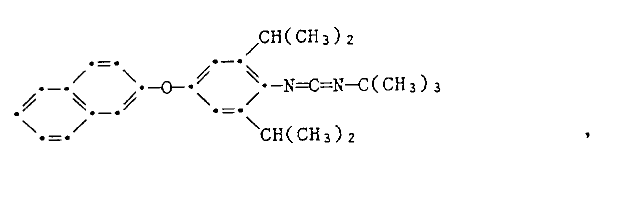 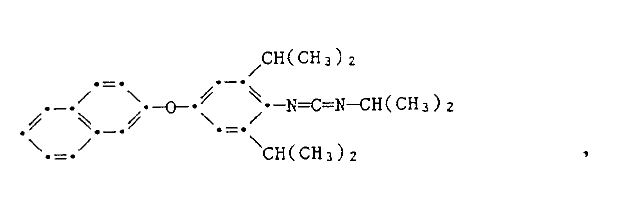 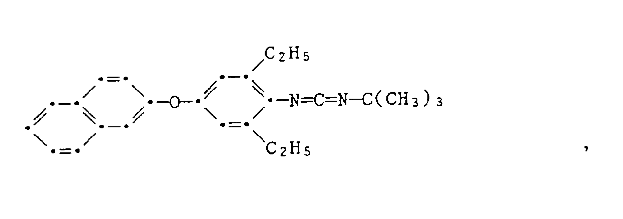 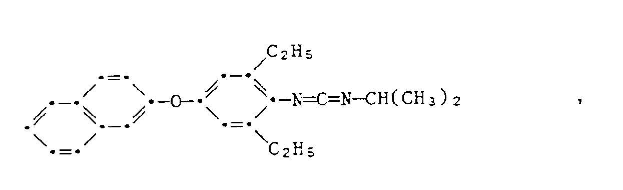 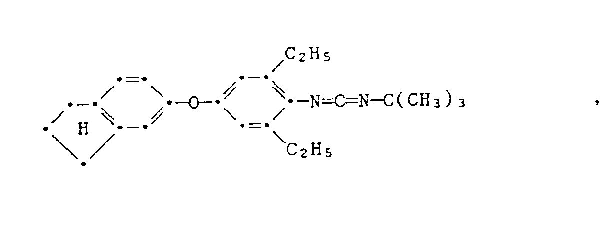 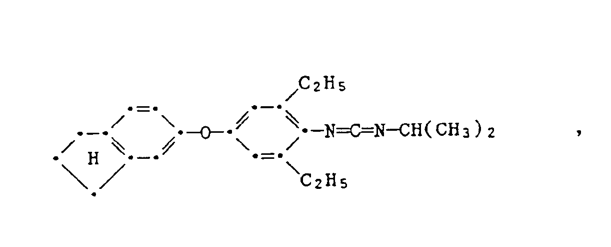 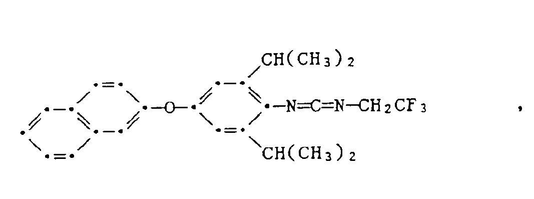 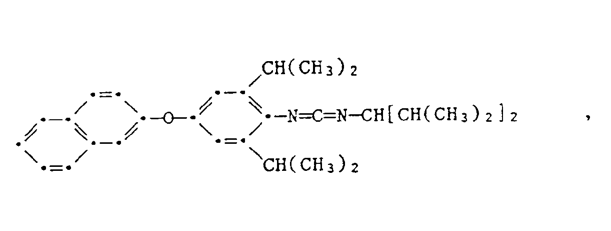 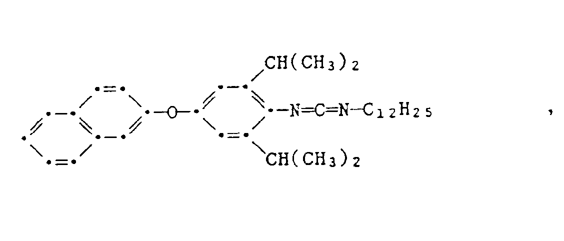 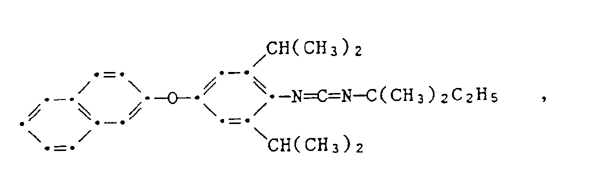 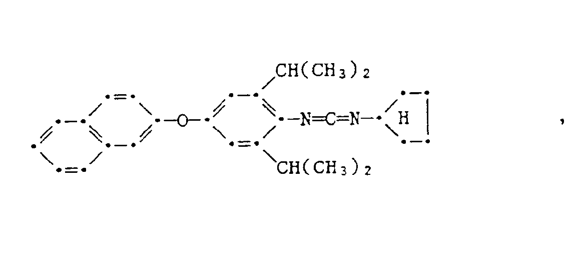 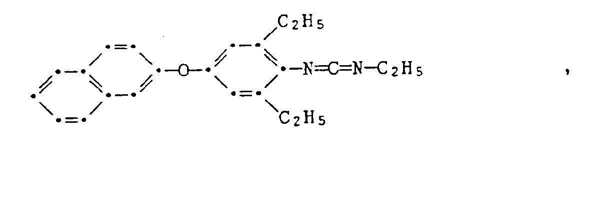 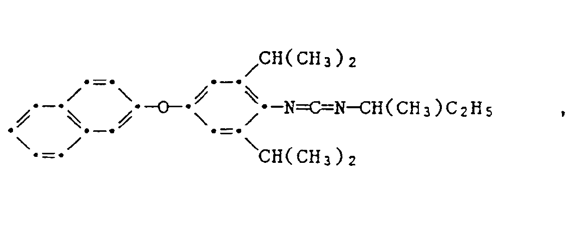 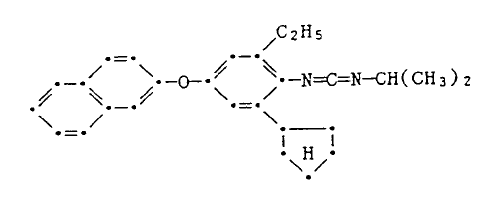 et

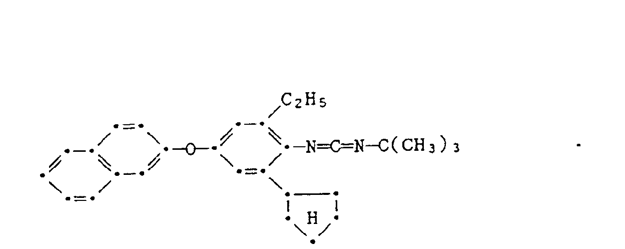

9. Produit pesticide contenant au moins un composant actif consistant en un composé de formule I 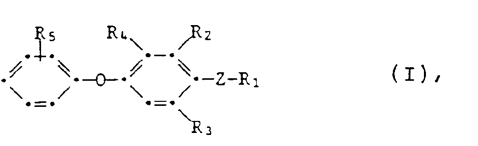 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3 ou un groupe (cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 3-C 6 ;
R₄ représente l'hydrogène ou un groupe méthyle ;
R₅ représente un pont (̵CH=CH)̵₂, (̵CH₂)̵₃, (̵CH₂)̵₄ en position 2,3 ou 3,4 ;
Z représente -NH-CS-NH-, -N=C(SR₆)-NH-, -N=C=N- ; et
R₆ représente un groupe alkyle en C 1-C 10 ou allyle, ou l'un de ses sels d'acide organique ou minéral avec des véhicules et/ou additifs appropriés.

10. Produit pesticide selon la revendication 9, contenant au moins un composant actif consistant en un composé de formule I dans laquelle R₁ représente un groupe alkyle en C 1-C 8, un groupe alkyle en C 1-C 8 portant un ou plusieurs substituants choisis parmi les halogènes et/ou les groupes alcoxy en C 1-C 5, ou un groupe cycloalkyle en C 3-C 8 ; R₂ représente un groupe alkyle en C 1-C 5 ; R₃ représente un groupe alkyle en C 1-C 5 ou cylcopentyle ; R₄ représente l'hydrogène ; R₅ représente un pont (̵CH=CH)̵₂, (̵CH₂)̵₃, (̵CH₂)̵₄ en position 2,3 ou 3,4 ; Z représente -NH-CS-NH-, -N=C(SR₆)-NH-, -N=C=N- et R₆ représente un groupe alkyle en C 1-C 5 ou allyle.

11. Produit pesticide selon la revendication 10, pour lequel R₁ représente un groupe alkyle en C 1-C 4 ; R₂ et R₃ représentent chacun un groupe alkyle en C 1-C 3; R₄ représente l'hydrogène ; R₅ représente un pont (̵CH=CH)̵₂ ou (̵CH₂)̵₃ en position 3,4 ; et Z représente -NH-CS-NH-.

12. Produit pesticide selon la revendication 10, pour lequel R₁ représente un groupe alkyle en C 1-C 4 ; R₂ et R₃ représentent chacun un groupe alkyle en C 1-C 3; R₄ représente l'hydrogène ; R₅ représente un pont (̵CH=CH)̵₂, (̵CH₂)̵₃ en position 3,4 ; Z représente -N=C(SRₛ)-NH- ; et R₆ représente un groupe alkyle en C 1-C 3.

13. Produit pesticide selon la revendication 10, pour lequel R₁ représente un groupe alkyle en C 1-C 4 ; R₂ et R₃ représentent chacun un groupe alkyle en C 1-C 3; R₄ représente l'hydrogène ; R₅ représente un pont (̵CH=CH)̵₂ ou (̵CH₂)̵₃ en position 3,4 ; et Z représente -N=C=N-.

14. Utilisation d'un composé de formule 1 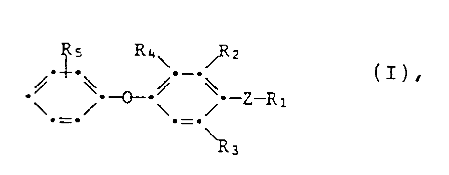 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3 ou un groupe (cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 3-C 6 ;
R₄ représente l'hydrogène ou un groupe méthyle ;
R₅ représente un pont (̵CH=CH)̵₂, (̵CH₂)̵₃, (̵CH₂)̵₄ en position 2,3 ou 3,4 ;
Z représente -NH-CS-NH-, -N=C(SR₆)-NH-, -N=C=N- ; et
R₆ représente un groupe alkyle en C 1-C 10 ou allyle,
ou de l'un de ses sels d'acide organique ou minéral pour la lutte contre les parasites des animaux et des végétaux.

15. Utilisation selon la revendication 14, pour la lutte contre les insectes et les arachnides.

16. Procédé pour combattre les parasites des animaux et des végétaux, caractérisé en ce que l'on met les parasites, dans leurs divers stades de développement, en contact avec un composé de formule 1 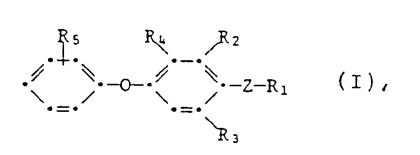 dans laquelle
R₁ représente un groupe alkyle en C 1-C 12, un groupe alkyle en C 1-C 12 portant un ou plusieurs substituants choisis parmi les halogènes et/ou les groupes alcoxy en C 1-C 6, un groupe cycloalkyle en C 3-C 8, un groupe cycloalkyle en C 3-C 8 portant un ou plusieurs substituants alkyle en C 1-C 3 ou un groupe (cycloalkyle en C 3-C 8)-alkyle en C 1-C 4 ;
R₂ représente l'hydrogène ou un groupe alkyle en C 1-C 5;
R₃ représente un groupe alkyle en C 1-C 5 ou cycloalkyle en C 3-C 6 ;
R₄ représente l'hydrogène ou un groupe méthyle ;
R₅ représente un pont (̵CH=CH)̵₂, (̵CH₂)̵₃, (̵CH₂)̵₄ en position 2,3 ou 3,4 ;
Z représente -NH-CS-NH-, -N=C(SR₆)-NH-, -N=C=N- ; et
R₆ représente un groupe alkyle en C 1-C 10 ou allyle, ou avec l'un de ses sels d'acide organique ou minéral.

17. Procédé de préparation d'un composé de formule V 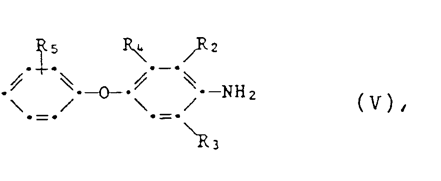 dans laquelle
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ; et
R₅ représente un pont (̵CH=CH)̵₂, (̵CH₂)̵₃ ou (̵CH₂)̵₄ en position 2,3 ou 3,4, caractérisé en ce que l'on fait réagir une aniline de formule VI 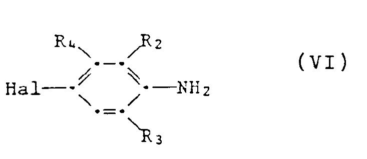 avec un phénol de formule VII 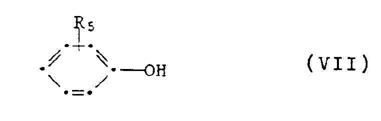 R₂, R₃, R₄ et R₅ ayant les significations indiquées en référence à la formule V, et Hal représentant un halogène, en particulier le chlore ou le brome.

18. Procédé selon la revendication 17, dans lequel R₂ représente un groupe alkyle en C 1-C 5 ; R₃ représente un groupe alkyle en C 1-C 5 ou cyclopentyle ; R₄ représente l'hydrogène et R₅ un pont (̵CH=CH)̵₂, (̵CH₂)̵₃ ou (̵CH₂)̵₄ en position 2,3 ou 3,4.

19. Procédé selon la revendication 18, dans lequel R₂ et R₃ représentent chacun un groupe alkyle en C 1-C 3 ; R₄ représente l'hydrogène et R₅ un pont (̵CH=CH)̵₂ ou (̵CH₂)̵₃ en position 3,4.

20. Procédé selon la revendication 19, pour la préparation des composés de formules 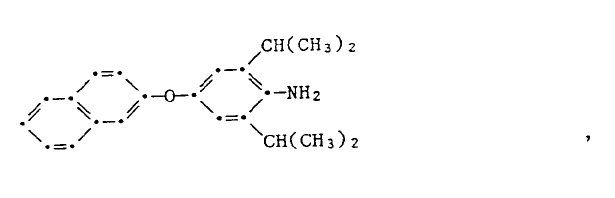 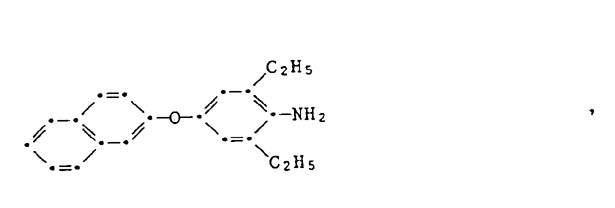 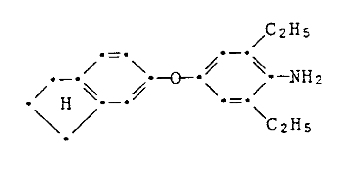 et

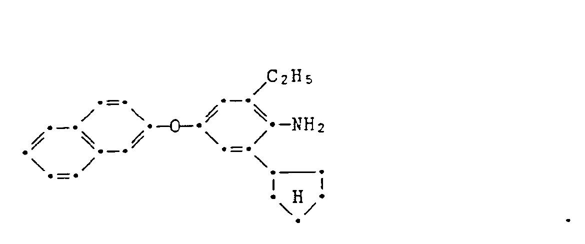

21. Procédé de préparation des composés de formule II 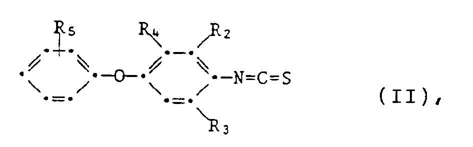 dans laquelle
R₂ représente l'hydrogène ou un groupe alkyle en C 1-C 5;
R₃ représente un groupe alkyle en C 1-C 5 ou cycloalkyle en C 5-C 6 ;
R₄ représente l'hydrogène ou un groupe méthyle ; et
R₅ représente un pont (̵CH=CH)̵₂, (̵CH₂)̵₃ ou (̵CH₂)̵₄ en position 2,3 ou 3,4,
caractérisé en ce que l'on fait réagir avec le thiophosgène une phénoxyaniline de formule V 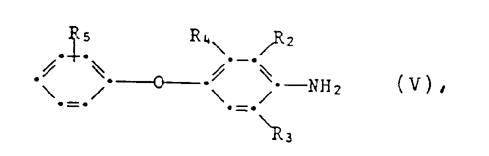 dans laquelle R₂, R₃, R₄ et R₅ ont les significations indiquées en référence à la formule II.

22. Procédé selon la revendication 21, dans lequel R₂ représente un groupe alkyle en C 1-C 5 ; R₃ un groupe alkyle en C 1-C 5 ou cyclopentyle ; R₄ l'hydrogène et R₅ un pont (̵CH=CH)̵₂, (̵CH₂)̵₃ ou (̵CH₂)̵₄ en position 2,3 ou 3,4.

23. Procédé selon la revendication 22, dans lequel R₂ et R₃ représentent chacun un groupe alkyle en C 1-C 3 ; R₄ l'hydrogène et R₅ un pont (̵CH=CH)̵₂ ou (̵CH₂)̵₃ en position 3,4.

24. Procédé selon la revendication 23, pour la préparation des composés de formules 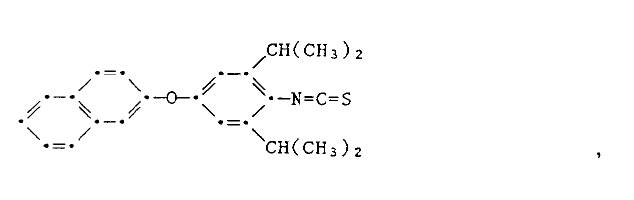 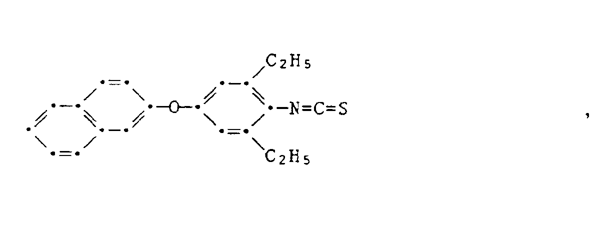 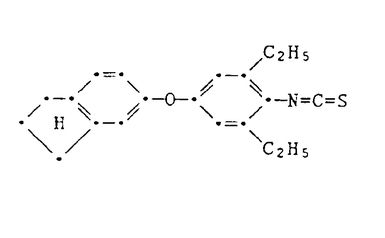 et
